(19) Europäisches Patentamt
European Patent Office
Office européen des brevets

(11) EP 2 017 620 A2

(12) EUROPEAN PATENT APPLICATION

(43) Date of publication:
21.01.2009 Bulletin 2009/04

(51) Int Cl.:
*G01N 33/573* (2006.01)

(21) Application number: 08017858.5

(22) Date of filing: 15.04.2005

(84) Designated Contracting States:
AT BE BG CH CY CZ DE DK EE ES FI FR GB GR HU IE IS IT LI LT LU MC NL PL PT RO SE SI SK TR

(30) Priority: 28.04.2004 EP 04010057

(62) Document number(s) of the earlier application(s) in accordance with Art. 76 EPC:
05729922.4 / 1 745 292

(71) Applicant: Bayer HealthCare AG
51368 Leverkusen (DE)

(72) Inventors:
- Golz, Stefan, Dr.
45326 Essen (DE)
- Brüggemeier, Ulf, Dr.
42799 Leichlingen (DE)
- Geerts, Andreas
42113 Wuppertal (DE)

Remarks:
This application was filed on 11-10-2008 as a divisional application to the application mentioned under INID code 62.

# (54) Diagnostics and therapeutics for diseases associated with dipeptidyl-peptidase 1 (DPP1)

(57) The invention provides a human DPP1 which is associated with the infections, cardiovascular diseases, respiratory diseases, cancer, endocrinological diseases, metabolic diseases, gastro-enterological diseases, inflammation, hematological diseases, muscle skeleton diseases, neurological diseases and urological diseases. The invention also provides assays for the identification of compounds useful in the treatment or prevention of infections, cardiovascular diseases, respiratory diseases, cancer, endocrinological diseases, metabolic diseases, gastro-enterological diseases, inflammation, hematological diseases, muscle skeleton diseases, neurological diseases and urological diseases. The invention also features compounds which bind to and/or activate or inhibit the activity of DPP1 as well as pharmaceutical compositions comprising such compounds.

EP 2 017 620 A2

## Description

## Technical field of the invention

The present invention is in the field of molecular biology, more particularly, the present invention relates to nucleic acid sequences and amino acid sequences of a human DPP1 and its regulation for the treatment of infections, cardiovascular diseases, respiratory diseases, cancer, endocrinological diseases, metabolic diseases, gastroenterological diseases, inflammation, hematological diseases, muscle skeleton diseases, neurological diseases and urological diseases in mammals.

## Background of the invention

Proteases play a role in carefully controlled processes, such as blood coagulation, fibrinolysis, complement activation, fertilization, and hormone production. These enzymes are also used in a variety of diagnostic, therapeutic, and industrial contexts. DPP1 is a member of the group of protease enzymes [[Paris et al. (1995)], [Rao et al. (1997)], [Pham et al. (1997)], EP1394274, WO02068579, US 5637462].

Proteases were recognized very early in the history of biochemistry. In the nineteenth century, one primary focus of research was on digestive proteases, like pepsin and trypsin. Proteases belong systematically to the C-N Hydrolases. More specifically, proteases catalyze the hyprolytic cleavage of a peptide bond and are therefore called peptidases as well.

Proteases can be classified according to several criteria, e.g. by localisation. Digestive proteases are located in the gastro-intestinal tract. These proteases are responsible for the digestion of food proteins.

Peptidases located extracellularly in the blood or other extracellular compartments of the body play often regulatory roles in processes like for example blood clotting, fibrinolysis, or activation of complement constituents.

Intracellularly located proteases exhibit a wide variety of roles. They are found in compartiments like the ER, the Golgi apparatus, or the lysomes. Their functions include for example activation of peptide hormons, ubiquitin mediated proteolysis, among others.

Proteases are most commonly classified according to their mechanism of action, or to specific active groups that are present in the active center. The following groups can be distinguished:

1. Serin-peptidases, 2. cystein-peptidases, 3. aspartyl- or acidic-peptidases, 4. metallo-peptidases, or 5. peptidases with yet unclear reaction mechanism.

*Serine peptidases*

Serine proteases exhibit a serine in the catalytic site which forms a covalent ester intermediate during the catalytic reaction pathway by a nucleophilic attack on the carboxy carbon of the peptide bond. In the active site of serine proteases a catalytic triad comprised of an aspartate, a histidine and the above mentioned serine is found. This triad functions in the reaction mechanism as a charge relay system.

To the large family of serine protease belong, for example, the digestive enzymes trypsin and chymotrypsin, components of the complement cascade, enzymes involved in the blood clotting cascade, as well as enzymes that function in degradation, rebuilding and maintenace of constituents of the extracellular matrix.

One feature of the serine protease family is the broad range of substrate specificity. Members of the trypase subgroup cleave after arginine or lysine, chymases after phenylalanine or leucine, aspases after aspartate, metases after methionine and serases after serine.

*Cysteine proteases*

During the catalytic reaction of cysteine proteases a covalent thioester intermediate is formed by a nucleophilic attack of the cysteine on the caboxy carbon of the peptide bond. Similar to the serine peptidases a catalytic triad comprised of the cysteine, a histidine and an asparagine is found which functions as a charge relay system to facilitate the formation of the thioester intermediate.

Members of the Cysteine protease family have roles in many different cellular processes, e.g. processing of precursers or intracellular degradation. Examples for cysteine proteases include lysosomal cathepsines, and cytosolic calpains.

*Aspartyl- or acidic peptidase*

The catalytic site of aspartyl proteases is composed of two aspartate residues. At the pH optimum of aspartyl proteases (2-3) one of the aspartyl carboxy groups is ionized and the other is neutral, which is important for the catalytic reaction to occur. Examples for aspartyl proteases are gastric pepsins A and C, chymosin, as well as mammalian renin.

*Metallo-peptidases*

Metallo-peptidases are proteases, whose proteolytic activity depends on the presence of divalent cations in the active center. Examples of members of this class are carboxypeptidase A, which represents a pancreatic digestive enzyme, the Angiotension Converting Enzymes (ACE), which are responsible for the conversion of angiotensin I to angiotensin II, or the Extracellular Matrix Metalloprotienases.

In summary, a huge number of proteases play a central role in several important cellular and intracellular processes. Furthermore, the value as pharmaceutical targets has been proven for several proteases. For example, the protease encoded by the HIV genome is used as a target for drugs for the treatment of HIV infections, the proteasom complex has been discovered as an anticancer target, or Cys-proteases have been implemented as drug targets for inflammatory disorders. Selective inhibitors have been developed as therapeutic agents for diseases such as HIV. Thus, the identification of further disease implications of protease species and their splice variants may lead to the development of specific inhibitors or modulators, or suggest new utilities for known compounds affecting proteases. That in turn will provide additional pharmacological approaches to treat diseases and conditions in which protease activities are involved. This diseases may include, but are not limited to, infections such as bacterial, fungal, protozoan, and viral infections, particularly those caused by HIV viruses, cancers, allergies including asthma, cardiovascular diseases including acute heart failure, hypotension, hypertension, angina pectoris, myocardial infarction, hematological diseases, genito-urinary diseases including urinary incontinence and benign prostate hyperplasia, osteoporosis, peripheral and central nervous system disorders including pain, Alzheimer's disease and Parkinson's disease, respiratory diseases, metabolic diseases, inflammatory diseases, gastro-enterological diseases, diseases of the endocrine system, dermatological diseases, diseases of muscles or the sceleton, immunological diseases, developmental diseases or diseases of the reproductive system.

**TaqMan-Technology / expression profiling**

TaqMan is a recently developed technique, in which the release of a fluorescent reporter dye from a hybridisation probe in real-time during a polymerase chain reaction (PCR) is proportional to the accumulation of the PCR product. Quantification is based on the early, linear part of the reaction, and by determining the threshold cycle (CT), at which fluorescence above background is first detected.

Gene expression technologies may be useful in several areas of drug discovery and development, such as target identification, lead optimization, and identification of mechanisms of action. The TaqMan technology can be used to compare differences between expression profiles of normal tissue and diseased tissue. Expression profiling has been used in identifying genes, which are up-or downregulated in a variety of diseases. An interesting application of expression profiling is temporal monitoring of changes in gene expression during disease progression and drug treatment or in patients versus healthy individuals. The premise in this approach is that changes in pattern of gene expression in response to physiological or environmental stimuli (e.g., drugs) may serve as indirect clues about disease-causing genes or drug targets. Moreover, the effects of drugs with established efficacy on global gene expression patterns may provide a guidepost, or a genetic signature, against which a new drug candidate can be compared.

**DPP1**

The nucleotide sequence of DPP1 is accessible in the databases by the accession number X87212 and is given in SEQ ID NO: 1. The amino acid sequence of DPP1 depicted in SEQ ID NO:2.

Cathepsin C, or dipeptidyl aminopeptidase I, is a lysosomal protease capable of removing dipeptides from the amino terminus of protein substrates. Unlike cathepsins B, H, L, and S, which are small monomeric enzymes, cathepsin C is a large (200 kD) oligomeric protein [Paris et al. (1995)].

Paris et al. [Paris et al. (1995)] cloned a human cDNA encoding cathepsin C. The gene encodes a 463-amino acid polypeptide with predicted features of the papain family of cysteine proteases. Northern blot analysis by Rao et al. [Rao et al. (1997)] showed that cathepsin C is expressed at high levels in lung, kidney, and placenta, and at moderate or low levels in a variety of other organs. Rao et al. [Rao et al. (1997)] found that the CTSC gene spans approximately 3.5 kb and consists of 2 exons. Analysis of the 5-prime flanking region of the gene revealed no classic TATA or CCAAT box, but did reveal several possible tissue-specific regulatory elements. Pham et al. [Pham et al. (1997)] cloned the mouse cathepsin C gene and reported that it is 77.8% identical to the human gene at the amino acid level.

DPP1 is published in patents EP1394274, WO02068579 and US 5637462.

## Summary of the invention

The invention relates to novel disease associations of DPP1 polypeptides and polynucleotides. The invention also relates to novel methods of screening for therapeutic agents for the treatment of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal. The invention also relates to pharmaceutical compositions for the treatment of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising a DPP1 polypeptide, a DPP1 polynucleotide, or regulators of DPP1 or modulators of DPP1 activity. The invention further comprises methods of diagnosing infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal.

## Brief Description of the Drawings

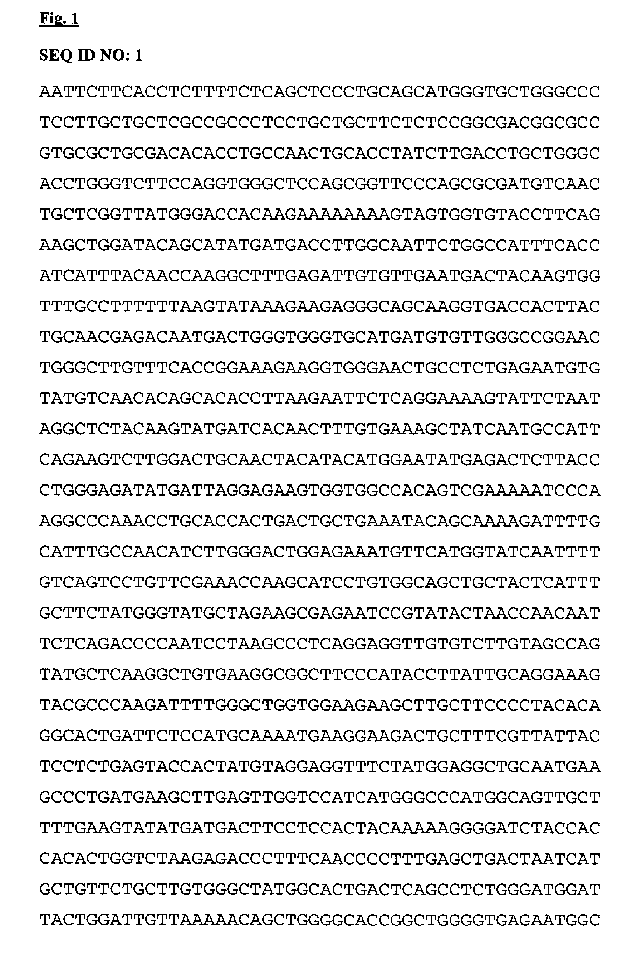

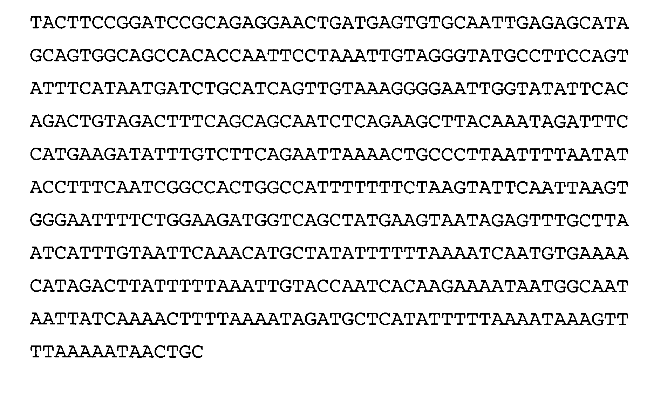

Fig. 1 shows the nucleotide sequence of a DPP1 receptor polynucleotide (SEQ ID NO:1).

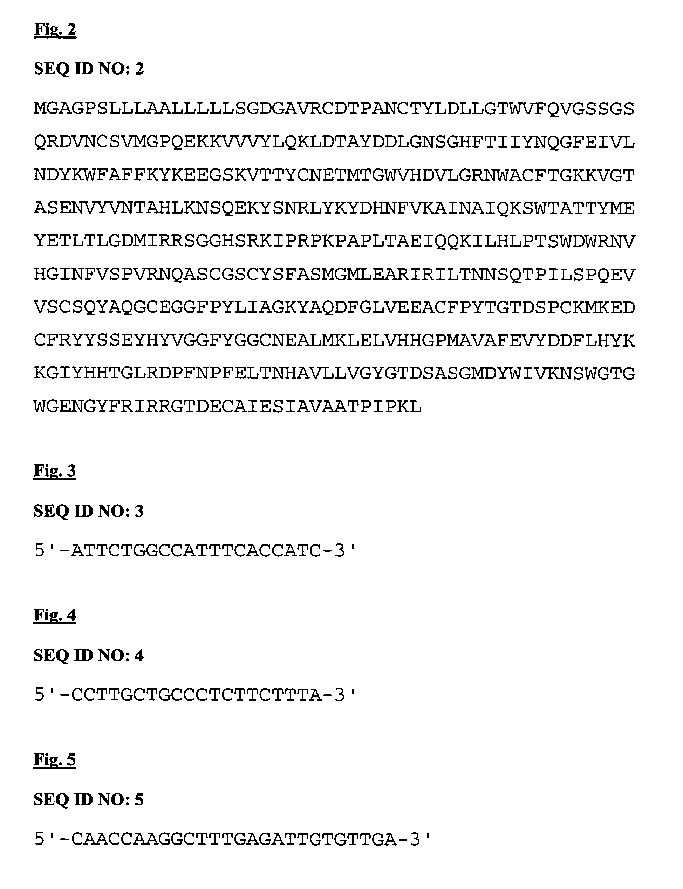

Fig. 2 shows the amino acid sequence of a DPP1 receptor polypeptide (SEQ ID NO:2).

Fig. 3 shows the nucleotide sequence of a primer useful for the invention (SEQ ID NO:3).

Fig. 4 shows the nucleotide sequence of a primer useful for the invention (SEQ ID NO:4).

Fig. 5 shows a nucleotide sequence useful as a probe to detect proteins of the invention (SEQ ID NO:5).

## Detailed description of the invention

### Definition of terms

An "oligonucleotide" is a stretch of nucleotide residues which has a sufficient number of bases to be used as an oligomer, amplimer or probe in a polymerase chain reaction (PCR). Oligonucleotides are prepared from genomic or cDNA sequence and are used to amplify, reveal, or confirm the presence of a similar DNA or RNA in a particular cell or tissue. Oligonucleotides or oligomers comprise portions of a DNA sequence having at least about 10 nucleotides and as many as about 35 nucleotides, preferably about 25 nucleotides.

"Probes" may be derived from naturally occurring or recombinant single- or double-stranded nucleic acids or may be chemically synthesized. They are useful in detecting the presence of identical or similar sequences. Such probes may be labeled with reporter molecules using nick translation, Klenow fill-in reaction, PCR or other methods well known in the art. Nucleic acid probes may be used in southern, northern or in situ hybridizations to determine whether DNA or RNA encoding a certain protein is present in a cell type, tissue, or organ.

A "fragment of a polynucleotide" is a nucleic acid that comprises all or any part of a given nucleotide molecule, the fragment having fewer nucleotides than about 6 kb, preferably fewer than about 1 kb.

"Reporter molecules" are radionuclides, enzymes, fluorescent, chemiluminescent, or chromogenic agents which associate with a particular nucleotide or amino acid sequence, thereby establishing the presence of a certain sequence, or allowing for the quantification of a certain sequence.

"Chimeric" molecules may be constructed by introducing all or part of the nucleotide sequence of this invention into a vector containing additional nucleic acid sequence which might be expected to change any one or several of the following DPP1 characteristics: cellular location, distribution, ligand-binding affinities, interchain affinities, degradation/turnover rate, signaling, etc.

"Active", with respect to a DPP1 polypeptide, refers to those forms, fragments, or domains of a DPP1 polypeptide which retain the biological and/or antigenic activity of a DPP1 polypeptide.

"Naturally occurring DPP1 polypeptide" refers to a polypeptide produced by cells which have not been genetically engineered and specifically contemplates various polypeptides arising from post-translational modifications of the polypeptide including but not limited to acetylation, carboxylation, glycosylation, phosphorylation, lipidation and acylation.

"Derivative" refers to polypeptides which have been chemically modified by techniques such as ubiquitination, labeling (see above), pegylation (derivatization with polyethylene glycol), and chemical insertion or substitution of amino acids such as ornithine which do not normally occur in human proteins.

"Conservative amino acid substitutions" result from replacing one amino acid with another having similar structural and/or chemical properties, such as the replacement of a leucine with an isoleucine or valine, an aspartate with a glutamate, or a threonine with a serine.

"Insertions" or "deletions" are typically in the range of about 1 to 5 amino acids. The variation allowed may be experimentally determined by producing the peptide synthetically while systematically making insertions, deletions, or substitutions of nucleotides in the sequence using recombinant DNA techniques.

A "signal sequence" or "leader sequence" can be used, when desired, to direct the polypeptide through a membrane of a cell. Such a sequence may be naturally present on the polypeptides of the present invention or provided from heterologous sources by recombinant DNA techniques.

An "oligopeptide" is a short stretch of amino acid residues and may be expressed from an oligonucleotide. Oligopeptides comprise a stretch of amino acid residues of at least 3, 5, 10 amino acids and at most 10, 15, 25 amino acids, typically of at least 9 to 13 amino acids, and of sufficient length to display biological and/or antigenic activity.

"Inhibitor" is any substance which retards or prevents a chemical or physiological reaction or response. Common inhibitors include but are not limited to antisense molecules, antibodies, and antagonists.

"Standard expression" is a quantitative or qualitative measurement for comparison. It is based on a statistically appropriate number of normal samples and is created to use as a basis of comparison when performing diagnostic assays, running clinical trials, or following patient treatment profiles.

"Animal" as used herein may be defined to include human, domestic (e.g., cats, dogs, etc.), agricultural (e.g., cows, horses, sheep, etc.) or test species (e.g., mouse, rat, rabbit, etc.).

A "DPP1 polynucleotide", within the meaning of the invention, shall be understood as being a nucleic acid molecule selected from a group consisting of

(i) nucleic acid molecules encoding a polypeptide comprising the amino acid sequence of SEQ ID NO: 2,

(ii) nucleic acid molecules comprising the sequence of SEQ ID NO: 1,

(iii) nucleic acid molecules having the sequence of SEQ ID NO: 1,

(iv) nucleic acid molecules the complementary strand of which hybridizes under stringent conditions to a nucleic acid molecule of (i), (ii), or (iii); and

(v) nucleic acid molecules the sequence of which differs from the sequence of a nucleic acid molecule of (iii) due to the degeneracy of the genetic code;

wherein the polypeptide encoded by said nucleic acid molecule has DPP1 activity.

A "DPP1 polypeptide", within the meaning of the invention, shall be understood as being a polypeptide selected from a group consisting of

(i) polypeptides having the sequence of SEQ ID NO: 2,

(ii) polypeptides comprising the sequence of SEQ ID NO: 2,

(iii) polypeptides encoded by DPP1 polynucleotides; and

(iv) polypeptides which show at least 99%, 98%, 95%, 90%, or 80% homology with a polypeptide of (i), (ii), or (iii);

wherein said polypeptide has DPP1 activity.

The nucleotide sequences encoding a DPP1 (or their complement) have numerous applications in techniques known to those skilled in the art of molecular biology. These techniques include use as hybridization probes, use in the construction of oligomers for PCR, use for chromosome and gene mapping, use in the recombinant production of DPP1, and use in generation of antisense DNA or RNA, their chemical analogs and the like. Uses of nucleotides encoding a DPP1 disclosed herein are exemplary of known techniques and are not intended to limit their use in any technique known to a person of ordinary skill in the art. Furthermore, the nucleotide sequences disclosed herein may be used in molecular biology techniques that have not yet been developed, provided the new techniques rely on properties of nucleotide sequences that are currently known, e.g., the triplet genetic code, specific base pair interactions, etc.

It will be appreciated by those skilled in the art that as a result of the degeneracy of the genetic code, a multitude of DPP1 - encoding nucleotide sequences may be produced. Some of these will only bear minimal homology to the nucleotide sequence of the known and naturally occurring DPP1. The invention has specifically contemplated each and every possible variation of nucleotide sequence that could be made by selecting combinations based on possible codon choices. These combinations are made in accordance with the standard triplet genetic code as applied to the nucleotide sequence of naturally occurring DPP1, and all such variations are to be considered as being specifically disclosed.

Although the nucleotide sequences which encode a DPP1, its derivatives or its variants are preferably capable of hybridizing to the nucleotide sequence of the naturally occurring DPP1 polynucleotide under stringent conditions, it may be advantageous to produce nucleotide sequences encoding DPP1 polypeptides or its derivatives possessing a substantially different codon usage. Codons can be selected to increase the rate at which expression of the peptide occurs in a particular prokaryotic or eukaryotic expression host in accordance with the frequency with which particular codons are utilized by the host. Other reasons for substantially altering the nucleotide sequence encoding a DPP1 polypeptide and/or its derivatives without altering the encoded amino acid sequence include the production of RNA transcripts having more desirable properties, such as a greater half-life, than transcripts produced from the naturally occurring sequence.

Nucleotide sequences encoding a DPP1 polypeptide may be joined to a variety of other nucleotide sequences by means of well established recombinant DNA techniques. Useful nucleotide sequences for joining to DPP1 polynucleotides include an assortment of cloning vectors such as plasmids, cosmids, lambda phage derivatives, phagemids, and the like. Vectors of interest include expression vectors, replication vectors, probe generation vectors, sequencing vectors, etc. In general, vectors of interest may contain an origin of replication functional in at least one organism, convenient restriction endonuclease sensitive sites, and selectable markers for one or more host cell systems.

Another aspect of the subject invention is to provide for DPP1-specific hybridization probes capable of hybridizing with naturally occurring nucleotide sequences encoding DPP1. Such probes may also be used for the detection of similar protease encoding sequences and should preferably show at least 40% nucleotide identity to DPP1 polynucleotides. The hybridization probes of the subject invention may be derived from the nucleotide sequence presented as SEQ ID NO: 1 or from genomic sequences including promoter, enhancers or introns of the native gene. Hybridization probes may be labelled by a variety of reporter molecules using techniques well known in the art.

It will be recognized that many deletional or mutational analogs of DPP1 polynucleotides will be effective hybridization probes for DPP1 polynucleotides. Accordingly, the invention relates to nucleic acid sequences that hybridize with such DPP1 encoding nucleic acid sequences under stringent conditions.

"Stringent conditions" refers to conditions that allow for the hybridization of substantially related nucleic acid sequences. For instance, such conditions will generally allow hybridization of sequence with at least about 85% sequence identity, preferably with at least about 90% sequence identity, more preferably with at least about 95% sequence identity. Hybridization conditions and probes can be adjusted in well-characterized ways to achieve selective hybridization of human-derived probes. Stringent conditions, within the meaning of the invention are 65°C in a buffer containing 1 mM EDTA, 0.5 M $NaHPO_4$ (pH 7.2), 7% (w/v) SDS.

Nucleic acid molecules that will hybridize to DPP1 polynucleotides under stringent conditions can be identified functionally. Without limitation, examples of the uses for hybridization probes include: histochemical uses such as identifying tissues that express DPP1; measuring mRNA levels, for instance to identify a sample's tissue type or to identify cells that express abnormal levels of DPP1; and detecting polymorphisms of DPP1.

PCR provides additional uses for oligonucleotides based upon the nucleotide sequence which encodes DPP1. Such probes used in PCR may be of recombinant origin, chemically synthesized, or a mixture of both. Oligomers may comprise discrete nucleotide sequences employed under optimized conditions for identification of DPP1 in specific tissues or diagnostic use. The same two oligomers, a nested set of oligomers, or even a degenerate pool of oligomers may be employed under less stringent conditions for identification of closely related DNAs or RNAs.

Rules for designing polymerase chain reaction (PCR) primers are now established, as reviewed by PCR Protocols. Degenerate primers, i.e., preparations of primers that are heterogeneous at given sequence locations, can be designed to amplify nucleic acid sequences that are highly homologous to, but not identical with DPP 1. Strategies are now available that allow for only one of the primers to be required to specifically hybridize with a known sequence. For example, appropriate nucleic acid primers can be ligated to the nucleic acid sought to be amplified to provide the hybridization partner for one of the primers. In this way, only one of the primers need be based on the sequence of the nucleic acid sought to be amplified.

PCR methods for amplifying nucleic acid will utilize at least two primers. One of these primers will be capable of hybridizing to a first strand of the nucleic acid to be amplified and of priming enzyme-driven nucleic acid synthesis in a first direction. The other will be capable of hybridizing the reciprocal sequence of the first strand (if the sequence to be amplified is single stranded, this sequence will initially be hypothetical, but will be synthesized in the first amplification cycle) and of priming nucleic acid synthesis from that strand in the direction opposite the first direction and towards the site of hybridization for the first primer. Conditions for conducting such amplifications, particularly under preferred stringent hybridization conditions, are well known.

Other means of producing specific hybridization probes for DPP1 include the cloning of nucleic acid sequences encoding DPP1 or DPP1 derivatives into vectors for the production of mRNA probes. Such vectors are known in the art, are commercially available and may be used to synthesize RNA probes in vitro by means of the addition of the appropriate RNA polymerase as T7 or SP6 RNA polymerase and the appropriate reporter molecules.

It is possible to produce a DNA sequence, or portions thereof, entirely by synthetic chemistry. After synthesis, the nucleic acid sequence can be inserted into any of the many available DNA vectors and their respective host cells using techniques which are well known in the art. Moreover, synthetic chemistry may be used to introduce mutations into the nucleotide sequence. Alternately, a portion of sequence in which a mutation is desired can be synthesized and recombined with longer portion of an existing genomic or recombinant sequence.

DPP1 polynucleotides may be used to produce a purified oligo-or polypeptide using well known methods of recombinant DNA technology. The oligopeptide may be expressed in a variety of host cells, either prokaryotic or eukaryotic. Host cells may be from the same species from which the nucleotide sequence was derived or from a different species. Advantages of producing an oligonucleotide by recombinant DNA technology include obtaining adequate amounts of the protein for purification and the availability of simplified purification procedures.

**Quantitative determinations of nucleic acids**

An important step in the molecular genetic analysis of human disease is often the enumeration of the copy number of a nucleis acid or the relative expression of a gene in particular tissues.

Several different approaches are currently available to make quantitative determinations of nucleic acids. Chromosome-based techniques, such as comparative genomic hybridization (CGH) and fluorescent in situ hybridization (FISH) facilitate efforts to cytogenetically localize genomic regions that are altered in tumor cells. Regions of genomic alteration can be narrowed further using loss of heterozygosity analysis (LOH), in which disease DNA is analyzed and compared with normal DNA for the loss of a heterozygous polymorphic marker. The first experiments used restriction fragment length polymorphisms (RFLPs) [Johnson, (1989)], or hypervariable minisatellite DNA [Barnes, 2000]. In recent years LOH has been performed primarily using PCR amplification of microsatellite markers and electrophoresis of the radio labelled [Jeffreys, (1985)] or fluorescently labelled PCR products [Weber, (1990)] and compared between paired normal and disease DNAs.

A number of other methods have also been developed to quantify nucleic acids [Gergen, (1992)]. More recently, PCR and RT-PCR methods have been developed which are capable of measuring the amount of a nucleic acid in a sample. One approach, for example, measures PCR product quantity in the log phase of the reaction before the formation of reaction products plateaus [Thomas, (1980)].

A gene sequence contained in all samples at relatively constant quantity is typically utilized for sample amplification efficiency normalization. This approach, however, suffers from several drawbacks. The method requires that each sample has equal input amounts of the nucleic acid and that the amplification efficiency between samples is identical until the time of analysis. Furthermore, it is difficult using the conventional methods of PCR quantitation such as gel electrophoresis or plate capture hybridization to determine that all samples are in fact analyzed during the log phase of the reaction as required by the method.

Another method called quantitative competitive (QC)-PCR, as the name implies, relies on the inclusion of an internal control competitor in each reaction [Piatak, (1993), BioTechniques]. The efficiency of each reaction is normalized to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency than the target molecule.

*5'fluorogenic Nuclease Assays*

Fluorogenic nuclease assays are a real time quantitation method that uses a probe to monitor formation of amplification product. The basis for this method of monitoring the formation of amplification product is to measure continuously PCR product accumulation using a dual-labelled fluorogenic oligonucleotide probe, an approach frequently referred to in the literature simply as the "TaqMan method" [Piatak,(1993), Science; Heid, (1996); Gibson, (1996); Holland. (1991)].

The probe used in such assays is typically a short (about 20-25 bases) oligonucleotide that is labeled with two different fluorescent dyes. The 5' terminus of the probe is attached to a reporter dye and the 3' terminus is attached to a quenching dye, although the dyes could be attached at other locations on the probe as well. The probe is designed to have at least substantial sequence complementarity with the probe binding site. Upstream and downstream PCR primers which bind to flanking regions of the locus are added to the reaction mixture. When the probe is intact, energy transfer between the two fluorophors occurs and the quencher quenches emission from the reporter. During the extension phase of PCR, the probe is cleaved by the 5' nuclease activity of a nucleic acid polymerase such as Taq polymerase, thereby releasing the reporter from the oligonucleotide-quencher and resulting in an increase of reporter emission intensity which can be measured by an appropriate detector.

One detector which is specifically adapted for measuring fluorescence emissions such as those created during a fluorogenic assay is the ABI 7700 or 4700 HT manufactured by Applied Biosystems, Inc. in Foster City, Calif. The ABI 7700 uses fiber optics connected with each well in a 96-or 384 well PCR tube arrangement. The instrument includes a laser for exciting the labels and is capable of measuring the fluorescence spectra intensity from each tube with continuous monitoring during PCR amplification. Each tube is re-examined every 8.5 seconds.

Computer software provided with the instrument is capable of recording the fluorescence intensity of reporter and quencher over the course of the amplification. The recorded values will then be used to calculate the increase in normalized reporter emission intensity on a continuous basis. The increase in emission intensity is plotted versus time, i.e., the number of amplification cycles, to produce a continuous measure of amplification. To quantify the locus in each amplification reaction, the amplification plot is examined at a point during the log phase of product accumulation. This is accomplished by assigning a fluorescence threshold intensity above background and determining the point at which each amplification plot crosses the threshold (defined as the threshold cycle number or Ct). Differences in threshold cycle number are used to quantify the relative amount of PCR target contained within each tube. Assuming that each reaction functions at 100% PCR efficiency, a difference of one Ct represents a two-fold difference in the amount of starting template. The fluorescence value can be used in conjunction with a standard curve to determine the amount of amplification product present.

*Non-Probe-Based Detection Methods*

A variety of options are available for measuring the amplification products as they are formed. One method utilizes labels, such as dyes, which only bind to double stranded DNA. In this type of approach, amplification product (which is double stranded) binds dye molecules in solution to form a complex. With the appropriate dyes, it is possible to distinguish between dye molecules free in solution and dye molecules bound to amplification product. For example, certain dyes fluoresce only when bound to amplification product. Examples of dyes which can be used in methods of this general type include, but are not limited to, Syber Green.TM. and Pico Green from Molecular Probes, Inc. of Eugene, Oreg., ethidium bromide, propidium iodide, chromomycin, acridine orange, Hoechst 33258, Toto-1, Yoyo-1, DAPI (4',6-diamidino-2-phenylindole hydrochloride).

Another real time detection technique measures alteration in energy fluorescence energy transfer between fluorophors conjugated with PCR primers [Livak, (1995)].

*Probe-Based Detection Methods*

These detection methods involve some alteration to the structure or conformation of a probe hybridized to the locus between the amplification primer pair. In some instances, the alteration is caused by the template-dependent extension catalyzed by a nucleic acid polymerase during the amplification process. The alteration generates a detectable signal which is an indirect measure of the amount of amplification product formed.

For example, some methods involve the degradation or digestion of the probe during the extension reaction. These methods are a consequence of the 5'-3' nuclease activity associated with some nucleic acid polymerases. Polymerases having this activity cleave mononucleotides or small oligonucleotides from an oligonucleotide probe annealed to its complementary sequence located within the locus.

The 3' end of the upstream primer provides the initial binding site for the nucleic acid polymerase. As the polymerase catalyzes extension of the upstream primer and encounters the bound probe, the nucleic acid polymerase displaces a portion of the 5' end of the probe and through its nuclease activity cleaves mononucleotides or oligonucleotides from the probe.

The upstream primer and the probe can be designed such that they anneal to the complementary strand in close proximity to one another. In fact, the 3' end of the upstream primer and the 5' end of the probe may abut one another. In this situation, extension of the upstream primer is not necessary in order for the nucleic acid polymerase to begin cleaving the probe. In the case in which intervening nucleotides separate the upstream primer and the probe, extension of the primer is necessary before the nucleic acid polymerase encounters the 5' end of the probe. Once contact occurs and polymerization continues, the 5'-3' exonuclease activity of the nucleic acid polymerase begins cleaving mononucleotides or oligonucleotides from the 5' end of the probe. Digestion of the probe continues until the remaining portion of the probe dissociates from the complementary strand.

In solution, the two end sections can hybridize with each other to form a hairpin loop. In this conformation, the reporter and quencher dye are in sufficiently close proximity that fluorescence from the reporter dye is effectively quenched by the quencher dye. Hybridized probe, in contrast, results in a linearized conformation in which the extent of quenching is decreased. Thus, by monitoring emission changes for the two dyes, it is possible to indirectly monitor the formation of amplification product.

*Probes*

The labeled probe is selected so that its sequence is substantially complementary to a segment of the test locus or a reference locus. As indicated above, the nucleic acid site to which the probe binds should be located between the primer binding sites for the upstream and downstream amplification primers.

*Primers*

The primers used in the amplification are selected so as to be capable of hybridizing to sequences at flanking regions of the locus being amplified. The primers are chosen to have at least substantial complementarity with the different strands of the nucleic acid being amplified. When a probe is utilized to detect the formation of amplification products, the primers are selected in such that they flank the probe, i.e. are located upstream and downstream of the probe.

The primer must have sufficient length so that it is capable of priming the synthesis of extension products in the presence of an agent for polymerization. The length and composition of the primer depends on many parameters, including, for example, the temperature at which the annealing reaction is conducted, proximity of the probe binding site to that of the primer, relative concentrations of the primer and probe and the particular nucleic acid composition of the probe.

Typically the primer includes 15-30 nucleotides. However, the length of the primer may be more or less depending on the complexity of the primer binding site and the factors listed above.

*Labels for Probes and Primers*

The labels used for labeling the probes or primers of the current invention and which can provide the signal corresponding to the quantity of amplification product can take a variety of forms. As indicated above with regard to the 5' fluorogenic nuclease method, a fluorescent signal is one signal which can be measured. However, measurements may also be made, for example, by monitoring radioactivity, colorimetry, absorption, magnetic parameters, or enzymatic activity. Thus, labels which can be employed include, but are not limited to, fluorophors, chromophores, radioactive isotopes, electron dense reagents, enzymes, and ligands having specific binding partners (e.g., biotin-avidin).

Monitoring changes in fluorescence is a particularly useful way to monitor the accumulation of amplification products. A number of labels useful for attachment to probes or primers are commercially available including fluorescein and various fluorescein derivatives such as FAM, HEX, TET and JOE (all which are available from Applied Biosystems, Foster City, Calif.); lucifer yellow, and coumarin derivatives.

Labels may be attached to the probe or primer using a variety of techniques and can be attached at the 5' end, and/or the 3' end and/or at an internal nucleotide. The label can also be attached to spacer arms of various sizes which are attached to the probe or primer. These spacer arms are useful for obtaining a desired distance between multiple labels attached to the probe or primer.

In some instances, a single label may be utilized; whereas, in other instances, such as with the 5' fluorogenic nuclease assays for example, two or more labels are attached to the probe. In cases wherein the probe includes multiple labels, it is generally advisable to maintain spacing between the labels which is sufficient to permit separation of the labels during digestion of the probe through the 5'-3' nuclease activity of the nucleic acid polymerase.

**Patients Exhibiting Symptoms of Disease**

A number of diseases are associated with changes in the copy number of a certain gene. For patients having symptoms of a disease, the real-time PCR method can be used to determine if the patient has copy number alterations which are known to be linked with diseases that are associated with the symptoms the patient has.

**DPP1 expression**

*DPP1 fusion proteins*

Fusion proteins are useful for generating antibodies against DPP1 polypeptides and for use in various assay systems. For example, fusion proteins can be used to identify proteins which interact with portions of DPP1 polypeptides. Protein affinity chromatography or library-based assays for protein-protein interactions, such as the yeast two-hybrid or phage display systems, can be used for this purpose. Such methods are well known in the art and also can be used as drug screens.

A DPP1 fusion protein comprises two polypeptide segments fused together by means of a peptide bond. The first polypeptide segment can comprise at least 54, 75, 100, 125, 139, 150, 175, 200, 225, 250, 275, 300, 325 or 350 contiguous amino acids of SEQ ID NO: 2 or of a biologically active variant, such as those described above. The first polypeptide segment also can comprise full-length DPP1.

The second polypeptide segment can be a full-length protein or a protein fragment. Proteins commonly used in fusion protein construction include, but are not limited to β galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located adjacent to the DPP1.

*Preparation of Polynucleotides*

A naturally occurring DPP1 polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated DPP1 polynucleotides. For example, restriction enzymes and probes can be used to isolate polynucleotide fragments which comprise DPP1 nucleotide sequences. Isolated polynucleotides are in preparations which are free or at least 70, 80, or 90% free of other molecules.

DPP1 cDNA molecules can be made with standard molecular biology techniques, using DPP1 mRNA as a template. DPP1 cDNA molecules can thereafter be replicated using molecular biology techniques known in the art. An amplification technique, such as PCR, can be used to obtain additional copies of polynucleotides of the invention, using either human genomic DNA or cDNA as a template.

Alternatively, synthetic chemistry techniques can be used to synthesizes DPP1 polynucleotides. The degeneracy of the genetic code allows alternate nucleotide sequences to be synthesized which will encode DPP1 having, for example, an amino acid sequence shown in SEQ ID NO: 2 or a biologically active variant thereof.

*Extending Polynucleotides*

Various PCR-based methods can be used to extend nucleic acid sequences encoding human DPP1, for example to detect upstream sequences of DPP1 gene such as promoters and regulatory elements. For example, restriction-site PCR uses universal primers to retrieve unknown sequence adjacent to a known locus. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region. Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA. In this method, multiple restriction enzyme digestions and ligations also can be used to place an engineered double-stranded sequence into an unknown fragment of the DNA molecule before performing PCR.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate equipment and software (*e.g.*, GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

*Obtaining Polypeptides*

DPP1 can be obtained, for example, by purification from human cells, by expression of DPP1 polynucleotides, or by direct chemical synthesis.

*Protein Purification*

DPP1 can be purified from any human cell which expresses the enzyme, including those which have been transfected with expression constructs which express DPP1. A purified DPP1 is separated from other compounds which normally associate with DPP1 in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

*Expression of DPP1 Polynucleotides*

To express DPP1, DPP1 polynucleotides can be inserted into an expression vector which contains the necessary elements for the transcription and translation of the inserted coding sequence. Methods which are well known to those skilled in the art can be used to construct expression vectors containing sequences encoding DPP1 and appropriate transcriptional and translational control elements. These methods include *in vitro* recombinant DNA techniques, synthetic techniques, and *in vivo* genetic recombination.

A variety of expression vector/host systems can be utilized to contain and express sequences encoding DPP1. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.*, baculovirus), plant cell systems transformed with virus expression vectors (*e.g.,* cauliflower mosaic virus, CaMV; tobacco mosaic virus, TMV) or with bacterial expression vectors (*e.g.*, Ti or pBR322 plasmids), or animal cell systems.

The control elements or regulatory sequences are those non-translated regions of the vector - enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.*, heat shock, RUBISCO, and storage protein genes) or from plant viruses (*e.g.*, viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding DPP1, vectors based on SV40 or EBV can be used with an appropriate selectable marker.

*Bacterial and Yeast Expression Systems*

In bacterial systems, a number of expression vectors can be selected. For example, when a large quantity of DPP1 is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding DPP1 can be ligated into the vector in frame with sequences for the amino-terminal Met and the subsequent 7 residues of β-galactosidase so that a hybrid protein is produced. pIN vectors or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

*Plant and Insect Expression Systems*

If plant expression vectors are used, the expression of sequences encoding DPP1 can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters can be used. These constructs can be introduced into plant cells by direct DNA transformation or by pathogen-mediated transfection.

An insect system also can be used to express DPP1. For example, in one such system *Autographa californica* nuclear polyhedrosis virus (AcNPV) is used as a vector to express foreign genes in *Spodoptera frugiperda* cells or in *Trichoplusia* larvae. Sequences encoding DPP1 can be cloned into a non-essential region of the virus, such as the polyhedrin gene, and placed under control of the polyhedrin promoter. Successful insertion of DPP1 will render the polyhedrin gene inactive and produce recombinant virus lacking coat protein. The recombinant viruses can then be used to infect S. *frugiperda cells* or *Trichoplusia* larvae in which DPP1 can be expressed.

*Mammalian Expression Systems*

A number of viral-based expression systems can be used to express DPP1 in mammalian host cells. For example, if an adenovirus is used as an expression vector, sequences encoding DPP1 can be ligated into an adenovirus transcription/translation complex comprising the late promoter and tripartite leader sequence. Insertion in a non-essential E1 or E3 region of the viral genome can be used to obtain a viable virus which is capable of expressing DPP1 in infected host cells [Engelhard, 1994)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e.g.*, liposomes, polycationic amino polymers, or vesicles). Specific initiation signals also can be used to achieve more efficient translation of sequences encoding DPP1. Such signals include the ATG initiation codon and adjacent sequences. In cases where sequences encoding DPP1, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic.

*Host Cells*

A host cell strain can be chosen for its ability to modulate the expression of the inserted sequences or to process the expressed DPP1 in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g.*, CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express DPP1 can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced DPP1 sequences. Resistant clones of stably transformed cells can be proliferated using tissue culture techniques appropriate to the cell type. Any number of selection systems can be used to recover transformed cell lines. These include, but are not limited to, the herpes simplex virus thymidine kinase [Logan, (1984)] and adenine phosphoribosyltransferase [Wigler, (1977)] genes which can be employed in *tk*- or *aprt*- cells, respectively. Also, antimetabolite, antibiotic, or herbicide resistance can be used as the basis for selection. For example, *dhfr* confers resistance to methotrexate [Lowy, (1980)], *npt* confers resistance to the aminoglycosides, neomycin and G-418 [Wigler, (1980)], and *als* and *pat* confer resistance to chlorsulfuron and phosphinotricin acetyltransferase, respectively [Colbere-Garapin, 1981]. Additional selectable genes have been described. For example, *trpB* allows cells to utilize indole in place of tryptophan, or *hisD,* which allows cells to utilize histinol in place of histidine. Visible markers such as anthocyanins, β-glucuronidase and its substrate GUS, and luciferase and its substrate luciferin, can be used to identify transformants and to quantify the amount of transient or stable protein expression attributable to a specific vector system

*Detecting Polypeptide Expression*

Although the presence of marker gene expression suggests that a DPP1 polynucleotide is also present, its presence and expression may need to be confirmed. For example, if a sequence encoding DPP1 is inserted within a marker gene sequence, transformed cells containing sequences which encode DPP1 can be identified by the absence of marker gene function. Alternatively, a marker gene can be placed in tandem with a sequence encoding DPP1 under the control of a single promoter. Expression of the marker gene in response to induction or selection usually indicates expression of DPP1 polynucleotide.

Alternatively, host cells which contain a DPP1 polynucleotide and which express DPP1 can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding DPP1 can be detected by DNA-DNA or DNA-RNA hybridization or amplification using probes or fragments or fragments of polynucleotides encoding DPP1. Nucleic acid amplification-based assays involve the use of oligonucleotides selected from sequences encoding DPP1 to detect transformants which contain a DPP1 polynucleotide.

A variety of protocols for detecting and measuring the expression of DPP1, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on DPP1 can be used, or a competitive binding assay can be employed.

A wide variety of labels and conjugation techniques are known by those skilled in the art and can be used in various nucleic acid and amino acid assays. Means for producing labeled hybridization or PCR probes for detecting sequences related to polynucleotides encoding DPP1 include oligolabeling, nick translation, end-labeling, or PCR amplification using a labeled nucleotide. Alternatively, sequences encoding DPP1 can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with DPP1 polynucleotides can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing DPP1 polynucleotides can be designed to contain signal sequences which direct secretion of soluble DPP1 through a prokaryotic or eukaryotic cell membrane or which direct the membrane insertion of membrane-bound DPP 1.

As discussed above, other constructions can be used to join a sequence encoding DPP1 to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor XA or enterokinase (Invitrogen, San Diego, CA) between the purification domain and DPP1 also can be used to facilitate purification. One such expression vector provides for expression of a fusion protein containing DPP1 and 6 histidine residues preceding a thioredoxin or an enterokinase cleavage site. The histidine residues facilitate purification by IMAC (immobilized metal ion affinity chromatography) Maddox, (1983)], while the enterokinase cleavage site provides a means for purifying DPP1 from the fusion protein [Porath, (1992)].

*Chemical Synthesis*

Sequences encoding DPP1 can be synthesized, in whole or in part, using chemical methods well known in the art. Alternatively, DPP1 itself can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin Elmer). Optionally, fragments of DPP1 can be separately synthesized and combined using chemical methods to produce a full-length molecule.

The newly synthesized peptide can be substantially purified by preparative high performance liquid chromatography. The composition of a synthetic DPP1 can be confirmed by amino acid analysis or sequencing. Additionally, any portion of the amino acid sequence of DPP1 can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce DPP1 polynucleotides possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences referred to herein can be engineered using methods generally known in the art to alter DPP1 polynucleotides for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*DPP1 Analogs*

One general class of DPP1 analogs are variants having an amino acid sequence that is a mutation of the amino acid sequence disclosed herein. Another general class of DPP1 analogs is provided by anti-idiotype antibodies, and fragments thereof, as described below. Moreover, recombinant antibodies comprising anti-idiotype variable domains can be used as analogs (see, for example, [Monfardini et al., (1996)]). Since the variable domains of anti-idiotype DPP1 antibodies mimic DPP1, these domains can provide DPP1 enzymatic activity. Methods of producing anti-idiotypic catalytic antibodies are known to those of skill in the art [Joron et al., (1992), Friboulet et al. (1994), Avalle et al., (1998)].

Another approach to identifying DPP1 analogs is provided by the use of combinatorial libraries. Methods for constructing and screening phage display and other combinatorial libraries are provided, for example, by [Kay et al., Phage Display of Peptides and Proteins (Academic Press 1996), U.S. 5,783,384, U.S. 5,747,334, and U.S. 5,723,323.

One illustrative in vitro use of DPP1 and its analogs is the production of labeled peptides from a labeled protein substrate. Proteases can also be used in detergents and cleaning solutions. For example, serine proteases are used in solutions to clean and to disinfect contact lenses (see, for example, [U.S. 5,985,629]). Another use for a serine protease is in the formulation of vaccines (see, for example, [U.S. 5,885,814]). Those of skill in the art can devise other uses for molecules having DPP1 activity.

*Antibodies*

Any type of antibody known in the art can be generated to bind specifically to an epitope of DPP 1.

"Antibody" as used herein includes intact immunoglobulin molecules, as well as fragments thereof, such as Fab, $F(ab')_2$, and Fv, which are capable of binding an epitope of DPP1. Typically, at least 6, 8, 10, or 12 contiguous amino acids are required to form an epitope. However, epitopes which involve non-contiguous amino acids may require more, *e.g.*, at least 15, 25, or 50 amino acid. An antibody which specifically binds to an epitope of DPP1 can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the DPP1 immunogen.

Typically, an antibody which specifically binds to DPP1 provides a detection signal at least 5-, 10-, or 20-fold higher than a detection signal provided with other proteins when used in an immunochemical assay. Preferably, antibodies which specifically bind to DPP1 do not detect other proteins in immunochemical assays and can immunoprecipitate DPP1 from solution.

DPP1 can be used to immunize a mammal, such as a mouse, rat, rabbit, guinea pig, monkey, or human, to produce polyclonal antibodies. If desired, DPP1 can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (*e.g.*, aluminum hydroxide), and surface active substances (*e.g.*, lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to DPP1 can be prepared using any technique which provides for the production of antibody molecules by continuous cell lines in culture. These techniques include, but are not limited to, the hybridoma technique, the human B-cell hybridoma technique, and the EBV-hybridoma technique [Roberge, (1995)].

In addition, techniques developed for the production of "chimeric antibodies", the splicing of mouse antibody genes to human antibody genes to obtain a molecule with appropriate antigen specificity and biological activity, can be used. Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Antibodies which specifically bind to DPP1 can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to DPP1. Antibodies with related specificity, but of distinct idiotypic composition, can be generated by chain shuffling from random combinatorial immunoglobin libraries. Single-chain antibodies also can be constructed using a DNA amplification method, such as PCR, using hybridoma cDNA as a template. Single-chain antibodies can be mono- or bispecific, and can be bivalent or tetravalent. Construction of tetravalent, bispecific single-chain antibodies is taught. A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology.

Antibodies which specifically bind to DPP1 also can be produced by inducing *in vivo* production in the lymphocyte population or by screening immunoglobulin libraries or panels of highly specific binding reagents. Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which DPP1 is bound. The bound antibodies can then be eluted from the column using a buffer with a high salt concentration.

*Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of DPP1 gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters.

Modifications of DPP1 gene expression can be obtained by designing antisense oligonucleotides which will form duplexes to the control, 5', or regulatory regions of the DPP1 gene. Oligonucleotides derived from the transcription initiation site, *e.g.*, between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Nicholls, (1993)]. An antisense oligonucleotide also can be designed to block translation of mRNA by preventing the transcript from binding to ribosomes.

Precise complementarity is not required for successful complex formation between an antisense oligonucleotide and the complementary sequence of a DPP1 polynucleotide. Antisense oligonucleotides which comprise, for example, 2, 3, 4, or 5 or more stretches of contiguous nucleotides which are precisely complementary to a DPP1 polynucleotide, each separated by a stretch of contiguous nucleotides which are not complementary to adjacent DPP1 nucleotides, can provide sufficient targeting specificity for DPP1 mRNA. Preferably, each stretch of complement-tary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular DPP1 polynucleotide sequence. Antisense oligonucleotides can be modified without affecting their ability to hybridize to a DPP1 polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art.

*Ribozymes*

Ribozymes are RNA molecules with catalytic activity [Uhlmann, (1987)]. Ribozymes can be used to inhibit gene function by cleaving an RNA sequence, as is known in the art. The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences. The coding sequence of a DPP1 polynucleotide can be used to generate ribozymes which will specifically bind to mRNA transcribed from a DPP1 polynucleotide. Methods of designing and constructing ribozymes which can cleave other RNA molecules in trans in a highly sequence specific manner have been developed and described in the art. For example, the cleavage activity of ribozymes can be targeted to specific RNAs by engineering a discrete "hybridization" region into the ribozyme. The hybridization region contains a sequence complementary to the target RNA and thus specifically hybridizes with the target RNA.

Specific ribozyme cleavage sites within a DPP1 RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate DPP1 RNA targets also can be evaluated by testing accessibility to hybridization with complementary oligonucleotides using ribonuclease protection assays. The nucleotide sequences shown in SEQ ID NO: 1 and its complement provide sources of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease DPP1 expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells (U.S. 5,641,673). Ribozymes also can be engineered to provide an additional level of regulation, so that destruction of mRNA occurs only when both a ribozyme and a target gene are induced in the cells.

**Screening / Screening Assays**

*Regulators*

Regulators as used herein, refer to compounds that affect the activity of DPP1 in vivo and/or in vitro. Regulators can be agonists and antagonists of DPP1 polypeptide and can be compounds that exert their effect on the DPP1 activity via the enzymatic activity, expression, post-translational modifications or by other means. Agonists of DPP1 are molecules which, when bound to DPP1, increase or prolong the activity of DPP1. Agonists of DPP1 include proteins, nucleic acids, carbohydrates, small molecules, or any other molecule which activate DPP1. Antagonists of DPP1 are molecules which, when bound to DPP1, decrease the amount or the duration of the activity of DPP1. Antagonists include proteins, nucleic acids, carbohydrates, antibodies, small molecules, or any other molecule which decrease the activity of DPP 1.

The term "modulate", as it appears herein, refers to a change in the activity of DPP1 polypeptide. For example, modulation may cause an increase or a decrease in enzymatic activity, binding characteristics, or any other biological, functional, or immunological properties of DPP1.

As used herein, the terms "specific binding" or "specifically binding" refer to that interaction between a protein or peptide and an agonist, an antibody, or an antagonist. The interaction is dependent upon the presence of a particular structure of the protein recognized by the binding molecule (i.e., the antigenic determinant or epitope). For example, if an antibody is specific for epitope "A" the presence of a polypeptide containing the epitope A, or the presence of free unlabeled A, in a reaction containing free labeled A and the antibody will reduce the amount of labeled A that binds to the antibody.

The invention provides methods (also referred to herein as "screening assays") for identifying compounds which can be used for the treatment of diseases related to DPP1. The methods entail the identification of candidate or test compounds or agents (e.g., peptides, peptidomimetics, small molecules or other molecules) which bind to DPP1 and/or have a stimulatory or inhibitory effect on the biological activity of DPP1 or its expression and then determining which of these compounds have an effect on symptoms or diseases related to DPP1 in an *in vivo* assay.

Candidate or test compounds or agents which bind to DPP1 and/or have a stimulatory or inhibitory effect on the activity or the expression of DPP1 are identified either in assays that employ cells which express DPP1 (cell-based assays) or in assays with isolated DPP1 (cell-free assays). The various assays can employ a variety of variants of DPP1 (e.g., full-length DPP1, a biologically active fragment of DPP 1, or a fusion protein which includes all or a portion of DPP1). Moreover, DPP1 can be derived from any suitable mammalian species (e.g., human DPP1, rat DPP1 or murine DPP1). The assay can be a binding assay entailing direct or indirect measurement of the binding of a test compound or a known DPP1 ligand to DPP1. The assay can also be an activity assay entailing direct or indirect measurement of the activity of DPP1. The assay can also be an expression assay entailing direct or indirect measurement of the expression of DPP1 mRNA or DPP1 protein. The various screening assays are combined with an *in vivo* assay entailing measuring the effect of the test compound on the symptoms of diseases related to DPP1.

The present invention includes biochemical, cell free assays that allow the identification of inhibitors and agonists of proteases suitable as lead structures for pharmacological drug development. Such assays involve contacting a form of DPP1 (e.g., full-length DPP1, a biologically active fragment of DPP1, or a fusion protein comprising all or a portion of DPP1) with a test compound and determining the ability of the test compound to act as an antagonist (preferably) or an agonist of the enzymatic activity of DPP1.

The activity of DPP1 molecules of the present invention can be measured using a variety of assays that measure DPP1 activity. For example, DPP1 enzyme activity can be assessed by a standard in vitro serine/metallo/... protease assay (see, for example, [U.S. 5,057,414]). Those of skill in the art are aware of a variety of substrates suitable for in vitro assays, such as SucAla-Ala-Pro-Phe-pNA, fluorescein mono-p-guanidinobenzoate hydrochloride, benzyloxycarbonyl-L-Arginyl-S-benzylester, Nalpha-Benzoyl-L-arginine ethyl ester hydrochloride, and the like. In addition, protease assay kits available from commercial sources, such as Calbiochem™ (San Diego, Calif.). For general references, see Barrett (Ed.), Methods in Enzymology, Proteolytic Enzymes: Serine and Cysteine Peptidases (Academic Press Inc. 1994), and Barrett et al., (Eds.), Handbook of Proteolytic Enzymes (Academic Press Inc. 1998).

Solution in vitro assays can be used to identify a DPP1 substrate or inhibitor. Solid phase systems can also be used to identify a substrate or inhibitor of a DPP1 polypeptide. For example, a DPP1 polypeptide or DPP1 fusion protein can be immobilized onto the surface of a receptor chip of a commercially available biosensor instrument (BIACORE, Biacore AB; Uppsala, Sweden). The use of this instrument is disclosed, for example, by [Karlsson, (1991), and Cunningham and Wells, (1993)].

In brief, a DPP1 polypeptide or fusion protein is covalently attached, using amine or sulfhydryl chemistry, to dextran fibers that are attached to gold film within a flow cell. A test sample is then passed through the cell. If a DPP1 substrate or inhibitor is present in the sample, it will bind to the immobilized polypeptide or fusion protein, causing a change in the refractive index of the medium, which is detected as a change in surface plasmon resonance of the gold film. This system allows the determination on- and off-rates, from which binding affinity can be calculated, and assessment of the stoichiometry of binding, as well as the kinetic effects of DPP1 mutation. This system can also be used to examine antibody-antigen interactions, and the interactions of other complement/anti-complement pairs.

In one embodiment, the invention provides assays for screening candidate or test compounds which bind to or modulate the activity of DPP1. Such assays can employ full-length DPP1, a biologically active fragment of DPP1, or a fusion protein which includes all or a portion of DPP1. As described in greater detail below, the test compound can be obtained by any suitable means, e.g., from conventional compound libraries.

Determining the ability of the test compound to modulate the activity of DPP1 can be accomplished, for example, by determining the ability of DPP1 to bind to or interact with a target molecule. The target molecule can be a molecule with which DPP1 binds or interacts with in nature. The target molecule can be a component of a signal transduction pathway which facilitates transduction of an extracellular signal. The target DPP1 molecule can be, for example, a second intracellular protein which has catalytic activity or a protein which facilitates the association of downstream signaling molecules with DPP1.

Determining the ability of DPP1 to bind to or interact with a target molecule can be accomplished by one of the methods described above for determining direct binding. In one embodiment, determining the ability of a polypeptide of the invention to bind to or interact with a target molecule can be accomplished by determining the activity of the target molecule. For example, the activity of the target molecule can be determined by detecting induction of a cellular second messenger of the target (e.g., intracellular $Ca^{2+}$, diacylglycerol, $IP_3$, etc.), detecting catalytic/enzymatic activity of the target on an appropriate substrate, detecting the induction of a reporter gene (e.g., a regulatory element that is responsive to a polypeptide of the invention operably linked to a nucleic acid encoding a detectable marker, e.g., luciferase), or detecting a cellular response.

In various embodiments of the above assay methods of the present invention, it may be desirable to immobilize DPP1 (or a DPP1 target molecule) to facilitate separation of complexed from uncomplexed forms of one or both of the proteins, as well as to accommodate automation of the assay. Binding of a test compound to DPP1, or interaction of DPP1 with a target molecule in the presence and absence of a candidate compound, can be accomplished in any vessel suitable for containing the reactants. Examples of such vessels include microtitre plates, test tubes, and microcentrifuge tubes. In one embodiment, a fusion protein can be provided which adds a domain that allows one or both of the proteins to be bound to a matrix. For example, glutathione-S-transferase (GST) fusion proteins or glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed target protein or DPP1, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of DPP1 can be determined using standard techniques.

Other techniques for immobilizing proteins on matrices can also be used in the screening assays of the invention. For example, either DPP1 or its target molecule can be immobilized utilizing conjugation of biotin and streptavidin. Biotinylated polypeptide of the invention or target molecules can be prepared from biotin-NHS (N-hydroxy-succinimide) using techniques well known in the art (e.g., biotinylation kit, Pierce Chemicals; Rockford, Ill.), and immobilized in the wells of streptavidin-coated plates (Pierce Chemical). Alternatively, antibodies reactive with DPP1 or target molecules but which do not interfere with binding of the polypeptide of the invention to its target molecule can be derivatized to the wells of the plate, and unbound target or polypeptide of the invention trapped in the wells by antibody conjugation. Methods for detecting such complexes, in addition to those described above for the GST-immobilized complexes, include immunodetection of complexes using antibodies reactive with DPP1 or target molecule, as well as enzyme-linked assays which rely on detecting an enzymatic activity associated with DPP1 or target molecule.

Another technique for drug screening which may be used provides for high throughput screening of compounds having suitable binding affinity to the protein of interest as described in published PCT application WO84/03564. In this method, large numbers of different small test compounds are synthesized on a solid substrate, such as plastic pins or some other surface. The test compounds are reacted with DPP1, or fragments thereof, and washed. Bound DPP1 is then detected by methods well known in the art. Purified DPP1 can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding DPP1 specifically compete with a testcompound for binding DPP1. In this manner, antibodies can be used to detect the presence of any peptide which shares one or more antigenic determinants with DPP 1.

The screening assay can also involve monitoring the expression of DPP1. For example, regulators of expression of DPP1 can be identified in a method in which a cell is contacted with a candidate compound and the expression of DPP1 protein or mRNA in the cell is determined. The level of expression of DPP1 protein or mRNA the presence of the candidate compound is compared to the level of expression of DPP1 protein or mRNA in the absence of the candidate compound. The candidate compound can then be identified as a regulator of expression of DPP1 based on this comparison. For example, when expression of DPP1 protein or mRNA protein is greater (statistically significantly greater) in the presence of the candidate compound than in its absence, the candidate compound is identified as a stimulator of DPP1 protein or mRNA expression. Alternatively, when expression of DPP1 protein or mRNA is less (statistically significantly less) in the presence of the candidate compound than in its absence, the candidate compound is identified as an inhibitor of DPP1 protein or mRNA expression. The level of DPP1 protein or mRNA expression in the cells can be determined by methods described below.

*Binding Assays*

For binding assays, the test compound is preferably a small molecule which binds to and occupies the active site of DPP1 polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known DPP1 proteases and analogues or derivatives thereof.

In binding assays, either the test compound or the DPP1 polypeptide can comprise a detectable label, such as a fluorescent, radioisotopic, chemiluminescent, or enzymatic label, such as horseradish peroxidase, alkaline phosphatase, or luciferase. Detection of a test compound which is bound to DPP1 polypeptide can then be accomplished, for example, by direct counting of radioemmission, by scintillation counting, or by determining conversion of an appropriate substrate to a detectable product. Alternatively, binding of a test compound to a DPP1 polypeptide can be determined without labeling either of the interactants. For example, a microphysiometer can be used to detect binding of a test compound with a DPP1 polypeptide. A microphysiometer (*e.g.*, Cytosensor™) is an analytical instrument that measures the rate at which a cell acidifies its environment using a light-addressable potentiometric sensor (LAPS). Changes in this acidification rate can be used as an indicator of the interaction between a test compound and DPP1 [Haseloff, (1988)].

Determining the ability of a test compound to bind to DPP1 also can be accomplished using a technology such as real-time Bimolecular Interaction Analysis (BIA) [McConnell, (1992); Sjolander, (1991)]. BIA is a technology for studying biospecific interactions in real time, without labeling any of the interactants (*e.g.*, BIAcore™). Changes in the optical phenomenon surface plasmon resonance (SPR) can be used as an indication of real-time reactions between biological molecules.

In yet another aspect of the invention, a DPP1-like polypeptide can be used as a "bait protein" in a two-hybrid assay or three-hybrid assay [Szabo, (1995); U.S. 5,283,317), to identify other proteins which bind to or interact with DPP1 and modulate its activity.

The two-hybrid system is based on the modular nature of most transcription factors, which consist of separable DNA-binding and activation domains. Briefly, the assay utilizes two different DNA constructs. For example, in one construct, polynucleotide encoding DPP1 can be fused to a polynucleotide encoding the DNA binding domain of a known transcription factor (*e.g.*, GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g.*, LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with DPP 1.

It may be desirable to immobilize either the DPP1 (or polynucleotide) or the test compound to facilitate separation of the bound form from unbound forms of one or both of the interactants, as well as to accommodate automation of the assay. Thus, either the DPP1-like polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach DPP1-like polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to DPP1 (or a polynucleotide encoding for DPP1) can be accomplished in any vessel suitable for containing the reactants. Examples of such vessels include microtiter plates, test tubes, and microcentrifuge tubes.

In one embodiment, DPP1 is a fusion protein comprising a domain that allows binding of DPP1 to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed DPP1; the mixture is then incubated under conditions conducive to complex formation (*e.g.*, at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either DPP1 (or a polynucleotide encoding DPP1) or a test compound can be immobilized utilizing conjugation of biotin and streptavidin. Biotinylated DPP1 (or a polynucleotide encoding biotinylated DPP1) or test compounds can be prepared from biotin-NHS (N-hydroxysuccinimide) using techniques well known in the art (*e.g.*, biotinylation kit, Pierce Chemicals, Rockford, Ill.) and immobilized in the wells of streptavidin-coated plates (Pierce Chemical). Alternatively, antibodies which specifically bind to DPP1, polynucleotide, or a test compound, but which do not interfere with a desired binding site, such as the active site of DPP1, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to DPP1 polypeptide or test compound, enzyme-linked assays which rely on detecting an activity of DPP1 polypeptide, and SDS gel electrophoresis under non-reducing conditions.

Screening for test compounds which bind to a DPP1 polypeptide or polynucleotide also can be carried out in an intact cell. Any cell which comprises a DPP1 polypeptide or polynucleotide can be used in a cell-based assay system. A DPP1 polynucleotide can be naturally occurring in the cell or can be introduced using techniques such as those described above. Binding of the test compound to DPP1 or a polynucleotide encoding DPP1 is determined as described above.

*Functional Assays*

Test compounds can be tested for the ability to increase or decrease DPP1 activity of a DPP1 polypeptide. The DPP1 activity can be measured, for example, using methods described in the specific examples, below. DPP1 activity can be measured after contacting either a purified DPP1 or an intact cell with a test compound. A test compound which decreases DPP1 activity by at least about 10, preferably about 50, more preferably about 75, 90, or 100% is identified as a potential agent for decreasing DPP1 activity. A test compound which increases DPP1 activity by at least about 10, preferably about 50, more preferably about 75, 90, or 100% is identified as a potential agent for increasing DPP1 activity.

*Gene Expression*

In another embodiment, test compounds which increase or decrease DPP1 gene expression are identified. As used herein, the term "correlates with expression of a polynucleotide" indicates that the detection of the presence of nucleic acids, the same or related to a nucleic acid sequence encoding DPP1, by northern analysis or realtime PCR is indicative of the presence of nucleic acids encoding DPP1 in a sample, and thereby correlates with expression of the transcript from the polynucleotide encoding DPP1. The term "microarray", as used herein, refers to an array of distinct polynucleotides or oligonucleotides arrayed on a substrate, such as paper, nylon or any other type of membrane, filter, chip, glass slide, or any other suitable solid support. A DPP1 polynucleotide is contacted with a test compound, and the expression of an RNA or polypeptide product of DPP1 polynucleotide is determined. The level of expression of appropriate mRNA or polypeptide in the presence of the test compound is compared to the level of expression of mRNA or polypeptide in the absence of the test compound. The test compound can then be identified as a regulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of DPP1 mRNA or polypeptide expression in the cells can be determined by methods well known in the art for detecting mRNA or polypeptide. Either qualitative or quantitative methods can be used. The presence of polypeptide products of DPP1 polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labelled amino acids into DPP1.

Such screening can be carried out either in a cell-free assay system or in an intact cell. Any cell which expresses DPP1 polynucleotide can be used in a cell-based assay system. The DPP1 polynucleotide can be naturally occurring in the cell or can be introduced using techniques such as those described above. Either a primary culture or an established cell line can be used.

*Test Compounds*

Suitable test compounds for use in the screening assays of the invention can be obtained from any suitable source, e.g., conventional compound libraries. The test compounds can also be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds [Lam, (1997)]. Examples of methods for the synthesis of molecular libraries can be found in the art. Libraries of compounds may be presented in solution or on beads, bacteria, spores, plasmids or phage.

*Modeling of Regulators*

Computer modeling and searching technologies permit identification of compounds, or the improvement of already identified compounds, that can modulate DPP1 expression or activity. Having identified such a compound or composition, the active sites or regions are identified. Such sites might typically be the enzymatic active site, regulator binding sites, or ligand binding sites. The active site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.
Next, the three dimensional geometric structure of the active site is determined. This can be done by known methods, including X-ray crystallography, which can determine a complete molecular structure. On the other hand, solid or liquid phase NMR can be used to determine certain intramolecular distances. Any other experimental method of structure determination can be used to obtain partial or complete geometric structures. The geometric structures may be measured with a complexed ligand, natural or artificial, which may increase the accuracy of the active site structure determined.
If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.
Finally, having determined the structure of the active site, either experimentally, by modeling, or by a combination, candidate modulating compounds can be identified by searching databases containing compounds along with information on their molecular structure. Such a search seeks compounds having structures that match the determined active site structure and that interact with the groups defining the active site. Such a search can be manual, but is preferably computer assisted. These compounds found from this search are potential DPP1 modulating compounds.
Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the active site structure of the compound to determine if an improved fit or interaction results. In this manner systematic variations in composition, such as by varying side groups, can be quickly evaluated to obtain modified modulating compounds or ligands of improved specificity or activity.

**Therapeutic Indications and Methods**

It was found by the present applicant that DPP1 is expressed in various human tissues.

*Neurology*

CNS disorders include disorders of the central nervous system as well as disorders of the peripheral nervous system.
CNS disorders include, but are not limited to brain injuries, cerebrovascular diseases and their consequences, Parkinson's disease, corticobasal degeneration, motor neuron disease, dementia, including ALS, multiple sclerosis, traumatic brain injury, stroke, post-stroke, post-traumatic brain injury, and small-vessel cerebrovascular disease. Dementias, such as Alzheimer's disease, vascular dementia, dementia with Lewy bodies, frontotemporal dementia and Parkinsonism linked to chromosome 17, frontotemporal dementias, including Pick's disease, progressive nuclear palsy, corticobasal degeneration, Huntington's disease, thalamic degeneration, Creutzfeld-Jakob dementia, HIV dementia, schizophrenia with dementia, and Korsakoff's psychosis, within the meaning of the definition are also considered to be CNS disorders.
Similarly, cognitive-related disorders, such as mild cognitive impairment, age-associated memory impairment, age-related cognitive decline, vascular cognitive impairment, attention deficit disorders, attention deficit hyperactivity disorders, and memory disturbances in children with learning disabilities are also considered to be CNS disorders.
Pain, within the meaning of this definition, is also considered to be a CNS disorder. Pain can be associated with CNS disorders, such as multiple sclerosis, spinal cord injury, sciatica, failed back surgery syndrome, traumatic brain injury, epilepsy, Parkinson's disease, post-stroke, and vascular lesions in the brain and spinal cord (e.g., infarct, hemorrhage, vascular malformation). Non-central neuropathic pain includes that associated with post mastectomy pain, phantom feeling, reflex sympathetic dystrophy (RSD), trigeminal neuralgiaradioculopathy, post-surgical pain, HIV/AIDS related pain, cancer pain, metabolic neuropathies (e.g., diabetic neuropathy, vasculitic neuropathy secondary to connective tissue disease), paraneoplastic polyneuropathy associated, for example, with carcinoma of lung, or leukemia, or lymphoma, or carcinoma of prostate, colon or stomach, trigeminal neuralgia, cranial neuralgias, and post-herpetic neuralgia. Pain associated with peripheral nerve damage, central pain (i.e. due to cerebral ischemia) and various chronic pain i.e., lumbago, back pain (low back pain), inflammatory and/or rheumatic pain. Headache pain (for example, migraine with aura, migraine without aura, and other migraine disorders), episodic and chronic tension-type headache, tension-type like headache, cluster headache, and chronic paroxysmal hemicrania are also CNS disorders.

Visceral pain such as pancreatits, intestinal cystitis, dysmenorrhea, irritable Bowel syndrome, Crohn's disease, biliary colic, ureteral colic, myocardial infarction and pain syndromes of the pelvic cavity, e.g., vulvodynia, orchialgia, urethral syndrome and protatodynia are also CNS disorders.

Also considered to be a disorder of the nervous system are acute pain, for example postoperative pain, and pain after trauma.

The human DPP1 is highly expressed in the following brain tissues: spinal cord. The expression in brain tissues demonstrates that the human DPP1 or mRNA can be utilized to diagnose nervous system diseases. Additionally the activity of the human DPP1 can be modulated to treat nervous system diseases.

*Cardiovascular Disorders*

Heart failure is defined as a pathophysiological state in which an abnormality of cardiac function is responsible for the failure of the heart to pump blood at a rate commensurate with the requirement of the metabolizing tissue. It includes all forms of pumping failures such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina and asymptomatic ischemia.

Arrhythmias include all forms of atrial and ventricular tachyarrhythmias, atrial tachycardia, atrial flutter, atrial fibrillation, atrio-ventricular reentrant tachycardia, preexitation syndrome, ventricular tachycardia, ventricular flutter, ventricular fibrillation, as well as bradycardic forms of arrhythmias.

Hypertensive vascular diseases include primary as well as all kinds of secondary arterial hypertension, renal, endocrine, neurogenic, others. The genes may be used as drug targets for the treatment of hypertension as well as for the prevention of all complications arising from cardiovascular diseases.

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis is a cardiovascular disease in which the vessel wall is remodeled, compromising the lumen of the vessel. The atherosclerotic remodeling process involves accumulation of cells, both smooth muscle cells and monocyte/macrophage inflammatory cells, in the intima of the vessel wall. These cells take up lipid, likely from the circulation, to form a mature atherosclerotic lesion. Although the formation of these lesions is a chronic process, occurring over decades of an adult human life, the majority of the morbidity associated with atherosclerosis occurs when a lesion ruptures, releasing thrombogenic debris that rapidly occludes the artery. When such an acute event occurs in the coronary artery, myocardial infarction can ensue, and in the worst case, can result in death.

The formation of the atherosclerotic lesion can be considered to occur in five overlapping stages such as migration, lipid accumulation, recruitment of inflammatory cells, proliferation of vascular smooth muscle cells, and extracellular matrix deposition. Each of these processes can be shown to occur in man and in animal models of atherosclerosis, but the relative contribution of each to the pathology and clinical significance of the lesion is unclear.

Thus, a need exists for therapeutic methods and agents to treat cardiovascular pathologies, such as atherosclerosis and other conditions related to coronary artery disease.

Cardiovascular diseases include but are not limited to disorders of the heart and the vascular system like congestive heart failure, myocardial infarction, ischemic diseases of the heart, all kinds of atrial and ventricular arrhythmias, hypertensive vascular diseases, peripheral vascular diseases, and atherosclerosis.

Too high or too low levels of fats in the bloodstream, especially cholesterol, can cause long-term problems. The risk to develop atherosclerosis and coronary artery or carotid artery disease (and thus the risk of having a heart attack or stroke) increases with the total cholesterol level increasing. Nevertheless, extremely low cholesterol levels may not be healthy. Examples of disorders of lipid metabolism are hyperlipidemia (abnormally high levels of fats (cholesterol, triglycerides, or both) in the blood, may be caused by family history of hyperlipidemia), obesity, a high-fat diet, lack of exercise, moderate to high alcohol consumption, cigarette smoking, poorly controlled diabetes, and an underactive thyroid gland), hereditary hyperlipidemias (type I hyperlipoproteinemia (familial hyperchylomicronemia), type II hyperlipoproteinemia (familial hypercholesterolemia), type III hyperlipoproteinemia, type IV hyperlipoproteinemia, or type V hyperlipoproteinemia), hypolipoproteinemia, lipidoses (caused by abnormalities in the enzymes that metabolize fats), Gaucher's disease, Niemann-Pick disease, Fabry's disease, Wolman's disease, cerebrotendinous xanthomatosis, sitosterolemia, Refsum's disease, or Tay-Sachs disease.

Kidney disorders may lead to hypertension or hypotension. Examples for kidney problems possibly leading to hypertension are renal artery stenosis, pyelonephritis, glomerulonephritis, kidney tumors, polycistic kidney disease, injury to the kidney, or radiation therapy affecting the kidney. Excessive urination may lead to hypotension.

The human DPP1 is highly expressed in the following cardiovascular related tissues: heart myocardial infarction, heart myocardial infarction, heart atrium (right), heart atrium (left), heart ventricle (right), heart apex, Purkinje fibers, coronary artery smooth muscle primary cells, aortic smooth muscle cells, pulmonary artery smooth muscle cells, aortic endothel cells, HUVEC cells, pulmonary artery endothel cells, iliac artery endothel cells, liver liver cirrhosis, liver lupus disease, liver tumor, adipose, adipose, fetal kidney, kidney, kidney, kidney tumor, renal epithelial cells, HEK 293 cells. Expression in the above mentioned tissues and in particular the differential expression between diseased tissue heart myocardial infarction and healthy tissue demonstrates that the human DPP1 or mRNA can be utilized to diagnose of cardiovascular diseases. Additionally the activity of the human DPP1 can be modulated to treat cardiovascular diseases.

The human DPP1 is highly expressed in liver tissues: liver liver cirrhosis, liver lupus disease, liver tumor. Expression in liver tissues demonstrates that the human DPP1 or mRNA can be utilized to diagnose of dyslipidemia disorders as an cardiovascular disorder. Additionally the activity of the human DPP1 can be modulated to treat - but not limited to - dyslipidemia disorders.

The human DPP1 is highly expressed in adipose tissues. Expression in adipose demonstrates that the human DPP1 or mRNA can be utilized to diagnose of dyslipidemia diseases as an cardiovascular disorder. Additionally the activity of the human DPP1 can be modulated to treat - but not limited to - dyslipidemia diseases.

The human DPP1 is highly expressed in kidney tissues : fetal kidney, kidney, kidney, kidney tumor, HEK 293 cells. Expression in kidney tissues demonstrates that the human DPP1 or mRNA can be utilized to diagnose of blood pressure disorders as an cardiovascular disorder. Additionally the activity of the human DPP1 can be modulated to treat - but not limited to - blood pressure disorders as hypertension or hypotension.

*Hematological Disorders*

Hematological disorders comprise diseases of the blood and all its constituents as well as diseases of organs and tissues involved in the generation or degradation of all the constituents of the blood. They include but are not limited to 1) Anemias, 2) Myeloproliferative Disorders, 3) Hemorrhagic Disorders, 4) Leukopenia, 5) Eosinophilic Disorders, 6) Leukemias, 7) Lymphomas, 8) Plasma Cell Dyscrasias, 9) Disorders of the Spleen in the course of hematological disorders. Disorders according to 1) include, but are not limited to anemias due to defective or deficient hem synthesis, deficient erythropoiesis. Disorders according to 2) include, but are not limited to polycythemia vera, tumor-associated erythrocytosis, myelofibrosis, thrombocythemia. Disorders according to 3) include, but are not limited to vasculitis, thrombocytopenia, heparin-induced thrombocytopenia, thrombotic thrombocytopenic purpura, hemolytic-uremic syndrome, hereditary and acquired disorders of platelet function, hereditary coagulation disorders. Disorders according to 4) include, but are not limited to neutropenia, lymphocytopenia. Disorders according to 5) include, but are not limited to hypereosinophilia, idiopathic hypereosinophilic syndrome. Disorders according to 6) include, but are not limited to acute myeloic leukemia, acute lymphoblastic leukemia, chronic myelocytic leukemia, chronic lymphocytic leukemia, myelodysplastic syndrome. Disorders according to 7) include, but are not limited to Hodgkin's disease, non-Hodgkin's lymphoma, Burkitt's lymphoma, mycosis fungoides cutaneous T-cell lymphoma. Disorders according to 8) include, but are not limited to multiple myeloma, macroglobulinemia, heavy chain diseases. In extension of the preceding idiopathic thrombocytopenic purpura, iron deficiency anemia, megaloblastic anemia (vitamin B12 deficiency), aplastic anemia, thalassemia, malignant lymphoma bone marrow invasion, malignant lymphoma skin invasion, hemolytic uremic syndrome, giant platelet disease are considered to be hematological diseases too.

The human DPP1 is highly expressed in the following tissues of the hematological system: leukocytes (peripheral blood), Jurkat (T-cells), Raji (B-cells), bone marrow, bone marrow stromal cells, T-cells peripheral blood CD8+, monocytes peripheral blood CD14+, neutrophils peripheral blood, spleen, spleen liver cirrhosis. The expression in the above mentioned tissues and in particular the differential expression between diseased tissue spleen liver cirrhosis and healthy tissue spleen demonstrates that the human DPP1 or mRNA can be utilized to diagnose of hematological diseases. Additionally the activity of the human DPP1 can be modulated to treat hematological disorders.

*Gastrointestinal and Liver Diseases*

Gastrointestinal diseases comprise primary or secondary, acute or chronic diseases of the organs of the gastrointestinal tract which may be acquired or inherited, benign or malignant or metaplastic, and which may affect the organs of the gastrointestinal tract or the body as a whole. They comprise but are not limited to 1) disorders of the esophagus like achalasia, vigoruos achalasia, dysphagia, cricopharyngeal incoordination, pre-esophageal dysphagia, diffuse esophageal spasm, globus sensation, Barrett's metaplasia, gastroesophageal reflux, 2) disorders of the stomach and duodenum like functional dyspepsia, inflammation of the gastric mucosa, gastritis, stress gastritis, chronic erosive gastritis, atrophy of gastric glands, metaplasia of gastric tissues, gastric ulcers, duodenal ulcers, neoplasms of the stomach, 3) disorders of the pancreas like acute or chronic pancreatitis, insufficiency of the exocrinic or endocrinic tissues of the pancreas like steatorrhea, diabetes, neoplasms of the exocrine or endocrine pancreas like 3.1) multiple endocrine neoplasia syndrome, ductal adenocarcinoma, cystadenocarcinoma, islet cell tumors, insulinoma, gastrinoma, carcinoid tumors, glucagonoma, Zollinger-Ellison syndrome, Vipoma syndrome, malabsorption syndrome, 4) disorders of the bowel like chronic inflammatory diseases of the bowel, Crohn's disease, ileus, diarrhea and constipation, colonic inertia, megacolon, malabsorption syndrome, ulcerative colitis, 4.1) functional bowel disorders like irritable bowel syndrome, 4.2) neoplasms of the bowel like familial polyposis, adenocarcinoma, primary malignant lymphoma, carcinoid tumors, Kaposi's sarcoma, polyps, cancer of the colon and rectum.

Liver diseases comprise primary or secondary, acute or chronic diseases or injury of the liver which may be acquired or inherited, benign or malignant, and which may affect the liver or the body as a whole. They comprise but are not limited to disorders of the bilirubin metabolism, jaundice, syndroms of Gilbert's, Crigler-Najjar, Dubin-Johnson and Rotor; intrahepatic cholestasis, hepatomegaly, portal hypertension, ascites, Budd-Chiari syndrome, portal-systemic encephalopathy, fatty liver, steatosis, Reye's syndrome, liver diseases due to alcohol, alcoholic hepatitis or cirrhosis, fibrosis and cirrhosis, fibrosis and cirrhosis of the liver due to inborn errors of metabolism or exogenous substances, storage diseases, syndromes of Gaucher's, Zellweger's, Wilson's - disease, acute or chronic hepatitis, viral hepatitis and its variants, inflammatory conditions of the liver due to viruses, bacteria, fungi, protozoa, helminths; drug induced disorders of the liver, chronic liver diseases like primary sclerosing cholangitis, $alpha_1$-antitrypsin-deficiency, primary biliary cirrhosis, postoperative liver disorders like postoperative intrahepatic cholestasis, hepatic granulomas, vascular liver disorders associated with systemic disease, benign or malignant neoplasms of the liver, disturbance of liver metabolism in the new-born or prematurely born.

The human DPP1 is highly expressed in the following tissues of the gastroenterological system: esophagus tumor, stomach, stomach tumor, colon, small intestine, ileum tumor, liver liver cirrhosis, liver lupus disease, liver tumor, HEP G2 cells. The expression in the above mentioned tissues and in particular the differential expression between diseased tissue liver liver cirrhosis and healthy tissue liver, between diseased tissue liver lupus disease and healthy tissue liver demonstrates that the human DPP1 or mRNA can be utilized to diagnose of gastroenterological disorders. Additionally the activity of the human DPP1 can be modulated to treat gastroenterological disorders.

*Endocrine System and Hormones*

The endocrine system consists of a group of organs whose main function is to produce and secrete hormones directly into the bloodstream. The major organs of the endocrine system are the hypothalamus, the pituitary gland, thyroid gland, the parathyroid glands, the islets of the pancreas, the adrenal glands, the testes, and the ovaries.

The hypothalamus secretes several hormones that stimulate the pituitary: Some trigger the release of pituitary hormones; others suppress the release of pituitary hormones.

The pituitary gland coordinates many functions of the other endocrine glands, but some pituitary hormones have direct effects.

The insulin-secreting cells of the pancreas respond to glucose and fatty acids. Parathyroid cells respond to calcium and phosphate. The adrenal medulla (part of the adrenal gland) responds to direct stimulation by the parasympathetic nervous system.

When endocrine glands malfunction, hormone levels in the blood can become abnormally high or low, disrupting body functions. Many disorders are caused by malfunction of the endocrine system or hormones. Examples of such disorders are presented in the following.

Diabetes mellitus is a disorder in which blood levels of glucose are abnormally high because the body doesn't release or use insulin adequately.

People with type I diabetes mellitus (insulin-dependent diabetes) produce little or no insulin at all. In type I diabetes more than 90 percent of the insulin-producing cells (beta cells) of the pancreas are permanently destroyed. The resulting insulin deficiency is severe, and to survive, a person with type I diabetes must regularly inject insulin.

In type II diabetes mellitus (non-insulin-dependent diabetes) the body develops resistance to insulin effects, resulting in a relative insulin deficiency.

The pancreas has two major functions: to secrete fluid containing digestive enzymes into the duodenum and to secrete the hormones insulin and glucagon. Chronic pancreatitis is a longstanding inflammation of the pancreas. Eventually, the insulin-secreting cells of the pancreas may be destroyed, gradually leading to diabetes. An insulinoma is a rare type of pancreatic tumor that secretes insulin. The symptoms of an insulinoma result from low blood glucose levels. A gastrinoma is a pancreatic tumor that produces excessive levels of the hormone gastrin, which stimulates the stomach to secrete acid and enzymes, causing peptic ulcers. The excess gastrin secreted by the gastrinoma causes symptoms, called the Zollinger-Ellison syndrome. A glucagonoma is a tumor that produces the hormone glucagon, which raises the level of glucose in the blood and produces a distinctive rash.

Diabetes insipidus is a disorder in which insufficient levels of antidiuretic hormone cause excessive thirst (polydipsia) and excessive production of very dilute urine (polyuria). Diabetes insipidus results from the decreased production of antidiuretic hormone (vasopressin).

The body has two adrenal glands. The medulla of the adrenal glands secretes hormones such as adrenaline (epinephrine) that affect blood pressure, heart rate, sweating, and other activities also regulated by the sympathetic nervous system. The cortex secretes many different hormones, including corticosteroids (cortisone-like hormones), androgens (male hormones), and mineralocorticoids, which control blood pressure and the levels of salt and potassium in the body.

A diseases characterized by underactive adrenal glands is Addison's disease (adrenocortical insufficiency).

Several disorders are characterized by overactive Adrenal Glands. The causes can be changes in the adrenal glands themselves or overstimulation by the pituitary gland. Examples of these diseases are listed in the following.

Overproduction of androgenic steroids (testosterone and similar hormones, leads to virilization), overproduction of corticosteroids (causes could be tumors of the pituitary or the adrenal gland, results in Cushing's syndrome), Nelson's syndrome (developed by people who have both adrenal glands removed, characterized by an enlargement of the pituitary gland), Overproduction of aldosterone (hyperaldosteronism), Conn's syndrome (hyperaldosterism caused by a tumor), pheochromocytoma (a tumor that originating from the adrenal gland's chromaffin cells, causing overproduction of catecholamines),

The thyroid is a small gland located under the Adam's apple. It secretes thyroid hormones, which control the metabolic rate. The thyroid gland traps iodine and processes it into thyroid hormones. The euthyroid sick syndrome is characterized by lack of conversion of the T4 form of thyroid hormone to the T3 form. Hyperthyroidism (overactive thyroid gland, production of too much hormone) may have several causes. Thyroiditis (an inflammation of the thyroid gland), typically leads to a phase of hyperthyroidism. The inflammation may damage the thyroid gland, so that in later stages the disease is characterized by transient or permanent underactivity (hypothyroidism). Toxic thyroid nodules (adenomas) often produce thyroid hormone in large quantities. Toxic multinodular goiter (Plummer's disease) is a disorder in which there are many nodules. Graves' disease (toxic diffuse goiter) is believed to be caused by an antibody that stimulates the thyroid to produce too much thyroid hormone. In toxic nodular goiter, one or more nodules in the thyroid produce too much thyroid hormone and aren't under the control of thyroid-stimulating hormone. Secondary hyperthyroidism may (rarely) be caused by a pituitary tumor that secretes too much thyroid-stimulating hormone, by resistance of the pituitary to thyroid hormone, which results in the pituitary gland secreting too much thyroid-stimulating hormone, or by a hydatidiform mole in women. Thyroid storm is a sudden extreme overactivity of the thyroid gland is a life-threatening emergency requiring prompt treatment.

Hypothyroidism is a condition in which the thyroid gland is underactive and produces too little thyroid hormone. Very severe hypothyroidism is called myxedema. In Hashimoto's thyroiditis (autoimmune thyroiditis) the thyroid gland is often enlarged, and hypothyroidism results because the gland's functioning areas are gradually destroyed. Rarer causes of hypothyroidism include some inherited disorders which are caused by abnormalities of the enzymes in thyroid cells. In other rare disorders, either the hypothalamus or the pituitary gland fails to secrete enough of the hormone needed to stimulate normal thyroid function.

Other examples of Thyroiditis are silent lymphocytic thyroiditis, Hashimoto's thyroiditis, or subacute granulomatous thyroiditis.

Thyroid cancer is any one of four main types of malignancy of the thyroid: papillary, follicular, anaplastic, or medullary.

The pituitary is a pea-sized gland that sits in a bony structure (sella turcica) at the base of the brain. The sella turcica protects the pituitary but allows very little room for expansion. If the pituitary enlarges, it tends to push upward, often pressing on the areas of the brain that carry signals from the eyes, possibly resulting in headaches or impaired vision. The pituitary gland has two distinct parts: the anterior (front) and the posterior (back) lobes. The anterior lobe produces (secretes) hormones that ultimately control the function of the thyroid gland, adrenal glands, and reproductive organs (ovaries and testes); milk production (lactation) in the breasts; and overall body growth. It also produces hormones that cause the skin to darken and that inhibit pain sensations. The posterior lobe produces hormones that regulate water balance, stimulate the let-down of milk from the breasts in lactating women, and stimulate contractions of the uterus.

Examples for disorders of the pituitary gland are Empty Sella Syndrome; hypopituitarism (an underactive pituitary gland); acromegaly, which is excessive growth caused by oversecretion of growth hormone, which is almost always caused by a benign pituitary tumor (adenoma); galactorrhea, which is the production of breast milk in men or in women who aren't breastfeeding, in both sexes, the most common cause of galactorrhea is a prolactin-producing tumor (prolactinoma) in the pituitary gland.

The human DPP1 is highly expressed in the following tissues of the endocrinological system: thyroid, thyroid tumor. The expression in the above mentioned tissues demonstrates that the human DPP1 or mRNA can be utilized to diagnose of endocrinological disorders. Additionally the activity of the human DPP1 can be modulated to treat endocrinological disorders.

*Musculoskeletal Diseases*

Components of the musculoskeletal system are skeleton, muscles, tendons, ligaments, and other components of joints. Disorders of the musculoskeletal system often cause chronic pain and physical disability. They range from injures, infections, inflammation or other types of disorders. Examples of musculoskeletal disorders are presented in the following.

Examples are osteoporosis, postmenopausal osteoporosis, senile osteoporosis, secondary osteoporosis, idiopathic juvenile osteoporosis, Paget's disease of the bone, osteochondromas (osteocartilaginous exostoses), tumors of the bone (benign chondromas, chondroblastomas, chondromyxoid fibromas, osteoid osteomas, giant cell tumors of the bone, multiple myeloma, osteosarcoma (osteogenic sarcoma), fibrosarcomas and malignant fibrous histiocytomas, chondrosarcomas, Ewing's tumor (Ewing's sarcoma), malignant lymphoma of bone (reticulum cell sarcoma, metastatic tumors of the bone), osteoarthritis, and gout and Pseudogout.

Examples of disorders of joints and connective tissue are rheumatoid arthritis, psoriatic arthritis, discoid lupus erythematosus, systemic lupus erythematosus, scleroderma (systemic sclerosis), Sjögren's syndrome, connective tissue disease, polymyositis and dermatomyositis, relapsing polychondritis, vasculitis, polyarteritis nodosa, polymyalgia rheumatica, temporal arteritis, Wegener's granulomatosis, Reiter's syndrome, Behçet's syndrome, ankylosing spondylitis, or Charcot's joints (neuropathic joint disease).

Examples for bone and joint infections are osteomyelitis, and infectious arthritis.

Examples of disorders of muscles, bursas, and tendons are spasmodic torticollis, fibromyalgia syndromes (myofascial pain syndromes, fibromyositis), bursitis, tendinitis and tenosynovitis.

Foot problems are, for example ankle sprain, foot fractures, heel spurs, Sever's disease, posterior achilles tendon bursitis, anterior achilles tendon bursitis, posterior tibial neuralgia, pain in the ball of the foot (caused by damage to the nerves between the toes or to the joints between the toes and foot), onychomycosis, or nail discoloration.

The human DPP1 is highly expressed in the following muscle/skeleton tissues: skeletal muscle, cartilage, adipose, adipose, fetal adipose. The expression in muscle/skleleton tissues demonstrates that the human DPP1 or mRNA can be utilized to diagnose of diseases of the muscle/skeleton system. Additionally the activity of the human DPP1 can be modulated to treat those diseases.

*Cancer Disorders*

Cancer disorders within the scope of this definition comprise any disease of an organ or tissue in mammals characterized by poorly controlled or uncontrolled multiplication of normal or abnormal cells in that tissue and its effect on the body as a whole. Cancer diseases within the scope of the definition comprise benign neoplasms, dysplasias, hyperplasias as well as neoplasms showing metastatic growth or any other transformations like e.g. leukoplakias which often precede a breakout of cancer. Cells and tissues are cancerous when they grow more rapidly than normal cells, displacing or spreading into the surrounding healthy tissue or any other tissues of the body described as metastatic growth, assume abnormal shapes and sizes, show changes in their nucleocytoplasmatic ratio, nuclear polychromasia, and finally may cease. Cancerous cells and tissues may affect the body as a whole when causing paraneoplastic syndromes or if cancer occurs within a vital organ or tissue, normal function will be impaired or halted, with possible fatal results. The ultimate involvement of a vital organ by cancer, either primary or metastatic, may lead to the death of the mammal affected. Cancer tends to spread, and the extent of its spread is usually related to an individual's chances of surviving the disease. Cancers are generally said to be in one of three stages of growth: early, or localized, when a tumor is still confined to the tissue of origin, or primary site; direct extension, where cancer cells from the tumour have invaded adjacent tissue or have spread only to regional lymph nodes; or metastasis, in which cancer cells have migrated to distant parts of the body from the primary site, via the blood or lymph systems, and have established secondary sites of infection. Cancer is said to be malignant because of its tendency to cause death if not treated. Benign tumors usually do not cause death, although they may if they interfere with a normal body function by virtue of their location, size, or paraneoplastic side effects. Hence benign tumors fall under the definition of cancer within the scope of this definition as well. In general, cancer cells divide at a higher rate than do normal cells, but the distinction between the growth of cancerous and normal tissues is not so much the rapidity of cell division in the former as it is the partial or complete loss of growth restraint in cancer cells and their failure to differentiate into a useful, limited tissue of the type that characterizes the functional equilibrium of growth of normal tissue. Cancer tissues may express certain molecular receptors and probably are influenced by the host's susceptibility and immunity and it is known that certain cancers of the breast and prostate, for example, are considered dependent on specific hormones for their existence. The term "cancer" under the scope of the definition is not limited to simple benign neoplasia but comprises any other benign and malign neoplasia like 1) Carcinoma, 2) Sarcoma, 3) Carcinosarcoma, 4) Cancers of the blood-forming tissues, 5) tumors of nerve tissues including the brain, 6) cancer of skin cells. Cancer according to 1) occurs in epithelial tissues, which cover the outer body (the skin) and line mucous membranes and the inner cavitary structures of organs e.g. such as the breast, lung, the respiratory and gastrointestinal tracts, the endocrine glands, and the genitourinary system. Ductal or glandular elements may persist in epithelial tumors, as in adenocarcinomas like e.g. thyroid adenocarcinoma, gastric adenocarcinoma, uterine adenocarcinoma. Cancers of the pavement-cell epithelium of the skin and of certain mucous membranes, such as e.g. cancers of the tongue, lip, larynx, urinary bladder, uterine cervix, or penis, may be termed epidermoid or squamous-cell carcinomas of the respective tissues and are in the scope of the definition of cancer as well. Cancer according to 2) develops in connective tissues, including fibrous tissues, adipose (fat) tissues, muscle, blood vessels, bone, and cartilage like e.g. osteogenic sarcoma; liposarcoma, fibrosarcoma, synovial sarcoma. Cancer according to 3) is cancer that develops in both epithelial and connective tissue. Cancer disease within the scope of this definition may be primary or secondary, whereby primary indicates that the cancer originated in the tissue where it is found rather than was established as a secondary site through metastasis from another lesion. Cancers and tumor diseases within the scope of this definition may be benign or malign and may affect all anatomical structures of the body of a mammal. By example but not limited to they comprise cancers and tumor diseases of I) the bone marrow and bone marrow derived cells (leukemias), II) the endocrine and exocrine glands like e.g. thyroid, parathyroid, pituitary, adrenal glands, salivary glands, pancreas III) the breast, like e.g. benign or malignant tumors in the mammary glands of either a male or a female, the mammary ducts, adenocarcinoma, medullary carcinoma, comedo carcinoma, Paget's disease of the nipple, inflammatory carcinoma of the young woman, IV) the lung, V) the stomach, VI) the liver and spleen, VII) the small intestine, VIII) the colon, IX) the bone and its supportive and connective tissues like malignant or benign bone tumour, e.g. malignant osteogenic sarcoma, benign osteoma, cartilage tumors; like malignant chondrosarcoma or benign chondroma; bone marrow tumors like malignant myeloma or benign eosinophilic granuloma, as well as metastatic tumors from bone tissues at other locations of the body; X) the mouth, throat, larynx, and the esophagus, XI) the urinary bladder and the internal and external organs and structures of the urogenital system of male and female like ovaries, uterus, cervix of the uterus, testes, and prostate gland, XII) the prostate, XIII) the pancreas, like ductal carcinoma of the pancreas; XIV) the lymphatic tissue like lymphomas and other tumors of lymphoid origin, XV) the skin, XVI) cancers and tumor diseases of all anatomical structures belonging to the respiration and respiratory systems including thoracal muscles and linings, XVII) primary or secondary cancer of the lymph nodes XVIII) the tongue and of the bony structures of the hard palate or sinuses, XVIV) the mouth, cheeks, neck and salivary glands, XX) the blood vessels including the heart and their linings, XXI) the smooth or skeletal muscles and their ligaments and linings, XXII) the peripheral, the autonomous, the central nervous system including the cerebellum, XXIII) the adipose tissue.

The human DPP1 is highly expressed in the following cancer tissues: HUVEC cells, thyroid tumor, esophagus tumor, stomach tumor, ileum tumor, liver tumor, HEP G2 cells, Jurkat (T-cells), Raji (B-cells), HeLa cells (cervix tumor), uterus tumor, ovary tumor, breast tumor, prostate tumor, kidney tumor, HEK 293 cells. The expression in the above mentioned tissues and in particular the differential expression between diseased tissue thyroid tumor and healthy tissue thyroid, between diseased tissue esophagus tumor and healthy tissue esophagus, between diseased tissue stomach tumor and healthy tissue stomach, between diseased tissue ileum tumor and healthy tissue , between diseased tissue liver tumor and healthy tissue liver, between diseased tissue HEP G2 cells and healthy tissue liver, between diseased tissue Jurkat (T-cells) and healthy tissue T-cells peripheral blood CD4+, between diseased tissue Raji (B-cells) and healthy tissue B-cells peripheral blood CD19+, between diseased tissue HeLa cells (cervix tumor) and healthy tissue cervix, between diseased tissue uterus tumor and healthy tissue uterus, between diseased tissue ovary tumor and healthy tissue ovary, between diseased tissue breast tumor and healthy tissue breast, between diseased tissue prostate tumor and healthy tissue prostate, between diseased tissue kidney tumor and healthy tissue kidney, between diseased tissue HEK 293 cells and healthy tissue kidney demonstrates that the human DPP1 or mRNA can be utilized to diagnose of cancer. Additionally the activity of the human DPP1 can be modulated to treat cancer.

*Inflammatory Diseases*

Inflammatory diseases comprise diseases triggered by cellular or non-cellular mediators of the immune system or tissues causing the inflammation of body tissues and subsequently producing an acute or chronic inflammatory condition. Examples for such inflammatory diseases are hypersensitivity reactions of type I - IV, for example but not limited to hypersensitivity diseases of the lung including asthma, atopic diseases, allergic rhinitis or conjunctivitis, angioedema of the lids, hereditary angioedema, antireceptor hypersensitivity reactions and autoimmune diseases, Hashimoto's thyroiditis, systemic lupus erythematosus, Goodpasture's syndrome, pemphigus, myasthenia gravis, Grave's and Raynaud's disease, type B insulin-resistant diabetes, rheumatoid arthritis, psoriasis, Crohn's disease, scleroderma, mixed connective tissue disease, polymyositis, sarcoidosis, glomerulonephritis, acute or chronic host versus graft reactions.

The human DPP1 is highly expressed in the following tissues of the immune system and tissues responsive to components of the immune system as well as in the following tissues responsive to mediators of inflammation: liver liver cirrhosis, leukocytes (peripheral blood), bone marrow, neutrophils peripheral blood, spleen liver cirrhosis. The expression in the above mentioned tissues and in particular the differential expression between diseased tissue liver liver cirrhosis and healthy tissue liver, between diseased tissue spleen liver cirrhosis and healthy tissue spleen demonstrates that the human DPP1 or mRNA can be utilized to diagnose of inflammatory diseases. Additionally the activity of the human DPP1 can be modulated to treat inflammatory diseases.

*Disorders Related to Pulmology*

Asthma is thought to arise as a result of interactions between multiple genetic and environmental factors and is characterized by three major features: 1) intermittent and reversible airway obstruction caused by bronchoconstriction, increased mucus production, and thickening of the walls of the airways that leads to a narrowing of the airways, 2) airway hyperresponsiveness, and 3) airway inflammation. Certain cells are critical to the inflammatory reaction of asthma and they include T cells and antigen presenting cells, B cells that produce IgE, and mast cells, basophils, eosinophils, and other cells that bind IgE. These effector cells accumulate at the site of allergic reaction in the airways and release toxic products that contribute to the acute pathology and eventually to tissue destruction related to the disorder. Other resident cells, such as smooth muscle cells, lung epithelial cells, mucus-producing cells, and nerve cells may also be abnormal in individuals with asthma and may contribute to its pathology. While the airway obstruction of asthma, presenting clinically as an intermittent wheeze and shortness of breath, is generally the most pressing symptom of the disease requiring immediate treatment, the inflammation and tissue destruction associated with the disease can lead to irreversible changes that eventually make asthma a chronic and disabling disorder requiring long-term management.

Chronic obstructive pulmonary (or airways) disease (COPD) is a condition defined physiologically as airflow obstruction that generally results from a mixture of emphysema and peripheral airway obstruction due to chronic bronchitis [Botstein, 1980]. Emphysema is characterised by destruction of alveolar walls leading to abnormal enlargement of the air spaces of the lung. Chronic bronchitis is defined clinically as the presence of chronic productive cough for three months in each of two successive years. In COPD, airflow obstruction is usually progressive and is only partially reversible. By far the most important risk factor for development of COPD is cigarette smoking, although the disease does also occur in non-smokers.

The human DPP1 is highly expressed in the following tissues of the respiratory system: pulmonary artery smooth muscle cells, leukocytes (peripheral blood), neutrophils peripheral blood, fetal lung fibroblast IMR-90 cells, fetal lung fibroblast MRC-5 cells, lung right upper lobe, trachea, bronchial epithelial cells, bronchial smooth muscle cells, small airway epithelial cells. The expression in the above mentioned tissues and in particular the differential expression between diseased tissue fetal lung fibroblast IMR-90 cells and healthy tissue fetal lung, between diseased tissue fetal lung fibroblast MRC-5 cells and healthy tissue fetal lung demonstrates that the human DPP1 or mRNA can be utilized to diagnose of respiratory diseases. Additionally the activity of the human DPP1 can be modulated to treat those diseases.

*Disorders Related to Urology*

Genitourinary disorders comprise benign and malign disorders of the organs constituting the genitourinary system of female and male, renal diseases like acute or chronic renal failure, immunologically mediated renal diseases like renal transplant rejection, lupus nephritis, immune complex renal diseases, glomerulopathies, nephritis, toxic nephropathy, obstructive uropathies like benign prostatic hyperplasia (BPH), neurogenic bladder syndrome, urinary incontinence like urge-, stress-, or overflow incontinence, pelvic pain, and erectile dysfunction.

The human DPP1 is highly expressed in the following urological tissues: spinal cord, prostate, prostate, prostate, prostate BPH, prostate tumor, bladder, bladder, fetal kidney, kidney, kidney, kidney tumor, renal epithelial cells, HEK 293 cells. The expression in the above mentioned tissues and in particular the differential expression between diseased tissue prostate BPH and healthy tissue prostate demonstrates that the human DPP1 or mRNA can be utilized to diagnose of urological disorders. Additionally the activity of the human DPP1 can be modulated to treat urological disorders.

The human DPP1 is highly expressed in spinal cord tissues: spinal cord. Expression in spinal cord tissues demonstrates that the human DPP1 or mRNA can be utilized to diagnose of incontinence as an urological disorder. The spinal cord tissues are involved in the neuronal regulation of the urological system. Additionally the activity of the human DPP 1 can be modulated to treat - but not limited to - incontinence.

*Metabolic Disorders*

Metabolic diseases are defined as conditions which result from an abnormality in any of the chemical or biochemical transformations and their regulating systems essential to producing energy, to regenerating cellular constituents, to eliminating unneeded products arising from these processes, and to regulate and maintain homeostasis in a mammal regardless of whether acquired or the result of a genetic transformation. Depending on which metabolic pathway is involved, a single defective transformation or disturbance of its regulation may produce consequences that are narrow, involving a single body function, or broad, affecting many organs, organ-systems or the body as a whole. Diseases resulting from abnormalities related to the fine and coarse mechanisms that affect each individual transformation, its rate and direction or the availability of substrates like amino acids, fatty acids, carbohydrates, minerals, cofactors, hormones, regardless whether they are inborn or acquired, are well within the scope of the definition of a metabolic disease according to this application.

Metabolic diseases often are caused by single defects in particular biochemical pathways, defects that are due to the deficient activity of individual enzymes or molecular receptors leading to the regulation of such enzymes. Hence in a broader sense disturbances of the underlying genes, their products and their regulation lie well within the scope of this definition of a metabolic disease. For example, but not limited to, metabolic diseases may affect 1) biochemical processes and tissues ubiquitous all over the body, 2) the bone, 3) the nervous system, 4) the endocrine system, 5) the muscle including the heart, 6) the skin and nervous tissue, 7) the urogenital system, 8) the homeostasis of body systems like water and electrolytes. For example, but not limited to, metabolic diseases according to 1) comprise obesity, amyloidosis, disturbances of the amino acid metabolism like branched chain disease, hyperaminoacidemia, hyperaminoaciduria, disturbances of the metabolism of urea, hyperammonemia, mucopolysaccharidoses e.g. Maroteaux-Lamy syndrom, storage diseases like glycogen storage diseases and lipid storage diseases, glycogenosis diseases like Cori's disease, malabsorption diseases like intestinal carbohydrate malabsorption, oligosaccharidase deficiency like maltase-, lactase-, sucrase-insufficiency, disorders of the metabolism of fructose, disorders of the metabolism of galactose, galactosaemia, disturbances of carbohydrate utilization like diabetes, hypoglycemia, disturbances of pyruvate metabolism, hypolipidemia, hypolipoproteinemia, hyperlipidemia, hyperlipoproteinemia, carnitine or carnitine acyltransferase deficiency, disturbances of the porphyrin metabolism, porphyrias, disturbances of the purine metabolism, lysosomal diseases, metabolic diseases of nerves and nervous systems like gangliosidoses, sphingolipidoses, sulfatidoses, leucodystrophies, Lesch-Nyhan syndrome. For example, but not limited to, metabolic diseases according to 2) comprise osteoporosis, osteomalacia like osteoporosis, osteopenia, osteogenesis imperfecta, osteopetrosis, osteonecrosis, Paget's disease of bone, hypophosphatemia. For example, but not limited to, metabolic diseases according to 3) comprise cerebellar dysfunction, disturbances of brain metabolism like dementia, Alzheimer's disease, Huntington's chorea, Parkinson's disease, Pick's disease, toxic encephalopathy, demyelinating neuropathies like inflammatory neuropathy, Guillain-Barré syndrome. For example, but not limited to, metabolic diseases according to 4) comprise primary and secondary metabolic disorders associated with hormonal defects like any disorder stemming from either an hyperfunction or hypofunction of some hormone-secreting endocrine gland and any combination thereof. They comprise Sipple's syndrome, pituitary gland dysfunction and its effects on other endocrine glands, such as the thyroid, adrenals, ovaries, and testes, acromegaly, hyper-and hypothyroidism, euthyroid goiter, euthyroid sick syndrome, thyroiditis, and thyroid cancer, over- or underproduction of the adrenal steroid hormones, adrenogenital syndrome, Cushing's syndrome, Addison's disease of the adrenal cortex, Addison's pernicious anemia, primary and secondary aldosteronism, diabetes insipidus, carcinoid syndrome, disturbances caused by the dysfunction of the parathyroid glands, pancreatic islet cell dysfunction, diabetes, disturbances of the endocrine system of the female like estrogen deficiency, resistant ovary syndrome. For example, but not limited to, metabolic diseases according to 5) comprise muscle weakness, myotonia, Duchenne's and other muscular dystrophies, dystrophia myotonica of Steinert, mitochondrial myopathies like disturbances of the catabolic metabolism in the muscle, carbohydrate and lipid storage myopathies, glycogenoses, myoglobinuria, malignant hyperthermia, polymyalgia rheumatica, dermatomyositis, primary myocardial disease, cardiomyopathy. For example, but not limited to, metabolic diseases according to 6) comprise disorders of the ectoderm, neurofibromatosis, scleroderma and polyarteritis, Louis-Bar syndrome, von Hippel-Lindau disease, Sturge-Weber syndrome, tuberous sclerosis, amyloidosis, porphyria. For example, but not limited to, metabolic diseases according to 7) comprise sexual dysfunction of the male and female. For example, but not limited to, metabolic diseases according to 8) comprise confused states and seizures due to inappropriate secretion of antidiuretic hormone from the pituitary gland, Liddle's syndrome, Bartter's syndrome, Fanconi's syndrome, renal electrolyte wasting, diabetes insipidus.

The human DPP1 is highly expressed in the following metabolic disease related tissues: thyroid, thyroid tumor, liver liver cirrhosis, liver lupus disease, HEP G2 cells, spleen liver cirrhosis, cartilage and adipose. The expression in the above mentioned tissues and in particular the differential expression between diseased tissue liver liver cirrhosis and healthy tissue liver, between diseased tissue liver lupus disease and healthy tissue liver, between diseased tissue spleen liver cirrhosis and healthy tissue spleen demonstrates that the human DPP1 or mRNA can be utilized to diagnose of metabolic diseases. Additionally the activity of the human DPP1 can be modulated to treat metabolic diseases.

*Infections*

Certain bacteria, virusses, fungi and parasites are able to establish an infection of the human body. This invention relates to treatment of infectious diseases. In the following, examples of pathogens potentially leading to infections and infectious diseases are presented. The diseases mentioned serve as examples, the scope of the invention is not limited to infections presented here.

Examples of infections of the skin and underlying tissue are: cellulitis, necrotizing fasciitis, skin gangrene, lymphadenitis, acute lymphangitis, impetigo, skin abscesses, folliculitis, boils (furuncles), erysipelas, carbuncles (clusters of boils and skin abscesses), staphylococcal scalded skin syndrome, erythrasma or paronychia (can be caused by many bacteria and fungi). Most of these are bacterial infections. The most common bacterial skin infections are caused by Staphylococcus and Streptococcus.

Skin infections caused by fungi are ringworm, a fungal skin infection caused by several different fungi and generally classified by its location on the body. Examples are Athlete's foot (foot ringworm, caused by either Trichophyton or Epidermophyton), jock itch (groin ringworm, can be caused by a variety of fungi and yeasts), scalp ringworm, caused by Trichophyton or Microsporum), nail ringworm and body ringworm (caused by Trichophyton).

Candidiasis (yeast infection, moniliasis) is an infection by the yeast Candida. The following types of candida infections can be distinguished: Infections in skinfolds (intertriginous infections), vaginal and penile candida infections (vulvovaginitis), thrush, Perlèche (candida infection at the corners of the mouth), candidal paronychia (candida growing in the nail beds, produces painful swelling and pus). Candida can also lead to generalized systemic infections especially in the immunocompromised host.

Tinea versicolor is a fungal infection that causes white to light brown patches on the skin.

The skin can also be affected by parasites, mainly tiny insects or worms. Examples are scabies (mite infestation), lice infestation (pediculosis, head lice and pubic lice are two different species), or creeping eruption (cutaneous larva migrans, a hookworm infection).

Many types of viruses invade the skin. Examples are papillomavirusses (causing warts), herpes simplex virus (causing e.g. cold sores), or members of the poxvirus family (molluscum contagiosum (infection of the skin, causing skin-colored, smooth, waxy bumps).

Abscesses are accumulation of pus, usually caused by a bacterial infection. Examples are abdominal abscesses, head and neck abscesses, muscle abscesses, or hand Abscesses.

Bacteremia, the presence of bacteria in the bloodstream, is common and usually causes no symptoms. Most bacteria that enter the bloodstream are rapidly removed by white blood cells. Sometimes, however, there are too many bacteria to be removed easily, and an infection called sepsis develops, causing severe symptoms. In some cases, sepsis leads to a life-threatening condition called septic shock.

Bacilli are a type of bacteria classified according to their distinctive rod-like shape. Bacteria are either spherical (coccal), rod-like (bacillary), or spiral/helical (spirochetal) in shape. Gram-positive or gram-negative bacilli are distinguished

Examples of gram-positive bacillary infections are are erysipelothricosis (caused by Erysipelothrix rhusiopathiae), listeriosis (caused by Listeria monocytogenes), and anthrax (caused by Bacillus anthracis). Within anthrax, pulmonary anthrax, gastrointestinal anthrax and anthrax skin sores can be distinguished.

Examples of gram-negative bacillary infections are Hemophilus infections, Hemophilus influenzae infections, Hemophilus ducreyi (causes chancroid), Brucellosis (undulant, Malta, Mediterranean, or Gibraltar fever, caused by Brucella bacteria), tularemia (rabbit fever, deer fly fever, caused by Francisella tularensis), plague (black death, caused by Yersinia pestis, bubonic plaque, pneumonic plague, septicemic plague and pestis minor are distinguished), cat-scratch disease (caused by the bacterium Bartonella henselae), Pseudomonas infections (especially Pseudomonas aeruginosa), infections of the gastrointestinal tract or blood caused by Campylobacter bacteria (e.g. Campylobacter pylori [Helicobacter pylori]), cholera (infection of the small intestine caused by Vibrio cholerae), infections with other Vibrio spp., Enterobacteriaceae infections (cause e.g. infections of the gastrointestinal tract, members of the group are Salmonella, Shigella, Escherichia, Klebsiella, Enterobacter, Serratia, Proteus, Morganella, Providencia, and Yersinia), Klebsella pneumonia infections (severe lung infection), typhoid fever (caused by Salmonella typhi), nontyphoidal Salmonella infections, or Shigellosis (bacillary dysentery, an intestinal infection caused by Shigella bacteria).

Bacteria that have a spherical shape are called cocci. Cocci that can cause infection in humans include staphylococci, streptococci (group A streptococci, group B streptococci, groups C and G streptococci, group D streptococci and enterocooci), pneumococci (cause e.g pneumonia, thoracic empyema, bacterial meningitis, bacteremia, pneumococcal endocarditis, peritonitis, pneumococcal arthritis or otitis media), and meningococci. Toxic shock syndrome is an infection usually caused by staphylococci, which may rapidly worsen to severe, untreatable shock. Meningococci (Neisseria meningitidis) may cause infection of the layers covering the brain and spinal cord (meningitis). Neisseria gonorrhoeae cause gonorrhea, a sexually transmitted disease.

Spirochetal Infections are infections with spirochetes, corkscrew-shaped bacteria. Examples include infections with Treponema, Borrelia, Leptospira, and Spirillum.

Treponematoses (e.g. yaws, pinta) are caused by a spirochete that is indistinguishable from Treponema pallidum (causes syphilis).

Relapsing fever (tick fever, recurrent fever, or famine fever) is a disease caused by several strains of Borrelia bacteria.

Lyme disease (transmitted by deer ticks) is caused by the spirochete Borrelia burgdorferi.

Other examples for infections with spirochetes are Leptospirosis (a group of infections including Weil's syndrome, infectious (spirochetal) jaundice, and canicola fever), or rat-bite fever).

Disease-causing anaerobic bacteria include clostridia, peptococci, and peptostreptococci. Other examples are Bacteroides fragilis, Prevotella melaninogenica and Fusobacterium. Infections with anaerobic bacteria include dental abscesses, jawbone infections, periodontal disease, chronic sinusitis and middle ear infection, and abscesses in the brain, spinal cord, lung, abdominal cavity, liver, uterus, genitals, skin, and blood vessels. Examples for Clostridial infections tetanus (lockjaw, caused by the bacterium Clostridium tetani), or Actinomycosis (a chronic infection caused mainly by Actinomyces israelii).

Tuberculosis and leprosy are caused by Mycobacteria. Tuberculosis is caused by the airborne bacterium Mycobacterium tuberculosis, M. bovis, or M. africanum. Leprosy (Hansen's disease) is caused by the bacterium Mycobacterium leprae.

Rickettsial infections are also known. Examples of diseases caused by Rickettsiae or Ehrlichieae are murine typhus (caused by Rickettsia typhi), Rocky Mountain spotted fever (caused by Rickettsia rickettsii), epidemic typhus (Rickettsia prowazekii), scrub typhus (Rickettsia tsutsugamushi), Ehrlichiosis (Ehrlichia canis or closely related species), Rickettsial-pox, (Rickettsia akari), Q fever (Coxiella burnetii), or trench fever (Bartonella quintana).

A parasite is an organism, such as a single-celled animal (protozoan) or worm, that survives by living inside another, usually much larger, organism. Examples for parasitic infections are Amebiasis (caused by Entamoeba histolytica), Giardiasis (Giardia lamblia), Malaria (Plasmodium), Toxoplasmosis (Toxoplasma gondii), Babesiosis (Babesia parasites), Trichuriasis (Trichuris trichiura, an intestinal roundworm), Ascariasis (Ascaris lumbricoides), Hookworm Infection (Ancylostoma duodenale or Necator americanus), Trichinosis (Trichinella spiralis), Toxocariasis (visceral larva migrans, caused by the invasion of organs by roundworm larvae, such as Toxocara canis and Toxocara cati), Pork tapeworm infection (Taenia solium), or Fish tapeworm infection (Diphyllobothrium latum).

Fungi tend to cause infections in people with a compromised immune system. Examples for fungal infections are Histoplasmosis (caused by Histoplasma capsulatum), Coccidioidomycosis (Coccidioides immitis), Blastomycosis (Blastomyces dermatitidis), Candidiasis (caused by strains of Candida, especially Candida albicans), or Sporotrichosis (Sporothrix schenckii).

Viral infections represent a very common type of infection. A virus is a small infectious particle that needs a living cell reproduce. Examples of viral infections are given in the following. Respiratory viral infections are, for example, common cold (caused by Picornaviruses [e.g. rhinoviruses], Influenza viruses or respiratory syncytial viruses), Influenza (caused by influenza A or influenza B virus), Herpesvirus Infections (herpes simplex, herpes zoster, Epstein-Barr virus, cytomegalovirus, herpesvirus 6, human herpesvirus 7, or herpesvirus 8 (cause of Kaposi's sarcoma in people with AIDS), central nervous system viral infections (e.g. Rabies, Creutzfeldt-Jakob disease (subacute spongiform encephalopathy), progressive multifocal leukoencephalopathy (rare manifestation of polyomavirus infection of the brain caused by the JC virus), Tropical spastic paraparesis (HTLV-I), Arbovirus infections (e.g. Arbovirus encephalitis, yellow fever, or dengue fever), Arenavirus Infections (e.g Lymphocytic choriomeningitis), hemorrhagic fevers (e.g. Bolivian and Argentinean hemorrhagic fever and Lassa fever, Hantavirus infection, Ebola and Marburg viruses).

Human immunodeficiency virus (HIV) infection is an infection caused by HIV-1 or HIV-II virus. The infection results in progressive destruction of lymphocytes. This leads to acquired immunodeficiency syndrome (AIDS).

Examples of typical infections of people with an impaired immune system (opportunistic infections are including but are not limited to nocardiosis (caused by Nocardia asteroides), aspergillosis, mucormycosis, and cytomegalovirus infection.

Examples for sexually transmitted (venereal) diseases are syphilis (caused by Treponema pallidum), gonorrhea (Neisseria gonorrhoeae), chancroid (Hemophilus ducreyi), lymphogranuloma venereum (Chlamydia trachomatis), granuloma inguinale( Calymmatobacterium granulomatis), nongonococcal urethritis and chlamydial cervicitis (caused by Chlamydia trachomatis, Ureaplasma urealyticum, Trichomonas vaginalis or herpes simplex virus), trichomoniasis (Trichomonas vaginalis), genital candidiasis, genital herpes, genital warts (caused by papillomaviruses), or HIV infection.

The human DPP1 is highly expressed in the following antiinfective tissues: T-cells peripheral blood CD4+ D34 virus infected, monocytes, monocytes HIV-1 infected. The expression in the above mentioned tissues and in particular the differential expression between diseased tissue T-cells peripheral blood CD4+ D34 virus infected and healthy tissue T-cells peripheral blood CD4+, between diseased tissue monocytes HIV-1 infected and healthy tissue monocytes demonstrates that the human DPP1 or mRNA can be utilized to diagnose of infective diseases. Additionally the activity of the human DPP1 can be modulated to treat infective diseases.

*Applications*

The present invention provides for both prophylactic and therapeutic methods for infections, cardiovascular diseases, respiratory diseases, cancer, endocrinological diseases, metabolic diseases, gastroenterological diseases, inflammation, hematological diseases, muscle skeleton diseases, neurological diseases and urological diseases.

The regulatory method of the invention involves contacting a cell with an agent that modulates one or more of the activities of DPP1. An agent that modulates activity can be an agent as described herein, such as a nucleic acid or a protein, a naturally-occurring cognate ligand of the polypeptide, a peptide, a peptidomimetic, or any small molecule. In one embodiment, the agent stimulates one or more of the biological activities of DPP1. Examples of such stimulatory agents include the active DPP1 and nucleic acid molecules encoding a portion of DPP1. In another embodiment, the agent inhibits one or more of the biological activities of DPP1. Examples of such inhibitory agents include antisense nucleic acid molecules and antibodies. These regulatory methods can be performed in vitro (e.g., by culturing the cell with the agent) or, alternatively, in vivo (e.g, by administering the agent to a subject). As such, the present invention provides methods of treating an individual afflicted with a disease or disorder characterized by unwanted expression or activity of DPP1 or a protein in the DPP1 signaling pathway. In one embodiment, the method involves administering an agent like any agent identified or being identifiable by a screening assay as described herein, or combination of such agents that modulate say upregulate or downregulate the expression or activity of DPP1 or of any protein in the DPP1 signaling pathway. In another embodiment, the method involves administering a regulator of DPP1 as therapy to compensate for reduced or undesirably low expression or activity of DPP1 or a protein in the DPP1 signaling pathway.

Stimulation of activity or expression of DPP1 is desirable in situations in which enzymatic activity or expression is abnormally low and in which increased activity is likely to have a beneficial effect. Conversely, inhibition of enzymatic activity or expression of DPP1 is desirable in situations in which activity or expression of DPP1 is abnormally high and in which decreasing its activity is likely to have a beneficial effect.

This invention is further illustrated by the following examples which should not be construed as limiting. The contents of all references, patents and published patent applications cited throughout this application are hereby incorporated by reference.

**Pharmaceutical Compositions**

This invention further pertains to novel agents identified by the above-described screening assays and uses thereof for treatments as described herein.

The nucleic acid molecules, polypeptides, and antibodies (also referred to herein as "active compounds") of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes pharmaceutical compositions comprising a regulator of DPP1 expression or activity (and/or a regulator of the activity or expression of a protein in the DPP1 signaling pathway) as well as methods for preparing such compositions by combining one or more such regulators and a pharmaceutically acceptable carrier. Also within the invention are pharmaceutical compositions comprising a regulator identified using the screening assays of the invention packaged with instructions for use. For regulators that are antagonists of DPP1 activity or which reduce DPP1 expression, the instructions would specify use of the pharmaceutical composition for treatment of infections, cardiovascular diseases, respiratory diseases, cancer, endocrinological diseases, metabolic diseases, gastroenterological diseases, inflammation, hematological diseases, muscle skeleton diseases, neurological diseases and urological diseases. For regulators that are agonists of DPP1 activity or increase DPP1 expression, the instructions would specify use of the pharmaceutical composition for treatment of infections, cardiovascular diseases, respiratory diseases, cancer, endocrinological diseases, metabolic diseases, gastroenterological diseases, inflammation, hematological diseases, muscle skeleton diseases, neurological diseases and urological diseases.

An inhibitor of DPP1 may be produced using methods which are generally known in the art. In particular, purified DPP1 may be used to produce antibodies or to screen libraries of pharmaceutical agents to identify those which specifically bind DPP1. Antibodies to DPP1 may also be generated using methods that are well known in the art. Such antibodies may include, but are not limited to, polyclonal, monoclonal, chimeric, single chain antibodies, Fab fragments, and fragments produced by a Fab expression library. Neutralizing antibodies like those which inhibit dimer formation are especially preferred for therapeutic use.

In another embodiment of the invention, the polynucleotides encoding DPP1, or any fragment or complement thereof, may be used for therapeutic purposes. In one aspect, the complement of the polynucleotide encoding DPP may be used in situations in which it would be desirable to block the transcription of the mRNA. In particular, cells may be transformed with sequences complementary to polynucleotides encoding DPP1. Thus, complementary molecules or fragments may be used to modulate DPP1 activity, or to achieve regulation of gene function. Such technology is now well known in the art, and sense or antisense oligonucleotides or larger fragments can be designed from various locations along the coding or control regions of sequences encoding DPP1.

Expression vectors derived from retroviruses, adenoviruses, or herpes or vaccinia viruses, or from various bacterial plasmids, may be used for delivery of nucleotide sequences to the targeted organ, tissue, or cell population. Methods which are well known to those skilled in the art can be used to construct vectors which will express nucleic acid sequence complementary to the polynucleotides of the gene encoding DPP1. These techniques are described, for example, in [Scott and Smith (1990)].

Any of the therapeutic methods described above may be applied to any subject in need of such therapy, including, for example, mammals such as dogs, cats, cows, horses, rabbits, monkeys, and most preferably, humans.

An additional embodiment of the invention relates to the administration of a pharmaceutical composition containing DPP1 in conjunction with a pharmaceutically acceptable carrier, for any of the therapeutic effects discussed above. Such pharmaceutical compositions may consist of DPP1, antibodies to DPP1, and mimetics, agonists, antagonists, or inhibitors of DPP1. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin. Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash,
wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration. For pharmaceutical compositions which include an antagonist of DPP1 activity, a compound which reduces expression of DPP1, or a compound which reduces expression or activity of a protein in the DPP1 signaling pathway or any combination thereof, the instructions for administration will specify use of the composition for infections, cardiovascular diseases, respiratory diseases, cancer, endocrinological diseases, metabolic diseases, gastroenterological diseases, inflammation, hematological diseases, muscle skeleton diseases, neurological diseases and urological diseases. For pharmaceutical compositions which include an agonist of DPP1 activity, a compound which increases expression of DPP1, or a compound which increases expression or activity of a protein in the DPP1 signaling pathway or any combination thereof, the instructions for administration will specify use of the composition for infections, cardiovascular diseases, respiratory diseases, cancer, endocrinological diseases, metabolic diseases, gastroenterological diseases, inflammation, hematological diseases, muscle skeleton diseases, neurological diseases and urological diseases.

## Diagnostics

In another embodiment, antibodies which specifically bind DPP1 may be used for the diagnosis of disorders characterized by the expression of DPP1, or in assays to monitor patients being treated with DPP1 or agonists, antagonists, and inhibitors of DPP1. Antibodies useful for diagnostic purposes may be prepared in the same manner as those described above for therapeutics. Diagnostic assays for DPP1 include methods which utilize the antibody and a label to detect DPP1 in human body fluids or in extracts of cells or tissues. The antibodies may be used with or without modification, and may be labeled by covalent or non-covalent joining with a reporter molecule. A wide variety of reporter molecules, several of which are described above, are known in the art and may be used.

A variety of protocols for measuring DPP1, including ELISAs, RIAs, and FACS, are known in the art and provide a basis for diagnosing altered or abnormal levels of DPP1 expression. Normal or standard values for DPP1 expression are established by combining body fluids or cell extracts taken from normal mammalian subjects, preferably human, with antibody to DPP1 under conditions suitable for complex formation. The amount of standard complex formation may be quantified by various methods, preferably by photometric means. Quantities of DPP1 expressed in subject samples from biopsied tissues are compared with the standard values. Deviation between standard and subject values establishes the parameters for diagnosing disease.

In another embodiment of the invention, the polynucleotides encoding DPP1 may be used for diagnostic purposes. The polynucleotides which may be used include oligonucleotide sequences, complementary RNA and DNA molecules, and PNAs. The polynucleotides may be used to detect and quantitate gene expression in biopsied tissues in which expression of DPP1 may be correlated with disease. The diagnostic assay may be used to distinguish between absence, presence, and excess expression of DPP1, and to monitor regulation of DPP1 levels during therapeutic intervention.

Polynucleotide sequences encoding DPP1 may be used for the diagnosis of infections, cardiovascular diseases, respiratory diseases, cancer, endocrinological diseases, metabolic diseases, gastroenterological diseases, inflammation, hematological diseases, muscle skeleton diseases, neurological diseases and urological diseases associated with expression of DPP1. The polynucleotide sequences encoding DPP1 may be used in Southern, Northern, or dot-blot analysis, or other membrane-based technologies; in PCR technologies; in dipstick, pin, and ELISA assays; and in microarrays utilizing fluids or tissues from patient biopsies to detect altered DPP1 expression. Such qualitative or quantitative methods are well known in the art.

In a particular aspect, the nucleotide sequences encoding DPP1 may be useful in assays that detect the presence of associated disorders, particularly those mentioned above. The nucleotide sequences encoding DPP1 may be labelled by standard methods and added to a fluid or tissue sample from a patient under conditions suitable for the formation of hybridization complexes. After a suitable incubation period, the sample is washed and the signal is quantitated and compared with a standard value. If the amount of signal in the patient sample is significantly altered from that of a comparable control sample, the nucleotide sequences have hybridized with nucleotide sequences in the sample, and the presence of altered levels of nucleotide sequences encoding DPP1 in the sample indicates the presence of the associated disorder. Such assays may also be used to evaluate the efficacy of a particular therapeutic treatment regimen in animal studies, in clinical trials, or in monitoring the treatment of an individual patient.

In order to provide a basis for the diagnosis of infections, cardiovascular diseases, respiratory diseases, cancer, endocrinological diseases, metabolic diseases, gastroenterological diseases, inflammation, hematological diseases, muscle skeleton diseases, neurological diseases and urological diseases associated with expression of DPP1, a normal or standard profile for expression is established. This may be accomplished by combining body fluids or cell extracts taken from normal subjects, either animal or human, with a sequence, or a fragment thereof, encoding DPP1, under conditions suitable for hybridization or amplification. Standard hybridization may be quantified by comparing the values obtained from normal subjects with values from an experiment in which a known amount of a substantially purified polynucleotide is used. Standard values obtained from normal samples may be compared with values obtained from samples from patients who are symptomatic for a disorder. Deviation from standard values is used to establish the presence of a disorder.

**Determination of a Therapeutically Effective Dose**

The determination of a therapeutically effective dose is well within the capability of those skilled in the art. A therapeutically effective dose refers to that amount of active ingredient which increases or decreases DPP1 activity relative to DPP1 activity which occurs in the absence of the therapeutically effective dose. For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the $ED_{50}$ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun", and DEAE- or calcium phosphate-mediated transfection.

If the expression product is mRNA, the reagent is preferably an antisense oligonucleotide or a ribozyme. Polynucleotides which express antisense oligonucleotides or ribozymes can be introduced into cells by a variety of methods, as described above. Preferably, a reagent reduces expression of DPP1 gene or the activity of DPP1 by at least about 10, preferably about 50, more preferably about 75, 90, or 100% relative to the absence of the reagent. The effectiveness of the mechanism chosen to decrease the level of expression of DPP1 gene or the activity of DPP1 can be assessed using methods well known in the art, such as hybridization of nucleotide probes to DPP1-specific mRNA, quantitative RT-PCR, immunologic detection of DPP1, or measurement of DPP1 activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects. Any of the therapeutic methods described above can be applied to any subject in need of such therapy, including, for example, mammals such as dogs, cats, cows, horses, rabbits, monkeys, and most preferably, humans.

Nucleic acid molecules of the invention are those nucleic acid molecules which are contained in a group of nucleic acid molecules consisting of (i) nucleic acid molecules encoding a polypeptide comprising the amino acid sequence of SEQ ID NO: 2, (ii) nucleic acid molecules comprising the sequence of SEQ ID NO: 1, (iii) nucleic acid molecules having the sequence of SEQ ID NO: 1, (iv)nucleic acid molecules the complementary strand of which hybridizes under stringent conditions to a nucleic acid molecule of (i), (ii), or (iii); and (v) nucleic acid molecules the sequence of which differs from the sequence of a nucleic acid molecule of (iii) due to the degeneracy of the genetic code, wherein the polypeptide encoded by said nucleic acid molecule has DPP1 activity.

Polypeptides of the invention are those polypeptides which are contained in a group of polypeptides consisting of (i) polypeptides having the sequence of SEQ ID NO: 2, (ii) polypeptides comprising the sequence of SEQ ID NO: 2, (iii) polypeptides encoded by nucleic acid molecules of the invention and (iv) polypeptides which show at least 99%, 98%, 95%, 90%, or 80% homology with a polypeptide of (i), (ii), or (iii), wherein said purified polypeptide has DPP1 activity.

An object of the invention is a method of screening for therapeutic agents useful in the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising the steps of (i) contacting a test compound with a DPP1 polypeptide, (ii) detect binding of said test compound to said DPP1 polypeptide. E.g., compounds that bind to the DPP1 polypeptide are identified potential therapeutic agents for such a disease.

Another object of the invention is a method of screening for therapeutic agents useful in the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising the steps of (i) determining the activity of a DPP1 polypeptide at a certain concentration of a test compound or in the absence of said test compound, (ii) determining the activity of said polypeptide at a different concentration of said test compound. E.g., compounds that lead to a difference in the activity of the DPP1 polypeptide in (i) and (ii) are identified potential therapeutic agents for such a disease.

Another object of the invention is a method of screening for therapeutic agents useful in the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising the steps of (i) determining the activity of a DPP1 polypeptide at a certain concentration of a test compound, (ii) determining the activity of a DPP1 polypeptide at the presence of a compound known to be a regulator of a DPP1 polypeptide. E.g., compounds that show similar effects on the activity of the DPP1 polypeptide in (i) as compared to compounds used in (ii) are identified potential therapeutic agents for such a disease.

Other objects of the invention are methods of the above, wherein the step of contacting is in or at the surface of a cell.

Other objects of the invention are methods of the above, wherein the cell is in vitro.

Other objects of the invention are methods of the above, wherein the step of contacting is in a cell-free system.

Other objects of the invention are methods of the above, wherein the polypeptide is coupled to a detectable label.

Other objects of the invention are methods of the above, wherein the compound is coupled to a detectable label.

Other objects of the invention are methods of the above, wherein the test compound displaces a ligand which is first bound to the polypeptide.

Other objects of the invention are methods of the above, wherein the polypeptide is attached to a solid support.

Other objects of the invention are methods of the above, wherein the compound is attached to a solid support.

Another object of the invention is a method of screening for therapeutic agents useful in the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising the steps of (i) contacting a test compound with a DPP1 polynucleotide, (ii) detect binding of said test compound to said DPP1 polynucleotide. Compounds that, e.g., bind to the DPP1 polynucleotide are potential therapeutic agents for the treatment of such diseases.

Another object of the invention is the method of the above, wherein the nucleic acid molecule is RNA.

Another object of the invention is a method of the above, wherein the contacting step is in or at the surface of a cell.

Another object of the invention is a method of the above, wherein the contacting step is in a cell-free system.

Another object of the invention is a method of the above, wherein the polynucleotide is coupled to a detectable label.

Another object of the invention is a method of the above, wherein the test compound is coupled to a detectable label.

Another object of the invention is a method of diagnosing a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising the steps of (i) determining the amount of a DPP1 polynucleotide in a sample taken from said mammal, (ii) determining the amount of DPP1 polynucleotide in healthy and/or diseased mammal. A disease is diagnosed, e.g., if there is a substantial similarity in the amount of DPP1 polynucleotide in said test mammal as compared to a diseased mammal.

Another object of the invention is a pharmaceutical composition for the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising a therapeutic agent which binds to a DPP1 polypeptide.

Another object of the invention is a pharmaceutical composition for the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising a therapeutic agent which regulates the activity of a DPP1 polypeptide.

Another object of the invention is a pharmaceutical composition for the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising a therapeutic agent which regulates the activity of a DPP1 polypeptide, wherein said therapeutic agent is (i) a small molecule, (ii) an RNA molecule, (iii) an antisense oligonucleotide, (iv) a polypeptide, (v) an antibody, or (vi) a ribozyme.

Another object of the invention is a pharmaceutical composition for the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising a DPP1 polynucleotide.

Another object of the invention is a pharmaceutical composition for the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising a DPP1 polypeptide.

Another object of the invention is the use of regulators of a DPP1 for the preparation of a pharmaceutical composition for the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal.

Another object of the invention is a method for the preparation of a pharmaceutical composition useful for the treatment of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal comprising the steps of (i) identifying a regulator of DPP1, (ii) determining whether said regulator ameliorates the symptoms of a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases in a mammal; and (iii) combining of said regulator with an acceptable pharmaceutical carrier.

Another object of the invention is the use of a regulator of DPP1 for the regulation of DPP1 activity in a mammal having a disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases.

The uses, methods or compositions of the invention are useful for each single disease comprised in a group of diseases consisting of infections, cardiovascular diseases, respiratory diseases, cancer, endocrinological diseases, metabolic diseases, gastroenterological diseases, inflammation, hematological diseases, muscle skeleton diseases, neurological diseases and urological diseases.

The expression of human DPP1 in cancer and urological related tissues (as described above) suggests a particular - but not limited to - utilization of DPP1 for diagnosis and modulation of urological diseases and cancer. Furthermore the above described expression suggest a - but not limited to - utilization of DPP1 to infections, cardiovascular diseases, respiratory diseases, endocrinological diseases, metabolic diseases, gastroenterological diseases, inflammation, hematological diseases, muscle skeleton diseases and neurological diseases.

The examples below are provided to illustrate the subject invention. These examples are provided by way of illustration and are not included for the purpose of limiting the invention.

## Examples

### Example 1: Search for homologous sequences in public sequence data bases

The degree of homology can readily be calculated by known methods. Preferred methods to determine homology are designed to give the largest match between the sequences tested. Methods to determine homology are codified in publicly available computer programs such as BestFit, BLASTP, BLASTN, and FASTA. The BLAST programs are publicly available from NCBI and other sources in the internet.

For DPP1 the following hits to known sequences were identified by using the BLAST algorithm [Altschul SF, Madden TL, Schaffer AA, Zhang J, Zhang Z, Miller W, Lipman DJ; Nucleic Acids Res 1997 Sep 1; 25(17): 3389-402] and the following set of parameters: matrix = BLOSUM62 and low complexity filter. The following databases were searched: NCBI (non-redundant database) and DERWENT patent database (Geneseq).

The following hits were found:

>emb|X87212.1|HSCATHCGE H.sapiens mRNA for cathepsin C
Length = 1838, Score = 3476 bits (1808), Expect = 0.0, Identities = 1823/1838 (99%)

>gb|AF254757.1|AF254757 Homo sapiens clone CMM3 cathepsin C (CTSC) mRNA, complete cds
Length = 1468, Score = 2786 bits (1449), Expect = 0.0, Identities = 1459/1468 (99%)

>gb|AF234264.1|AF234264 Homo sapiens clone CMM2 cathepsin C (CTSC) mRNA, complete cds
Length = 1468, Score = 2786 bits (1449), Expect = 0.0, Identities = 1459/1468 (99%)

>gb|AF234263.1|AF234263 Homo sapiens clone CMM1 cathepsin C (CTSC) mRNA, complete cds
Length = 1468, Score = 2786 bits (1449), Expect = 0.0, Identities = 1459/1468 (99%)

>gb|AF525033.1| Homo sapiens mutant cathepsin C mRNA, complete cds
Length = 1468, Score = 2786 bits (1449), Expect = 0.0, Identities = 1459/1468 (99%)

>gb|AF525032.1| Homo sapiens cathepsin C mRNA, complete cds
Length = 1468, Score = 2786 bits (1449), Expect = 0.0, Identities = 1459/1468 (99%)

>gb|BC008059.1| Homo sapiens cathepsin C, mRNA (cDNA clone MGC:1766 IMAGE:2967491),complete cds
Length = 1836, Score = 2511 bits (1306), Expect = 0.0, Identities = 1313/1320 (99%)

>gb|AC011088.8|AC011088 Homo sapiens chromosome 11, clone RP11-292E14, complete sequence
Length = 164991, Score = 1738 bits (904), Expect = 0.0, Identities = 911/918 (99%)

>gb|U79415.1|HSU79415 Homo sapiens prepro dipeptidyl peptidase I (DPP-I) gene, complete cds
Length = 4715, Score = 1738 bits (904), Expect = 0.0, Identities = 914/923 (99%)

>db|AB060542.1| Bos taurus mRNA for cathepsin C, partial cds
Length = 1806, Score = 1586 bits (825), Expect = 0.0, Identities = 1187/1372 (86%)

>ref|NM_017097.1| Rattus norvegicus cathepsin C (Ctsc), mRNA
Length = 1850, Score = 1246 bits (648), Expect = 0.0, Identities = 1129/1371 (82%), Gaps = 3/1371 (0%)

>dbj|D90404.1|RATCATC Rattus norvegicus mRNA for cathepsin C
Length = 1850, Score = 1246 bits (648), Expect = 0.0, Identities = 1129/1371 (82%), Gaps = 3/1371 (0%)

>gb|U74683.3|MMU74683 Mus musculus dipeptidyl peptidase I precursor mRNA, complete cds
Length = 1969, Score = 1190 bits (619), Expect = 0.0, Identities = 1134/1393 (81%), Gaps = 3/1393 (0%)

>gb|BC067063.1| Mus musculus cathepsin C, mRNA (cDNA clone MGC:91337 IMAGE:30541061),complete cds
Length = 1866, Score = 1190 bits (619), Expect = 0.0, Identities = 1134/1393 (81%), Gaps = 3/1393 (0%)

>gb|U89269.1|MMU89269 Mus musculus preprodipeptidyl peptidase I mRNA, complete cds
Length = 1850, Score = 1190 bits (619), Expect = 0.0, Identities = 1134/1393 (81%), Gaps = 3/1393 (0%)

>dbj|AK049406.1| Mus musculus 7 days embryo whole body cDNA, RIKEN full-lengthenriched library, clone: C430004E08 product:cathepsin C,full insert sequence
Length = 2444, Score = 1185 bits (616), Expect = 0.0, Identities = 1133/1393 (81%), Gaps = 3/1393 (0%)

>ref|NM_009982.2| Mus musculus cathepsin C (Ctsc), mRNA
Length = 2444, Score = 1185 bits (616), Expect = 0.0, Identities = 1133/1393 (81%), Gaps = 3/1393 (0%)

>dbj|AK002454.1| Mus musculus adult male kidney cDNA, RIKEN full-length enrichedlibrary, clone:0610010C21 product:cathepsin C, fullinsert sequence
Length = 1861, Score = 1121 bits (583), Expect = 0.0, Identities = 1127/1393 (80%), Gaps = 6/1393 (0%)

>emb|BX537913.1|HSM806022 Homo sapiens mRNA; cDNA DKFZp686N18114 (from clone DKFZp686N18114); complete cds
Length = 6173, Score = 644 bits (335), Expect = 0.0, Identities = 343/351 (97%)

>ref|NM_148170.1| Homo sapiens cathepsin C (CTSC), transcript variant 2, mRNA
Length = 859, Score = 644 bits (335), Expect = 0.0, Identities = 343/351 (97%)

>dbj|AP000795.5|Homo sapiens genomic DNA, chromosome 11 clone:RP11-876F8, completesequence
Length = 76999, Score = 396 bits (206), Expect = e-107, Identities = 206/206 (100%)

>dbj|AK088398.1| Mus musculus 2 days neonate thymus thymic cells cDNA, RIKENfull-length enriched library, clone:E430014M17product:cathepsin C, full insert sequence
Length = 612, Score = 221 bits (115), Expect = 2e-54, Identities = 189/226 (83%)

>gb|BC064286.1|Danio rerio cathepsin C, mRNA (cDNA clone MGC:77609 IMAGE:6996489),complete cds
Length = 3170, Score = 183 bits (95), Expect = 9e-43, Identities = 498/692 (71%), Gaps = 5/692 (0%)

>gb|AF058717.1| Volvox carteri f. nagariensis clone B cysteine protease mRNA,complete cds
Length = 3798, Score = 69.9 bits (36), Expect = 1e-08, Identities = 52/60 (86%)

>gb|U69120.1|PWU69120 Paragonimus westermani cysteine proteinase PWCP1 mRNA, partial cds
Length = 1414, Score = 62.2 bits (32), Expect = 3e-06, Identities = 48/56 (85%)

>gb|AY069923.1| Ascaris suum cathepsin L (cpl-1) mRNA, partial cds
Length = 668, Score = 58.4 bits (30), Expect = 4e-05, Identities = 44/51 (86%)

>dbj|AK110539.1| Oryza sativa (japonica cultivar-group) cDNA clone:002-168-B01, fullinsert sequence
Length = 2375, Score = 58.4 bits (30), Expect = 4e-05, Identities = 40/45 (88%)

>gb|AF362768.1|AF362768 Paragonimus westermani cysteine proteinase mRNA, complete cds
Length = 1053, Score = 58.4 bits (30), Expect = 4e-05, Identities = 48/57 (84%)

>gb|AF031819.1|AF031819 Brugia pahangi cathepsin L-like cysteine protease mRNA, complete cds
Length = 1188, Score = 58.4 bits (30), Expect = 4e-05, Identities = 38/42 (90%)

>gb|L39939.1 |CELCPR6G Caenorhabditis elegans cathepsin B-like cysteine proteinase (cpr-6)gene, complete cds
Length = 2289, Score = 58.4 bits (30), Expect = 4e-05, Identities = 52/63 (82%)

>gb|L39894.1|CELCPR6A Caenorhabditis elegans Bristol N2 cathepsin B-like cysteineproteinase (cpr-6) mRNA, complete cds
Length = 1261, Score = 58.4 bits (30), Expect = 4e-05, Identities = 52/63 (82%)

>gb|AF320084.1| Ancylostoma caninum cathepsin L-like protease (cpl-1) mRNA, partialcds
Length = 855, Score = 56.4 bits (29), Expect = 1e-04, Identities = 49/59 (83%)

>gb|AF071801.1|AF071801 Paragonimus westermani cysteine proteinase mRNA, complete cds
Length = 869, Score = 52.6 bits (27), Expect = 0.002, Identities = 47/57 (82%)

>gb|BC066625.1| Danio rerio hypothetical protein MGC66267, mRNA (cDNA cloneIMAGE:6960075), partial cds
Length = 1571, Score = 52.6 bits (27), Expect = 0.002, Identities = 45/54 (83%)

>gb|BC054668.1| Danio rerio hypothetical protein MGC66267, mRNA (cDNA clone MGC:66267IMAGE:5410623), complete cds
Length = 1664, Score = 52.6 bits (27), Expect = 0.002, Identities = 45/54 (83%)

>ref|NM_200426.1| Danio rerio hypothetical protein MGC66267 (zgc:66267), mRNA
Length = 1664, Score = 52.6 bits (27), Expect = 0.002, Identities = 45/54 (83%).

**Example 2: Expression profiling**

Total cellular RNA was isolated from cells by one of two standard methods: 1) guanidine isothiocyanate/Cesium chloride density gradient centrifugation [Kellogg, (1990)]; or with the Tri-Reagent protocol according to the manufacturer's specifications (Molecular Research Center, Inc., Cincinatti, Ohio). Total RNA prepared by the Tri-reagent protocol was treated with DNAse I to remove genomic DNA contamination.

For relative quantitation of the mRNA distribution of DPP1, total RNA from each cell or tissue source was first reverse transcribed. 85 μg of total RNA was reverse transcribed using 1 μmole random hexamer primers, 0.5 mM each of dATP, dCTP, dGTP and dTTP (Qiagen, Hilden, Germany), 3000 U RnaseQut (Invitrogen, Groningen, Netherlands) in a final volume of 680 μl. The first strand synthesis buffer and Omniscript reverse transcriptase (2 u/μl) were from (Qiagen, Hilden, Germany). The reaction was incubated at 37°C for 90 minutes and cooled on ice. The volume was adjusted to 6800 μl with water, yielding a final concentration of 12.5 ng/μl of starting RNA.

For relative quantitation of the distribution of DPP1 mRNA in cells and tissues the Perkin Elmer ABI Prism RTM. 7700 Sequence Detection system or Biorad iCycler was used according to the manufacturer's specifications and protocols. PCR reactions were set up to quantitate DPP1 and the housekeeping genes HPRT (hypoxanthine phosphoribosyltransferase), GAPDH (glyceraldehyde-3-phosphate dehydrogenase), ß-actin, and others. Forward and reverse primers and probes for DPP1 were designed using the Perkin Elmer ABI Primer Express™ software and were synthesized by TibMolBiol (Berlin, Germany). The DPP1 forward primer sequence was: Primer1 (SEQ ID NO: 3). The DPP1 reverse primer sequence was Primer2 (SEQ ID NO: 4). Probe1 (SEQ ID NO: 5), labelled with FAM (carboxyfluorescein succinimidyl ester) as the reporter dye and TAMRA (carboxytetramethylrhodamine) as the quencher, is used as a probe for DPP1. The following reagents were prepared in a total of 25 μl : 1x TaqMan buffer A, 5.5 mM $MgCl_2$, 200 nM of dATP, dCTP, dGTP, and dUTP, 0.025 U/μl AmpliTaq Gold™, 0.01 U/ μl AmpErase and Probe1 (SEQ ID NO: 5), DPP1 forward and reverse primers each at 200 nM, 200 nM DPP1 FAM/TAMRA-labelled probe, and 5 μl of template cDNA. Thermal cycling parameters were 2 min at 50°C, followed by 10 min at 95°C, followed by 40 cycles of melting at 95°C for 15 sec and annealing/extending at 60°C for 1 min.

*Calculation of corrected CT values*

The CT (threshold cycle) value is calculated as described in the "Quantitative determination of nucleic acids" section. The CF-value (factor for threshold cycle correction) is calculated as follows :

1. PCR reactions were set up to quantitate the housekeeping genes (HKG) for each cDNA sample.

2. $CT_{HKG}$-values (threshold cycle for housekeeping gene) were calculated as described in the "Quantitative determination of nucleic acids" section.

3. $CT_{HKG}$-mean values (CT mean value of all HKG tested on one cDNAs) of all HKG for each cDNA are calculated (n = number of HKG):

$$CT_{HKG\text{-}n}\text{-mean value} = (CT_{HKG1}\text{-value} + CT_{HKG2}\text{-value} + \ldots + CT_{HKG\text{-}n}\text{-value}) / n$$

4.

$$CT_{pannel}\text{ mean value (CT mean value of all HKG in all tested cDNAs)} =$$

$$(CT_{HKG1}\text{-mean value} + CT_{HKG2}\text{-mean value} + \ldots + CT_{HKG\text{-}y}\text{-mean value}) / y$$

(y = number of cDNAs)

5.

$$CF_{cDNA\text{-}n}\text{ (correction factor for cDNA n)} = CT_{pannel}\text{-mean value} - CT_{HKG\text{-}n}\text{-mean value}$$

6.

$$CT_{cDNA\text{-}n}\text{ (CT value of the tested gene for the cDNA n)} + CF_{cDNA\text{-}n}\text{ (correction factor for cDNA n)} = CT_{cor\text{-}cDNA\text{-}n}\text{ (corrected CT value for a gene on cDNA n)}$$

*Calculation of relative expression*

Definition : highest $CT_{cor\text{-}cDNA\text{-}n} \neq 40$ is defined as $CT_{cor\text{-}cDNA}$ [high]

$$\text{Relative Expression} = 2^{(CTcor\text{-}cDNA[high]\ -\ CTcor\text{-}cDNA\text{-}n)}$$

*Tissues*

The expression of DPP1 was investigated in the tissues in table 1.

*Expression profile*

The results of the the mRNA-quantification (expression profiling) is shown in Table 1.

*Table 1: Relative expression of DPP1 in various human tissues.*

| | |
|---|---|
| T-cells peripheral blood CD4+ | 290 |
| T-cells peripheral blood CD4+ | 345 |
| T-cells peripheral blood CD4+ D117 II virus infected | 383 |
| T-cells peripheral blood CD4+ D34 virus infected | 560 |
| monocytes | 1448 |
| monocytes HIV-1 infected | 1795 |
| fetal heart | 77 |
| heart | 133 |
| heart | 149 |
| heart | 223 |
| heart | 123 |
| heart myocardial infarction | 375 |
| heart myocardial infarction | 422 |
| heart myocardial infarction | 309 |
| pericardium | 191 |
| heart atrium (right) | 519 |
| heart atrium (right) | 228 |
| heart atrium (left) | 324 |
| heart atrium (left) | 422 |
| heart ventricle (left) | 9 |
| heart ventricle (left) | 260 |
| heart ventricle (right) | 14 |
| heart ventricle (right) | 3566 |
| heart apex | 443 |
| Purkinje fibers | 402 |
| interventricular septum | 172 |
| fetal aorta | 72 |
| aorta | 15 |
| aorta | 55 |
| aorta | 47 |
| arcus aorta | 79 |
| aorta valve | 31 |
| artery | 36 |
| coronary artery | 217 |
| coronary artery | 38 |
| coronary artery | 43 |
| pulmonary artery | 17 |
| carotid artery | 82 |
| mesenteric artery | 42 |
| arteria radialis | 47 |
| vein | 49 |
| pulmonic valve | 30 |
| vein (saphena magna) | 5 |
| (caval) vein | 1 |
| coronary artery endothel cells | 315 |
| coronary artery smooth muscle primary cells | 936 |
| aortic smooth muscle cells | 484 |

(continued)

| | |
|---|---|
| pulmonary artery smooth muscle cells | 541 |
| aortic endothel cells | 541 |
| HUVEC cells | 541 |
| pulmonary artery endothel cells | 1243 |
| iliac artery endothel cells | 1075 |
| | |
| skin | 1269 |
| adrenal gland | 626 |
| thyroid | 1038 |
| thyroid tumor | 942 |
| pancreas | 223 |
| pancreas liver cirrhosis | 194 |
| | |
| esophagus | 70 |
| esophagus tumor | 19619 |
| stomach | 1629 |
| stomach tumor | 2998 |
| colon | 1038 |
| colon tumor | 405 |
| small intestine | 2048 |
| ileum | 360 |
| ileum tumor | 2150 |
| ileum chronic inflammation | 2 |
| rectum | 350 |
| rectum tumor | 61 |
| fetal liver | 184 |
| liver | 407 |
| liver | 65 |
| liver | 38 |
| liver liver cirrhosis | 1278 |
| liver lupus disease | 413 |
| liver tumor | 4270 |
| HEP G2 cells | 879 |
| | |
| leukocytes (peripheral blood) | 982 |
| Jurkat (T-cells) | 849 |
| Raji (B-cells) | 6123 |
| bone marrow | 750 |
| erythrocytes | 23 |
| lymphnode | 232 |
| thymus | 324 |
| thrombocytes | 21 |
| bone marrow stromal cells | 2998 |
| bone marrow CD71+ cells | 11 |
| bone marrow CD33+ cells | 164 |
| bone marrow CD34+ cells | 91 |
| bone marrow CD15+ cells | 14 |
| cord blood CD71+ cells | 3 |
| cord blood CD34+ cells | 115 |
| neutrophils cord blood | 263 |
| T-cells peripheral blood CD8+ | 516 |

(continued)

| | |
|---|---|
| monocytes peripheral blood CD14+ | 1808 |
| B-cells peripheral blood CD 19+ | 189 |
| neutrophils peripheral blood | 644 |
| spleen | 1296 |
| spleen liver cirrhosis | 1060 |
| | |
| skeletal muscle | 422 |
| cartilage | 592 |
| bone connective tissue | 290 |
| adipose | 635 |
| adipose | 9541 |
| adipose | 648 |
| fetal adipose | 1734 |
| adipose (subcutaneous) BMI 21.74 | 55 |
| adipose (subcutaneous) BMI 35.04 | 20 |
| brain | 136 |
| cerebellum | 17 |
| cerebral cortex | 72 |
| frontal lobe | 64 |
| occipital lobe | 126 |
| parietal lobe | 106 |
| temporal lobe | 133 |
| substantia nigra | 49 |
| caudatum | 98 |
| corpus callosum | 232 |
| nucleus accumbens | 6 |
| putamen | 17 |
| hippocampus | 128 |
| thalamus | 104 |
| posteroventral thalamus | 20 |
| dorsalmedial thalamus | 10 |
| hypothalamus | 185 |
| dorsal root ganglia | 32 |
| spinal cord | 541 |
| spinal cord (ventral horn) | 22 |
| spinal cord (dorsal horn) | 3 |
| glial tumor H4 cells | 564 |
| astrocytes | 13 |
| retina | 3 |
| | |
| fetal lung | 1144 |
| fetal lung fibroblast IMR-90 cells | 52499 |
| fetal lung fibroblast MRC-5 cells | 1082 |
| lung | 164 |
| lung | 343 |
| lung | 74 |
| lung right upper lobe | 481 |
| lung right mid lobe | 286 |
| lung right lower lobe | 228 |
| lung lupus disease | 172 |
| lung tumor | 254 |

(continued)

| | |
|---|---|
| lung COPD | 56 |
| trachea | 2665 |
| primary bronchia | 97 |
| secondary bronchia | 44 |
| bronchial epithelial cells | 1938 |
| bronchial smooth muscle cells | 709 |
| small airway epithelial cells | 1795 |
| | |
| cervix | 89 |
| testis | 910 |
| HeLa cells (cervix tumor) | 1351 |
| placenta | 1361 |
| uterus | 452 |
| uterus tumor | 7913 |
| ovary | 278 |
| ovary tumor | 3566 |
| breast | 263 |
| breast tumor | 1911 |
| mammary gland | 329 |
| | |
| prostate | 431 |
| prostate | 428 |
| prostate | 385 |
| prostate BPH | 16 |
| prostate tumor | 111 |
| bladder | 657 |
| bladder | 635 |
| bladder | 352 |
| ureter | 61 |
| penis | 16 |
| corpus cavernosum | 26 |
| fetal kidney | 1184 |
| kidney | 2195 |
| kidney | 153 |
| kidney | 1808 |
| kidney tumor | 7750 |
| renal epithelial cells | 1409 |
| HEK 293 cells | 405 |

**Example 3: Antisense Analysis**

Knowledge of the correct, complete cDNA sequence coding for DPP1 enables its use as a tool for antisense technology in the investigation of gene function. Oligonucleotides, cDNA or genomic fragments comprising the antisense strand of a polynucleotide coding for DPP are used either in vitro or in vivo to inhibit translation of the mRNA. Such technology is now well known in the art, and antisense molecules can be designed at various locations along the nucleotide sequences. By treatment of cells or whole test animals with such antisense sequences, the gene of interest is effectively turned off. Frequently, the function of the gene is ascertained by observing behavior at the intracellular, cellular, tissue or organismal level (e.g., lethality, loss of differentiated function, changes in morphology, etc.).

In addition to using sequences constructed to interrupt transcription of a particular open reading frame, modifications of gene expression is obtained by designing antisense sequences to intron regions, promoter/enhancer elements, or even to trans-acting regulatory genes.

**Example 4: Expression of DPP1**

Expression of DPP1 is accomplished by subcloning the cDNAs into appropriate expression vectors and transfecting the vectors into expression hosts such as, e.g., *E. coli.* In a particular case, the vector is engineered such that it contains a promoter for β-galactosidase, upstream of the cloning site, followed by sequence containing the amino-terminal Methionine and the subsequent seven residues of β-galactosidase. Immediately following these eight residues is an engineered bacteriophage promoter useful for artificial priming and transcription and for providing a number of unique endonuclease restriction sites for cloning.

Induction of the isolated, transfected bacterial strain with Isopropyl-ß-D-thiogalactopyranoside (IPTG) using standard methods produces a fusion protein corresponding to the first seven residues of β-galactosidase, about 15 residues of "linker", and the peptide encoded within the cDNA. Since cDNA clone inserts are generated by an essentially random process, there is probability of 33% that the included cDNA will lie in the correct reading frame for proper translation. If the cDNA is not in the proper reading frame, it is obtained by deletion or insertion of the appropriate number of bases using well known methods including in vitro mutagenesis, digestion with exonuclease III or mung bean nuclease, or the inclusion of an oligonucleotide linker of appropriate length.

The DPP1 cDNA is shuttled into other vectors known to be useful for expression of proteins in specific hosts. Oligonucleotide primers containing cloning sites as well as a segment of DNA (about 25 bases) sufficient to hybridize to stretches at both ends of the target cDNA is synthesized chemically by standard methods. These primers are then used to amplify the desired gene segment by PCR. The resulting gene segment is digested with appropriate restriction enzymes under standard conditions and isolated by gel electrophoresis. Alternately, similar gene segments are produced by digestion of the cDNA with appropriate restriction enzymes. Using appropriate primers, segments of coding sequence from more than one gene are ligated together and cloned in appropriate vectors. It is possible to optimize expression by construction of such chimeric sequences.

Suitable expression hosts for such chimeric molecules include, but are not limited to, mammalian cells such as Chinese Hamster Ovary (CHO) and human 293 cells., insect cells such as Sf9 cells, yeast cells such as *Saccharomyces cerevisiae* and bacterial cells such as *E. coli.* For each of these cell systems, a useful expression vector also includes an origin of replication to allow propagation in bacteria, and a selectable marker such as the β-lactamase antibiotic resistance gene to allow plasmid selection in bacteria. In addition, the vector may include a second selectable marker such as the neomycin phosphotransferase gene to allow selection in transfected eukaryotic host cells. Vectors for use in eukaryotic expression hosts require RNA processing elements such as 3' polyadenylation sequences if such are not part of the cDNA of interest.

Additionally, the vector contains promoters or enhancers which increase gene expression. Such promoters are host specific and include MMTV, SV40, and metallothionine promoters for CHO cells; trp, lac, tac and T7 promoters for bacterial hosts; and alpha factor, alcohol oxidase and PGH promoters for yeast. Transcription enhancers, such as the rous sarcoma virus enhancer, are used in mammalian host cells. Once homogeneous cultures of recombinant cells are obtained through standard culture methods, large quantities of recombinantly produced DPP 1 are recovered from the conditioned medium and analyzed using chromatographic methods known in the art. For example, DPP1 can be cloned into the expression vector pcDNA3, as exemplified herein. This product can be used to transform, for example, HEK293 or COS by methodology standard in the art. Specifically, for example, using Lipofectamine (Gibco BRL catolog no. 18324-020) mediated gene transfer.

**Example 5: Isolation of Recombinant DPP1**

DPP1 is expressed as a chimeric protein with one or more additional polypeptide domains added to facilitate protein purification. Such purification facilitating domains include, but are not limited to, metal chelating peptides such as histidine-tryptophan modules that allow purification on immobilized metals [Appa Rao, 1997] and the domain utilized in the FLAGS extension/affinity purification system (Immunex Corp., Seattle, Washington). The inclusion of a cleavable linker sequence such as Factor Xa or enterokinase (Invitrogen, Groningen, The Netherlands) between the purification domain and the DPP1 sequence is useful to facilitate expression of DPP1.

The following example provides a method for purifying DPP 1.

DPP1 is generated using the baculovirus expression system BAC-TO-BAC (GIBCO BRL) based on *Autographa californica* nuclear polyhedrosis virus (AcNPV) infection of *Spodoptera frugiperda* insect cells (Sf9 cells).

cDNA encoding proteases cloned into either the donor plasmid pFASTBAC1 or pFASTBAC-HT which contain a mini-Tn7 transposition element. The recombinant plasmid is transformed into DH10BAC competent cells which contain the parent bacmid bMON14272 (AcNPV infectious DNA) and a helper plasmid. The mini-Tn7 element on the pFASTBAC donor can transpose to the attTn7 attachment site on the bacmid thus introducing the protease gene into the viral genome. Colonies containing recombinant bacmids are identified by disruption of the *lacZ* gene. The protease/bacmid construct can then be isolated and infected into insect cells (Sf9 cells) resulting in the production of infectious recombinant baculovirus particles and expression of either unfused recombinant enzyme (pFastbacl) or DPP1-His fusion protein (pFastbacHT).

Cells are harvested and extracts prepared 24, 48 and 72 hours after transfection. Expression of DPP1 is confirmed by coomassie staining after sodium dodecyl sulphate-polyacrylamide gel electrophoresis (SDS-PAGE) and western blotting onto a PVDF membrane of an unstained SDS-PAGE. The protease-His fusion protein is detected due to the interaction between the Ni-NTA HRP conjugate and the His-tag which is fused to DPP1.

**Example 6: Production of DPP1 Specific Antibodies**

Two approaches are utilized to raise antibodies to DPP1, and each approach is useful for generating either polyclonal or monoclonal antibodies. In one approach, denatured protein from reverse phase HPLC separation is obtained in quantities up to 75 mg. This denatured protein is used to immunize mice or rabbits using standard protocols; about 100 μg are adequate for immunization of a mouse, while up to 1 mg might be used to immunize a rabbit. For identifying mouse hybridomas, the denatured protein is radioiodinated and used to screen potential murine B-cell hybridomas for those which produce antibody. This procedure requires only small quantities of protein, such that 20 mg is sufficient for labeling and screening of several thousand clones.

In the second approach, the amino acid sequence of an appropriate DPP1 domain, as deduced from translation of the cDNA, is analyzed to determine regions of high antigenicity. Oligopeptides comprising appropriate hydrophilic regions are synthesized and used in suitable immunization protocols to raise antibodies. The optimal amino acid sequences for immunization are usually at the C-terminus, the N-terminus and those intervening, hydrophilic regions of the polypeptide which are likely to be exposed to the external environment when the protein is in its natural conformation.

Typically, selected peptides, about 15 residues in length, are synthesized using an Applied Biosystems Peptide Synthesizer Model 431A using fmoc-chemistry and coupled to keyhole limpet hemocyanin (KLH; Sigma, St. Louis, MO) by reaction with M-maleimidobenzoyl-N-hydroxysuccinimide ester, MBS. If necessary, a cysteine is introduced at the N-terminus of the peptide to permit coupling to KLH. Rabbits are immunized with the peptide-KLH complex in complete Freund's adjuvant. The resulting antisera are tested for antipeptide activity by binding the peptide to plastic, blocking with 1% bovine serum albumin, reacting with antisera, washing and reacting with labeled (radioactive or fluorescent), affinity purified, specific goat anti-rabbit IgG.

Hybridomas are prepared and screened using standard techniques. Hybridomas of interest are detected by screening with labeled DPP1 to identify those fusions producing the monoclonal antibody with the desired specificity. In a typical protocol, wells of plates (FAST; Becton-Dickinson, Palo Alto, CA) are coated during incubation with affinity purified, specific rabbit antimouse (or suitable antispecies 1 g) antibodies at 10 mg/ml. The coated wells are blocked with 1% bovine serum albumin, (BSA), washed and incubated with supernatants from hybridomas. After washing the wells are incubated with labeled DPP1 at 1 mg/ml. Supernatants with specific antibodies bind more labeled DPP1 than is detectable in the background. Then clones producing specific antibodies are expanded and subjected to two cycles of cloning at limiting dilution. Cloned hybridomas are injected into pristane-treated mice to produce ascites, and monoclonal antibody is purified from mouse ascitic fluid by affinity chromatography on Protein A. Monoclonal antibodies with affinities of at least
$10^8$ $M^{-1}$, preferably $10^9$ to $10^{10}$ $M^{-1}$ or stronger, are typically made by standard procedures.

**Example 7: Diagnostic Test Using DPP1 Specific Antibodies**

Particular DPP1 antibodies are useful for investigating signal transduction and the diagnosis of infectious or hereditary conditions which are characterized by differences in the amount or distribution of DPP1 or downstream products of an active signaling cascade.

Diagnostic tests for DPP1 include methods utilizing antibody and a label to detect DPP1 in human body fluids, membranes, cells, tissues or extracts of such. The polypeptides and antibodies of the present invention are used with or without modification. Frequently, the polypeptides and antibodies are labeled by joining them, either covalently or noncovalently, with a substance which provides for a detectable signal. A wide variety of labels and conjugation techniques are known and have been reported extensively in both the scientific and patent literature. Suitable labels include radionuclides, enzymes, substrates, cofactors, inhibitors, fluorescent agents, chemiluminescent agents, chromogenic agents, magnetic particles and the like.

A variety of protocols for measuring soluble or membrane-bound DPP1, using either polyclonal or monoclonal antibodies specific for the protein, are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on DPP1 is preferred, but a competitive binding assay may be employed.

**Example 8: Purification of Native DPP1 Using Specific Antibodies**

Native or recombinant DPP1 is purified by immunoaffinity chromatography using antibodies specific for DPP1. In general, an immunoaffinity column is constructed by covalently coupling the anti-TRH antibody to an activated chromatographic resin.

Polyclonal immunoglobulins are prepared from immune sera either by precipitation with ammonium sulfate or by purification on immobilized Protein A (Pharmacia LKB Biotechnology, Piscataway N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated Sepharose (Pharmacia LKB Biotechnology). The antibody is coupled to the resin, the resin is blocked, and the derivative resin is washed according to the manufacturer's instructions.

Such immunoaffinity columns are utilized in the purification of DPP1 by preparing a fraction from cells containing DPP1 in a soluble form. This preparation is derived by solubilization of whole cells or of a subcellular fraction obtained via differential centrifugation (with or without addition of detergent) or by other methods well known in the art. Alternatively, soluble DPP1 containing a signal sequence is secreted in useful quantity into the medium in which the cells are grown.

A soluble DPP1-containing preparation is passed over the immunoaffinity column, and the column is washed under conditions that allow the preferential absorbance of DPP1 (e.g., high ionic strength buffers in the presence of detergent). Then, the column is eluted under conditions that disrupt antibody/protein binding (e.g., a buffer of pH 2-3 or a high concentration of a chaotrope such as urea or thiocyanate ion), and DPP1 is collected.

**Example 9: Drug Screening**

This invention is particularly useful for screening therapeutic compounds by using DPP1 or fragments thereof in any of a variety of drug screening techniques.

The following example provides a system for drug screening measuring the protease activity.

The recombinant protease-His fusion protein can be purified from the crude lysate by metal-affinity chromatography using Ni-NTA agarose. This allows the specific retention of the recombinant material (since this is fused to the His-tag) whilst the endogenous insect proteins are washed off. The recombinant material is then eluted by competition with imidazol.

The activity of DPP1 molecules of the present invention can be measured using a variety of assays that measure DPP1 activity. For example, DPP1 enzyme activity can be assessed by a standard in vitro serine/metallo/... protease assay (see, for example, [U.S. 5,057,414]). Those of skill in the art are aware of a variety of substrates suitable for in vitro assays, such as SucAla-Ala-Pro-Phe-pNA, fluorescein mono-p-guanidinobenzoate hydrochloride, benzyloxycarbonyl-L-Arginyl-S-benzylester, Nalpha-Benzoyl-L-arginine ethyl ester hydrochloride, and the like. In addition, protease assay kits available from commercial sources, such as Calbiochem™ (San Diego, Calif.). For general references, see Barrett (Ed.), Methods in Enzymology, Proteolytic Enzymes: Serine and Cysteine Peptidases (Academic Press Inc. 1994), and Barrett et al., (Eds.), Handbook of Proteolytic Enzymes (Academic Press Inc. 1998).

**Example 10: Rational Drug Design**

The goal of rational drug design is to produce structural analogs of biologically active polypeptides of interest or of small molecules with which they interact, agonists, antagonists, or inhibitors. Any of these examples are used to fashion drugs which are more active or stable forms of the polypeptide or which enhance or interfere with the function of a polypeptide in vivo.

In one approach, the three-dimensional structure of a protein of interest, or of a protein-inhibitor complex, is determined by x-ray crystallography, by computer modeling or, most typically, by a combination of the two approaches. Both the shape and charges of the polypeptide must be ascertained to elucidate the structure and to determine active site(s) of the molecule. Less often, useful information regarding the structure of a polypeptide is gained by modeling based on the structure of homologous proteins. In both cases, relevant structural information is used to design efficient inhibitors. Useful examples of rational drug design include molecules which have improved activity or stability or which act as inhibitors, agonists, or antagonists of native peptides.

It is also possible to isolate a target-specific antibody, selected by functional assay, as described above, and then to solve its crystal structure. This approach, in principle, yields a pharmacore upon which subsequent drug design is based. It is possible to bypass protein crystallography altogether by generating anti-idiotypic antibodies (anti-ids) to a functional, pharmacologically active antibody. As a mirror image of a mirror image, the binding site of the anti-ids is expected to be an analog of the original receptor. The anti-id is then used to identify and isolate peptides from banks of chemically or biologically produced peptides. The isolated peptides then act as the pharmacore.

By virtue of the present invention, sufficient amount of polypeptide are made available to perform such analytical studies as X-ray crystallography. In addition, knowledge of the DPP1 amino acid sequence provided herein provides guidance to those employing computer modeling techniques in place of or in addition to x-ray crystallography.

**Example 11: Identification of Other Members of the Signal Transduction Complex**

Labeled DPP1 is useful as a reagent for the purification of molecules with which it interacts. In one embodiment of affinity purification, DPP1 is covalently coupled to a chromatography column. Cell-free extract derived from synovial cells or putative target cells is passed over the column, and molecules with appropriate affinity bind to DPP1. DPP1-complex is recovered from the column, and the DPP1-binding ligand disassociated and subjected to N-terminal protein sequencing. The amino acid sequence information is then used to identify the captured molecule or to design degenerate oligonucleotide probes for cloning the relevant gene from an appropriate cDNA library.

In an alternate method, antibodies are raised against DPP1, specifically monoclonal antibodies. The monoclonal antibodies are screened to identify those which inhibit the binding of labeled DPP1. These monoclonal antibodies are then used therapeutically.

**Example 12: Use and Administration of Antibodies, Inhibitors, or Antagonists**

Antibodies, inhibitors, or antagonists of DPP1 or other treatments and compunds that are limiters of signal transduction (LSTs), provide different effects when administered therapeutically. LSTs are formulated in a nontoxic, inert, pharmaceutically acceptable aqueous carrier medium preferably at a pH of about 5 to 8, more preferably 6 to 8, although pH may vary according to the characteristics of the antibody, inhibitor, or antagonist being formulated and the condition to be treated. Characteristics of LSTs include solubility of the molecule, its half-life and antigenicity/immunogenicity. These and other characteristics aid in defining an effective carrier. Native human proteins are preferred as LSTs, but organic or synthetic molecules resulting from drug screens are equally effective in particular situations.

LSTs are delivered by known routes of administration including but not limited to topical creams and gels; transmucosal spray and aerosol; transdermal patch and bandage; injectable, intravenous and lavage formulations; and orally administered liquids and pills particularly formulated to resist stomach acid and enzymes. The particular formulation, exact dosage, and route of administration is determined by the attending physician and varies according to each specific situation.

Such determinations are made by considering multiple variables such as the condition to be treated, the LST to be administered, and the pharmacokinetic profile of a particular LST. Additional factors which are taken into account include severity of the disease state, patient's age, weight, gender and diet, time and frequency of LST administration, possible combination with other drugs, reaction sensitivities, and tolerance/response to therapy. Long acting LST formulations might be administered every 3 to 4 days, every week, or once every two weeks depending on half-life and clearance rate of the particular LST.

Normal dosage amounts vary from 0.1 to $10^5$ μg, up to a total dose of about 1 g, depending upon the route of administration. Guidance as to particular dosages and methods of delivery is provided in the literature; see U.S. Pat. Nos. 4,657,760; 5,206,344; or 5,225,212. Those skilled in the art employ different formulations for different LSTs. Administration to cells such as nerve cells necessitates delivery in a manner different from that to other cells such as vascular endothelial cells.

It is contemplated that abnormal signal transduction, trauma, or diseases which trigger DPP1 activity are treatable with LSTs. These conditions or diseases are specifically diagnosed by the tests discussed above, and such testing should be performed in suspected cases of viral, bacterial or fungal infections, allergic responses, mechanical injury associated with trauma, hereditary diseases, lymphoma or carcinoma, or other conditions which activate the genes of lymphoid or neuronal tissues.

**Example 13: Production of Non-human Transgenic Animals**

Animal model systems which elucidate the physiological and behavioral roles of the DPP1 are produced by creating nonhuman transgenic animals in which the activity of the DPP1 is either increased or decreased, or the amino acid sequence of the expressed DPP1 is altered, by a variety of techniques. Examples of these techniques include, but are not limited to: 1) Insertion of normal or mutant versions of DNA encoding a DPP1, by microinjection, electroporation, retroviral transfection or other means well known to those skilled in the art, into appropriately fertilized embryos in order to produce a transgenic animal or 2) homologous recombination of mutant or normal, human or animal versions of these genes with the native gene locus in transgenic animals to alter the regulation of expression or the structure of these DPP1 sequences. The technique of homologous recombination is well known in the art. It replaces the native gene with the inserted gene and hence is useful for producing an animal that cannot express native DPP1s but does express, for example, an inserted mutant DPP1, which has replaced the native DPP1 in the animal's genome by recombination, resulting in underexpression of the transporter. Microinjection adds genes to the genome, but does not remove them, and the technique is useful for producing an animal which expresses its own and added DPP1, resulting in overexpression of the DPP1.

One means available for producing a transgenic animal, with a mouse as an example, is as follows:

Female mice are mated, and the resulting fertilized eggs are dissected out of their oviducts. The eggs are stored in an appropriate medium such as cesiumchloride M2 medium. DNA or cDNA encoding DPP1 is purified from a vector by methods well known to the one skilled in the art. Inducible promoters may be fused with the coding region of the DNA to provide an experimental means to regulate expression of the transgene. Alternatively or in addition, tissue specific regulatory elements may be fused with the coding region to permit tissue-specific expression of the transgene. The DNA, in an appropriately buffered solution, is put into a microinjection needle (which may be made from capillary tubing using a piper puller) and the egg to be injected is put in a depression slide. The needle is inserted into the pronucleus of the egg, and the DNA solution is injected. The injected egg is then transferred into the oviduct of a pseudopregnant mouse which is a mouse stimulated by the appropriate hormones in order to maintain false pregnancy, where it proceeds to the uterus, implants, and develops to term. As noted above, microinjection is not the only method for inserting DNA into the egg but is used here only for exemplary purposes.

## References

EP 1 394 274
U.S. 4,522,811
U.S. 5,057,414
U.S. 5,283,317
U.S. 5,565,332
U.S. 5,637,462
U.S. 5,723,323.
U.S. 5,747,334
U.S. 5,783,384
U.S. 5,885,814
U.S. 5,985,629
WO 84/03564
WO 93/03151
WO 94/13804
WO 02/068579
Altschul SF, Madden TL, Schaffer AA, Zhang J, Zhang Z, Miller W, Lipman DJ; Nucleic Acids Res 1997 Sep 1; 25 (17): 3389-402
Appa Rao et al., 1997, Protein Expr Purif Nov, 11 (2): 201-8
Avalle et al., Ann. N Y Acad.Sci. 864:118 (1998)).
Barnes, 2000, Chest, 117:10S14S
Barrett et al., (Eds.), Handbook of Proteolytic Enzymes (Academic Press Inc. 1998).
Barrett (Ed.), Methods in Enzymology, Proteolytic Enzymes: Serine and Cysteine Peptidases (Academic Press Inc. 1994)
Botstein et al., 1980, Am J Hum Genet. 32: 314-31
Colbere-Garapin et al., 1981, J. Mol. Biol. 150, 1-14
Cunningham and Wells, J. Mol. Biol. 234:554 (1993).
Engelhard et al., 1994, Proc. Nat. Acad. Sci. 91, 3224-3227
Friboulet et al., Appl. Biochem. Biotechnol. 47:229 (1994)
Gergen and Weiss , 1992, Am Rev Respir Dis 146:823-824
Gibson et al., 1996, Genome Research 6: 995-1001
Haseloff et al., 1988 , Nature 334, 585-591
Heid et al., 1996, Genome Research 6: 986-994
Holland et al., 1991, PNAS 88: 7276-7280
Jeffreys et al., 1985, Nature 316: 76-9
Johnson et al., 1989, Endoc. Rev. 10, 317-331
Joron et al., Ann. N Y Acad. Sci. 672:216 (1992)

Karlsson, Immunol. Methods 145:229 (1991)
Kellogg et al., 1990, Anal. Biochem. 189:202-208
Lam, 1997, Anticancer Drug Res. 12(3):145-67
Livak et al., 1995 , PCR Methods and Applications 357-362
Logan, Shenk, 1984, Proc. Natl. Acad. Sci. 81, 3655-3659
Lowy et al., 1980, Cell 22, 817-23
Maddox et al., 1983, J. Exp. Med. 158, 1211-1216
Monfardini et al., Proc. Assoc. Am. Physicians 108:420 (1996)
McConnell et al., 1992, Science 257, 1906-1912
Nicholls et al., 1993, J. Immunol. Meth. 165, 81-91
Paris et al. (1995), FEBS Lett. 369: 326-330
Pham et al. (1997), J. Biol. Chem. 272: 10695-10703
Piatak et al., 1993, BioTechniques 14:70-81
Piatak et al., 1993, Science 259:1749-1754
Porath et al., 1992, Prot. Exp. Purif. 3, 263-281
Rao et al. (1997), J. Biol. Chem. 272: 10260-10265
Roberge et al., 1995, Science 269, 202-204
Scott and Smith (1990) Science 249:386-390
Sjolander, Urbaniczky, 1991, Anal. Chem. 63, 2338-2345
Szabo et al., 1995, Curr. Opin. Struct. Biol. 5, 699-705
Thomas, 1980, Proc. Nat. Acad. Sci., 77:5201-5205
Uhlmann et al., 1987, Tetrahedron. Lett. 215, 3539-3542
Weber et al., 1990, Genomics 7: 524-30
Wigler et al., 1977, Cell 11, 223-32
Wigler et al., 1980, Proc. Natl. Acad. Sci. 77, 3567-70

**Claims**

**1.** A method of screening for therapeutic agents useful in the treatment of cancer in a mammal comprising the steps of

i) contacting a test compound with a DPP1 polypeptide,
ii) detect binding of said test compound to said DPP1 polypeptide.

**2.** A method of screening for therapeutic agents useful in the treatment of cancer in a mammal comprising the steps of

i) determining the activity of a DPP1 polypeptide at a certain concentration of a test compound or in the absence of said test compound,
ii) determining the activity of said polypeptide at a different concentration of said test compound.

**3.** A method of screening for therapeutic agents useful in the treatment of cancer in a mammal comprising the steps of

i) determining the activity of a DPP1 polypeptide at a certain concentration of a test compound,
ii) determining the activity of a DPP1 polypeptide at the presence of a compound known to be a regulator of a DPP1 polypeptide.

**4.** The method of any of claims 1 to 3, wherein the cell is in vitro.

**5.** The method of any of claims 1 to 3, wherein the step of contacting is in a cell-free system.

**6.** The method of any of claims 1 to 3, wherein the polypeptide is coupled to a detectable label.

7. The method of any of claims 1 to 3, wherein the compound is coupled to a detectable label.

8. The method of any of claims 1 to 3, wherein the test compound displaces a ligand which is first bound to the polypeptide.

9. A method of screening for therapeutic agents useful in the treatment of cancer in a mammal comprising the steps of

   i) contacting a test compound with a DPP1 polynucleotide,
   ii) detect binding of said test compound to said DPP1 polynucleotide.

10. The method of claim 12 wherein the nucleic acid molecule is RNA.

11. The method of claim 12 wherein the contacting step is in or at the surface of a cell.

12. A method of diagnosing cancer in a mammal comprising the steps of

   i) determining the amount of a DPP1 polynucleotide in a sample taken from said mammal,
   ii) determining the amount of DPP1 polynucleotide in healthy and/or diseased mammals.

13. A pharmaceutical composition for the treatment of cancer in a mammal comprising a therapeutic agent which regulates the activity of a DPP1 polypeptide, wherein said therapeutic agent is

   i) an antisense oligonucleotide,
   ii) an antibody, or
   iii) a ribozyme.

14. A pharmaceutical composition for the treatment of cancer in a mammal comprising a DPP1 polynucleotide.

15. A pharmaceutical composition for the treatment of cancer in a mammal comprising a DPP1 polypeptide.

**Fig. 1**

**SEQ ID NO: 1**

AATTCTTCACCTCTTTTCTCAGCTCCCTGCAGCATGGGTGCTGGGCCC
TCCTTGCTGCTCGCCGCCCTCCTGCTGCTTCTCTCCGGCGACGGCGCC
GTGCGCTGCGACACACCTGCCAACTGCACCTATCTTGACCTGCTGGGC
ACCTGGGTCTTCCAGGTGGGCTCCAGCGGTTCCCAGCGCGATGTCAAC
TGCTCGGTTATGGGACCACAAGAAAAAAAAGTAGTGGTGTACCTTCAG
AAGCTGGATACAGCATATGATGACCTTGGCAATTCTGGCCATTTCACC
ATCATTTACAACCAAGGCTTTGAGATTGTGTTGAATGACTACAAGTGG
TTTGCCTTTTTTAAGTATAAAGAAGAGGGCAGCAAGGTGACCACTTAC
TGCAACGAGACAATGACTGGGTGGGTGCATGATGTGTTGGGCCGGAAC
TGGGCTTGTTTCACCGGAAAGAAGGTGGGAACTGCCTCTGAGAATGTG
TATGTCAACACAGCACACCTTAAGAATTCTCAGGAAAAGTATTCTAAT
AGGCTCTACAAGTATGATCACAACTTTGTGAAAGCTATCAATGCCATT
CAGAAGTCTTGGACTGCAACTACATACATGGAATATGAGACTCTTACC
CTGGGAGATATGATTAGGAGAAGTGGTGGCCACAGTCGAAAAATCCCA
AGGCCCAAACCTGCACCACTGACTGCTGAAATACAGCAAAAGATTTTG
CATTTGCCAACATCTTGGGACTGGAGAAATGTTCATGGTATCAATTTT
GTCAGTCCTGTTCGAAACCAAGCATCCTGTGGCAGCTGCTACTCATTT
GCTTCTATGGGTATGCTAGAAGCGAGAATCCGTATACTAACCAACAAT
TCTCAGACCCCAATCCTAAGCCCTCAGGAGGTTGTGTCTTGTAGCCAG
TATGCTCAAGGCTGTGAAGGCGGCTTCCCATACCTTATTGCAGGAAAG
TACGCCCAAGATTTTGGGCTGGTGGAAGAAGCTTGCTTCCCCTACACA
GGCACTGATTCTCCATGCAAAATGAAGGAAGACTGCTTTCGTTATTAC
TCCTCTGAGTACCACTATGTAGGAGGTTTCTATGGAGGCTGCAATGAA
GCCCTGATGAAGCTTGAGTTGGTCCATCATGGGCCCATGGCAGTTGCT
TTTGAAGTATATGATGACTTCCTCCACTACAAAAAGGGGATCTACCAC
CACACTGGTCTAAGAGACCCTTTCAACCCCTTTGAGCTGACTAATCAT
GCTGTTCTGCTTGTGGGCTATGGCACTGACTCAGCCTCTGGGATGGAT
TACTGGATTGTTAAAAACAGCTGGGGCACCGGCTGGGGTGAGAATGGC

TACTTCCGGATCCGCAGAGGAACTGATGAGTGTGCAATTGAGAGCATA
GCAGTGGCAGCCACACCAATTCCTAAATTGTAGGGTATGCCTTCCAGT
ATTTCATAATGATCTGCATCAGTTGTAAAGGGGAATTGGTATATTCAC
AGACTGTAGACTTTCAGCAGCAATCTCAGAAGCTTACAAATAGATTTC
CATGAAGATATTTGTCTTCAGAATTAAAACTGCCCTTAATTTTAATAT
ACCTTTCAATCGGCCACTGGCCATTTTTTTCTAAGTATTCAATTAAGT
GGGAATTTTCTGGAAGATGGTCAGCTATGAAGTAATAGAGTTTGCTTA
ATCATTTGTAATTCAAACATGCTATATTTTTTAAAATCAATGTGAAAA
CATAGACTTATTTTTAAATTGTACCAATCACAAGAAAATAATGGCAAT
AATTATCAAAACTTTTAAAATAGATGCTCATATTTTTAAAATAAAGTT
TTAAAAATAACTGC

**Fig. 2**

**SEQ ID NO: 2**

MGAGPSLLLAALLLLLSGDGAVRCDTPANCTYLDLLGTWVFQVGSSGS
QRDVNCSVMGPQEKKVVVYLQKLDTAYDDLGNSGHFTIIYNQGFEIVL
NDYKWFAFFKYKEEGSKVTTYCNETMTGWVHDVLGRNWACFTGKKVGT
ASENVYVNTAHLKNSQEKYSNRLYKYDHNFVKAINAIQKSWTATTYME
YETLTLGDMIRRSGGHSRKIPRPKPAPLTAEIQQKILHLPTSWDWRNV
HGINFVSPVRNQASCGSCYSFASMGMLEARIRILTNNSQTPILSPQEV
VSCSQYAQGCEGGFPYLIAGKYAQDFGLVEEACFPYTGTDSPCKMKED
CFRYYSSEYHYVGGFYGGCNEALMKLELVHHGPMAVAFEVYDDFLHYK
KGIYHHTGLRDPFNPFELTNHAVLLVGYGTDSASGMDYWIVKNSWGTG
WGENGYFRIRRGTDECAIESIAVAATPIPKL

**Fig. 3**

**SEQ ID NO: 3**

5'-ATTCTGGCCATTTCACCATC-3'

**Fig. 4**

**SEQ ID NO: 4**

5'-CCTTGCTGCCCTCTTCTTTA-3'

**Fig. 5**

**SEQ ID NO: 5**

5'-CAACCAAGGCTTTGAGATTGTGTTGA-3'

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1394274 A
- WO 02068579 A
- US 5637462 A
- US 5783384 A
- US 5747334 A
- US 5723323 A
- US 5985629 A
- US 5885814 A
- US 5565332 A
- WO 9303151 A
- WO 9413804 A
- US 5641673 A
- US 5057414 A
- WO 8403564 A
- US 5283317 A
- US 4522811 A
- US 4657760 A
- US 5206344 A
- US 5225212 A


### Non-patent literature cited in the description


- **PIATAK.** *BioTechniques,* 1993
- **PIATAK.** TaqMan method. *Science,* 1993
- **KAY et al.** Phage Display of Peptides and Proteins. Academic Press, 1996
- Methods in Enzymology, Proteolytic Enzymes: Serine and Cysteine Peptidases. Academic Press Inc, 1994
- Handbook of Proteolytic Enzymes. Academic Press Inc, 1998
- **ALTSCHUL SF ; MADDEN TL ; SCHAFFER AA ; ZHANG J ; ZHANG Z ; MILLER W ; LIPMAN DJ.** *Nucleic Acids Res,* 01 September 1997, vol. 25 (17), 3389-402
- **APPA RAO et al.** *Protein Expr Purif,* November 1997, vol. 11 (2), 201-8
- **AVALLE et al.** *Ann. N Y Acad.Sci.,* 1998, vol. 864, 118
- **BARNES.** *Chest,* 2000, vol. 117, 10S14S
- Handbook of Proteolytic Enzymes. Academic Press, 1998
- Proteolytic Enzymes: Serine and Cysteine Peptidases. Methods in Enzymology. Academic Press, 1994
- **BOTSTEIN et al.** *Am J Hum Genet.,* 1980, vol. 32, 314-31
- **COLBERE-GARAPIN et al.** *J. Mol. Biol.,* 1981, vol. 150, 1-14
- **CUNNINGHAM ; WELLS.** *J. Mol. Biol.,* 1993, vol. 234, 554
- **ENGELHARD et al.** *Proc. Nat. Acad. Sci.,* 1994, vol. 91, 3224-3227
- **FRIBOULET et al.** *Appl. Biochem. Biotechnol.,* 1994, vol. 47, 229
- **GERGEN ; WEISS.** *Am Rev Respir Dis,* 1992, vol. 146, 823-824
- **GIBSON et al.** *Genome Research,* 1996, vol. 6, 995-1001
- **HASELOFF et al.** *Nature,* 1988, vol. 334, 585-591
- **HEID et al.** *Genome Research,* 1996, vol. 6, 986-994
- **HOLLAND et al.** *PNAS,* 1991, vol. 88, 7276-7280
- **JEFFREYS et al.** *Nature,* 1985, vol. 316, 76-9
- **JOHNSON et al.** *Endoc. Rev.,* 1989, vol. 10, 317-331
- **JORON et al.** *Ann. N Y Acad. Sci.,* 1992, vol. 672, 216
- **KARLSSON.** *Immunol. Methods,* 1991, vol. 145, 229
- **KELLOGG et al.** *Anal. Biochem.,* 1990, vol. 189, 202-208
- **LAM.** *Anticancer Drug Res.,* 1997, vol. 12 (3), 145-67
- **LIVAK et al.** *PCR Methods and Applications,* 1995, 357-362
- **LOGAN ; SHENK.** *Proc. Natl. Acad. Sci.,* 1984, vol. 81, 3655-3659
- **LOWY et al.** *Cell,* 1980, vol. 22, 817-23
- **MADDOX et al.** *J. Exp. Med.,* 1983, vol. 158, 1211-1216
- **MONFARDINI et al.** *Proc. Assoc. Am. Physicians,* 1996, vol. 108, 420
- **MCCONNELL et al.** *Science,* 1992, vol. 257, 1906-1912
- **NICHOLLS et al.** *J. Immunol. Meth.,* 1993, vol. 165, 81-91
- **PARIS et al.** *FEBS Lett.,* 1995, vol. 369, 326-330

- **PHAM et al.** *J. Biol. Chem.,* 1997, vol. 272, 10695-10703
- **PIATAK et al.** *BioTechniques,* 1993, vol. 14, 70-81
- **PIATAK et al.** *Science,* 1993, vol. 259, 1749-1754
- **PORATH et al.** *Prot. Exp. Purif.,* 1992, vol. 3, 263-281
- **RAO et al.** *J. Biol. Chem.,* 1997, vol. 272, 10260-10265
- **ROBERGE et al.** *Science,* 1995, vol. 269, 202-204
- **SCOTT ; SMITH.** *Science,* 1990, vol. 249, 386-390
- **SJOLANDER ; URBANICZKY.** *Anal. Chem.,* 1991, vol. 63, 2338-2345
- **SZABO et al.** *Curr. Opin. Struct. Biol.,* 1995, vol. 5, 699-705
- **THOMAS.** *Proc. Nat. Acad. Sci.,* 1980, vol. 77, 5201-5205
- **UHLMANN et al.** *Tetrahedron. Lett.,* vol. 215, 3539-3542
- **WEBER et al.** *Genomics,* 1990, vol. 7, 524-30
- **WIGLER et al.** *Cell,* 1977, vol. 11, 223-32
- **WIGLER et al.** *Proc. Natl. Acad. Sci.,* 1980, vol. 77, 3567-70

SEQUENCE LISTING

<110> Bayer HealthCare AG

<120> Diagnostics and Therapeutics for Diseases Associated with Dipeptidyl-Peptidase 1 (DPP1)

<130> BHC 04 01 130

<160> 5

<170> PatentIn version 3.2

<210> 1
<211> 1838
<212> DNA
<213> Homo sapiens

<400> 1

```
aattcttcac ctcttttctc agctccctgc agcatgggtg ctgggccctc cttgctgctc      60
gccgccctcc tgctgcttct ctccggcgac ggcgccgtgc gctgcgacac acctgccaac     120
tgcacctatc ttgacctgct gggcacctgg gtcttccagg tgggctccag cggttcccag     180
cgcgatgtca actgctcggt tatgggacca caagaaaaaa aagtagtggt gtaccttcag     240
aagctggata cagcatatga tgaccttggc aattctggcc atttcaccat catttacaac     300
caaggctttg agattgtgtt gaatgactac aagtggtttg ccttttttaa gtataaagaa     360
gagggcagca aggtgaccac ttactgcaac gagacaatga ctgggtgggt gcatgatgtg     420
ttgggccgga actgggcttg tttcaccgga aagaaggtgg gaactgcctc tgagaatgtg     480
tatgtcaaca cagcacacct taagaattct caggaaaagt attctaatag gctctacaag     540
tatgatcaca actttgtgaa agctatcaat gccattcaga agtcttggac tgcaactaca     600
tacatggaat atgagactct taccctggga gatatgatta ggagaagtgg tggccacagt     660
cgaaaaatcc caaggcccaa acctgcacca ctgactgctg aaatacagca aaagattttg     720
catttgccaa catcttggga ctggagaaat gttcatggta tcaattttgt cagtcctgtt     780
cgaaaccaag catcctgtgg cagctgctac tcatttgctt ctatgggtat gctagaagcg     840
agaatccgta tactaaccaa caattctcag accccaatcc taagccctca ggaggttgtg     900
tcttgtagcc agtatgctca aggctgtgaa ggcggcttcc cataccttat tgcaggaaag     960
tacgcccaag attttgggct ggtggaagaa gcttgcttcc cctacacagg cactgattct    1020
ccatgcaaaa tgaaggaaga ctgctttcgt tattactcct ctgagtacca ctatgtagga    1080
ggtttctatg gaggctgcaa tgaagccctg atgaagcttg agttggtcca tcatgggccc    1140
atggcagttg cttttgaagt atatgatgac ttcctccact acaaaaaggg gatctaccac    1200
cacactggtc taagagaccc tttcaacccc tttgagctga ctaatcatgc tgttctgctt    1260
gtgggctatg gcactgactc agcctctggg atggattact ggattgttaa aaacagctgg    1320
ggcaccggct ggggtgagaa tggctacttc cggatccgca gaggaactga tgagtgtgca    1380
attgagagca tagcagtggc agccacacca attcctaaat tgtagggtat gccttccagt    1440
atttcataat gatctgcatc agttgtaaag ggaattggta tattcacag actgtagact    1500
```

ttcagcagca atctcagaag cttacaaata gatttccatg aagatatttg tcttcagaat 1560

taaaactgcc cttaatttta atataccttt caatcggcca ctggccattt ttttctaagt 1620

attcaattaa gtgggaattt tctggaagat ggtcagctat gaagtaatag agtttgctta 1680

atcatttgta attcaaacat gctatatttt ttaaaatcaa tgtgaaaaca tagacttatt 1740

tttaaattgt accaatcaca agaaaataat ggcaataatt atcaaaactt ttaaaataga 1800

tgctcatatt tttaaaataa agttttaaaa ataactgc 1838

<210> 2
<211> 463
<212> PRT
<213> Homo sapiens

<400> 2

Met Gly Ala Gly Pro Ser Leu Leu Leu Ala Ala Leu Leu Leu Leu Leu
1 5 10 15

Ser Gly Asp Gly Ala Val Arg Cys Asp Thr Pro Ala Asn Cys Thr Tyr
20 25 30

Leu Asp Leu Leu Gly Thr Trp Val Phe Gln Val Gly Ser Ser Gly Ser
35 40 45

Gln Arg Asp Val Asn Cys Ser Val Met Gly Pro Gln Glu Lys Lys Val
50 55 60

Val Val Tyr Leu Gln Lys Leu Asp Thr Ala Tyr Asp Asp Leu Gly Asn
65 70 75 80

Ser Gly His Phe Thr Ile Ile Tyr Asn Gln Gly Phe Glu Ile Val Leu
85 90 95

Asn Asp Tyr Lys Trp Phe Ala Phe Phe Lys Tyr Lys Glu Glu Gly Ser
100 105 110

Lys Val Thr Thr Tyr Cys Asn Glu Thr Met Thr Gly Trp Val His Asp
115 120 125

Val Leu Gly Arg Asn Trp Ala Cys Phe Thr Gly Lys Lys Val Gly Thr
130 135 140

Ala Ser Glu Asn Val Tyr Val Asn Thr Ala His Leu Lys Asn Ser Gln
145 150 155 160

Glu Lys Tyr Ser Asn Arg Leu Tyr Lys Tyr Asp His Asn Phe Val Lys
165 170 175

Ala Ile Asn Ala Ile Gln Lys Ser Trp Thr Ala Thr Thr Tyr Met Glu
180 185 190

Tyr Glu Thr Leu Thr Leu Gly Asp Met Ile Arg Arg Ser Gly Gly His

195 200 205

Ser Arg Lys Ile Pro Arg Pro Lys Pro Ala Pro Leu Thr Ala Glu Ile
210 215 220

Gln Gln Lys Ile Leu His Leu Pro Thr Ser Trp Asp Trp Arg Asn Val
225 230 235 240

His Gly Ile Asn Phe Val Ser Pro Val Arg Asn Gln Ala Ser Cys Gly
245 250 255

Ser Cys Tyr Ser Phe Ala Ser Met Gly Met Leu Glu Ala Arg Ile Arg
260 265 270

Ile Leu Thr Asn Asn Ser Gln Thr Pro Ile Leu Ser Pro Gln Glu Val
275 280 285

Val Ser Cys Ser Gln Tyr Ala Gln Gly Cys Glu Gly Gly Phe Pro Tyr
290 295 300

Leu Ile Ala Gly Lys Tyr Ala Gln Asp Phe Gly Leu Val Glu Glu Ala
305 310 315 320

Cys Phe Pro Tyr Thr Gly Thr Asp Ser Pro Cys Lys Met Lys Glu Asp
325 330 335

Cys Phe Arg Tyr Tyr Ser Ser Glu Tyr His Tyr Val Gly Gly Phe Tyr
340 345 350

Gly Gly Cys Asn Glu Ala Leu Met Lys Leu Glu Leu Val His His Gly
355 360 365

Pro Met Ala Val Ala Phe Glu Val Tyr Asp Asp Phe Leu His Tyr Lys
370 375 380

Lys Gly Ile Tyr His His Thr Gly Leu Arg Asp Pro Phe Asn Pro Phe
385 390 395 400

Glu Leu Thr Asn His Ala Val Leu Leu Val Gly Tyr Gly Thr Asp Ser
405 410 415

Ala Ser Gly Met Asp Tyr Trp Ile Val Lys Asn Ser Trp Gly Thr Gly
420 425 430

Trp Gly Glu Asn Gly Tyr Phe Arg Ile Arg Arg Gly Thr Asp Glu Cys
435 440 445

Ala Ile Glu Ser Ile Ala Val Ala Ala Thr Pro Ile Pro Lys Leu
450 455 460

<210> 3
<211> 20

<212> DNA
<213> artificial sequence

<220>
<223> forward primer

<400> 3
attctggcca tttcaccatc 20

<210> 4
<211> 20
<212> DNA
<213> artificial sequence

<220>
<223> reverse primer

<400> 4
ccttgctgcc ctcttcttta 20

<210> 5
<211> 26
<212> DNA
<213> artificial sequence

<220>
<223> probe

<400> 5
caaccaaggc tttgagattg tgttga 26